# EUROPÄISCHE PATENTSCHRIFT

(11) **EP 2 534 162 B1**
(45) Veröffentlichungstag und Bekanntmachung des Hinweises auf die Patenterteilung: **20.04.2016**
(21) Anmeldenummer: 11702070.1
(22) Anmeldetag: 08.02.2011
(51) Int. Cl.: C07H 9/04, B29C 67/00

(54) **VERFAHREN ZUM ERZEUGEN BIOLOGISCH VERTRÄGLICHER, DREIDIMENSIONALER GEGENSTÄNDE ODER OBERFLÄCHEN DURCH LASER-BESTRAHLUNG, SOLCHE GEGENSTÄNDE, DEREN VERWENDUNG SOWIE AUSGANGSMATERIALIEN FÜR DAS VERFAHREN**
METHOD FOR GENERATING BIOLOGICALLY FRIENDLY, THREE-DIMENSIONAL OBJECTS OR SURFACES USING LASER RADIATION, SUCH OBJECTS, USE OF SAME AND STARTING MATERIAL FOR THE METHOD
PROCÉDÉ DE PRODUCTION D'OBJETS OU DE SURFACES EN TROIS DIMENSIONS BIOCOMPATIBLES PAR RAYONNEMENT LASER, LESDITS OBJETS, LEUR UTILISATION, AINSI QUE DES MATÉRIAUX DE DÉPART POUR LE PROCÉDÉ

(30) Priorität: 12.02.2010 EP 10153550
(43) Veröffentlichungstag der Anmeldung: 19.12.2012
(60) Teilanmeldung: 14162852.9
(73) Patentinhaber: Fraunhofer-Gesellschaft zur Förderung der angewandten Forschung e.V., 80686 München (DE)
(72) Erfinder: HOUBERTZ-KRAUSS, Ruth, 97074 Würzburg (DE); BEYER, Matthias, 97070 Würzburg (DE); PROBST, Joern, 97273 Kürnach (DE); STICHEL, Thomas, 97070 Würzburg (DE)
(74) Vertreter: Olgemöller, Luitgard Maria
(86) Internationale Anmeldenummer: PCT/EP2011/051843
(87) Internationale Veröffentlichungsnummer: WO 2011/098460

(56) Entgegenhaltungen:
- WO-A1-03/037606
- FREDERIK CLAEYSSENS ET AL: "Three-Dimensional Biodegradable Structures Fabricated by Two-Photon Polymerization", LANGMUIR, Bd. 25, 2009, Seiten 3219-3223, XP002590369,
- I. R. SALAGEAN ET AL: "Copolymerization of 3-O-Acryloyl-1,2:5,6-di-O-Isopropylidene-a lpha-D-Glucofuranose and Buthyl Acrylate at Different Molar Ratios", CHEM. BULL. "POLITEHNICA" UNIV. TIMISOARA (ROMANIA), Bd. 53, Nr. 1-2, 2008, Seiten 69-72, XP002590370,
- Morrison R. T. und Boyd R. N.: "LEHRBUCH DER ORGANISCHEN CHEMIE", 1974, verlag chemie, Weinheim, XP002632097, * pages732, 733, 734, 741,742 * Absätze [20.15], [ 20.20]

## Beschreibung

Die Erfindung betrifft die Erzeugung biologisch verträglicher, beliebig geformter Körper mit dreidimensionalen Strukturen, die entweder freitragend und/oder durch einen Träger gehalten sein können, durch Verfestigen einer polymerisierbaren Flüssigkeit. Diese Körper sind für vielerlei Zwecke im medizinischen Bereich geeignet, insbesondere jedoch als abbaubare, teilabbaubare oder im Körper verbleibende Implantate, die ggf. vor der Implantation mit geeigneten Zellen besiedelt worden sein können, sowie als Substrate für die Züchtung von für eine Implantierung vorgesehenem Zellgewebe oder von Zellen, deren Inhaltsstoffe gewonnen werden sollen. Insbesondere können Strukturen oder Körper in Größenordnungen von mehreren cm bis unterhalb des µm-Bereichs generiert werden. Die genannten Körper können als Ganzes mit dem erfindungsgemäßen Verfahren generiert werden; gegebenenfalls kann das Verfahren jedoch auch eingesetzt werden, um die Oberfläche bereits vorhandener Körper mit einer dreidimensional geformten Schicht zu versehen, die die gewünschten Eigenschaften aufweist.

Die Erfindung betrifft weiterhin die mit dem Verfahren erhältlichen oder erhaltenen Körper sowie Verwendungen dafür. Schließlich betrifft die Erfindung geeignete Ausgangsmaterialien sowie ein Verfahren zum Herstellen solcher Ausgangsmaterialien, die keine oder nur tolerabel geringe toxische Reaktionen seitens der physiologischen Umgebung hervorrufen.

Um die Zwecke der Erfindung zu erreichen, sollen die erfindungsgemäßen Körper biologisch verträglich sein. Unter diesem Ausdruck soll verstanden werden, dass die Körper je nach Anwendungsgebiet in einem ersten Fall biodegradierbar, d.h. unter physiologischen Bedingungen durch im menschlichen oder tierischen Körper vorhandenen Enzyme oder sonstige Bestandteile oder durch die chemische Umgebung, in der sie sich befinden, zumindest teilweise zerlegbar sein sollen. Unter "zerlegbar" soll dabei verstanden werden, dass die physiologische Umgebung die Spaltung von chemischen - anorganischen, organischen oder anorganisch-organischen wie Kohlenstoff-Metall - Bindungen bewirkt, wodurch der Zusammenhalt des Körpers geschwächt wird oder Poren gebildet werden, bis der Körper im Extremfall in kleinere Bestandteile aufgelöst ist, die innerhalb des Körpers abtransportiert und letztendlich ausgeschieden oder weiter metabolisiert werden. Neben den genannten enzymatischen Vorgängen ist dabei vor allem auf das Auftreten einer unspezifischen Hydrolyse zu verweisen. Wird der biokompatible Körper ganz oder teilweise ohne nachhaltige Entzündungsreaktionen metabolisiert, soll er als bioresorbierbar gelten. Die Biodegradierbarkeit und ggf Bioresorbierbarkeit der Körper wird z.B. dann gewünscht, wenn sie als Knochenimplantate eingesetzt werden sollen, deren Volumen im Lauf der Zeit durch körpereigenen Knochen ersetzt werden sollen. Ähnliches gilt für poröse Körper, die *in vitro* mit Zellen vorbesiedelt wurden und nach der Implantierung durch einen durchgehenden Zellverbund ersetzt werden sollen, z.B. einem Hautstück.

Jedoch ist nicht bei allen Implantaten eine teilweise oder vollständige Biodegradierbarkeit innerhalb eines überschaubaren Zeitraums wünschenswert. Knorpelgewebe beispielsweise bildet sich sehr langsam oder überhaupt nicht mehr vollständig nach, so dass Knorpel-Implantate zumindest sehr lange, wenn nicht für immer, wenigstens in Form eines Rest-Stützgerüsts im Körper, z.B. im Ohr, verbleiben sollten.

Schließlich betrifft die Erfindung auch Substrate für das An- und Aufwachsen von Zellen, die möglicherweise, aber nicht notwendigerweise zum Einsetzen in den menschlichen oder tierischen Körper vorgesehen sind, diesen aber in jedem Fall ein Stützgerüst bieten, das das Vorhandensein einer physiologischen Umgebung vorspiegelt. Dieses technische Gebiet wird häufig als "Tissue Engineering" bezeichnet. So benötigen manche Zelltypen eine spezifische Unterlage, um wachsen zu können, beispielsweise Endothelzellen eine Unterlage, die der natürlichen, collagenhaltigen Basalmembran möglichst ähnlich sein sollte. Die Erfindung betrifft Substrate zum Aufwachsenlassen solcher Zellverbünde, die anschließend entweder an geeigneter Stelle als Implantate eingesetzt werden können oder zur Gewinnung von endokrin oder exokrin erzeugten Stoffe (Hormone, Interleukine, Entzündungsmediatoren, Enzyme oder dgl.) genutzt werden können. Wird eine Implantation nicht ins Auge gefasst, ist eine Biodegradierbarkeit nicht notwendig; allerdings müssen die erfindungsgemäßen Körper auf ihrer Oberfläche Gruppen aufweisen, die der natürlichen Unterlage der jeweiligen Zelltypen soweit angepasst sind, dass sich damit Stoffwechselprodukte der Zellen gewinnen lassen. Diese Eigenschaft kann als Biokompatibilität bezeichnet werden.

Zur Herstellung dreidimensionaler Körper aus polymerisierbaren Flüssigkeiten hat man in der Vergangenheit vor allem stereolithographische Verfahren, aber auch Zwei- oder Mehrphotonenpolymerisation durch selektive Laserbestrahlung spezifischer Volumina (Volumenpixel = Voxel) innerhalb eines transparenten Bades eingesetzt. Stereolithographische Verfahren werden beispielsweise in den beiden WO-Schriften 93/08506 und 95/25003 beschrieben, die eine durch Strahlungseinwirkung verfestigbare Flüssigkeit angeben, welche durch schichtweise (Stereo)Lithographie zu einem dreidimensionalen Körper verfestigt wird. Da die Entwicklung von Strukturen/Komponenten/Bauelementen im Bereich der Sensorik, der Mikrotechnik, der Daten- und Telekommunikation, der Elektronik, der Chiptechnologie und der Medizintechnik zu immer kleiner werdenden Strukturen bei gleichzeitiger Erhöhung der Integrationsdichte sowie auch ihrer Funktionalität tendiert, wurde in jüngerer Zeit vermehrt Zwei- oder Mehrphotonenpolymerisation eingesetzt, um die Struktureigenschaften zu verbessern. Mit diesem Verfahren lässt sich nämlich mit relativ guter Genauigkeit ein nur kleiner Raum innerhalb der zu verfestigenden Flüssigkeit ansteuern, der durch die eingetragene Energie verfestigt wird. Die Herstellung des Formkörpers kann daher innerhalb einer entsprechenden Flüssigkeit erfolgen, nicht mehr (nur) an deren Oberfläche wie bei den stereolithographischen Verfahren. Die Zwei- oder Mehrphotonenpolymerisation ist beispielsweise in H.-B. Sun, et al., Opt. Lett. 25, 1110 (2000) und H.-B. Sun et al., Appl. Phys. Lett. 74, 786 (1999) beschrieben. Ein verfeinertes Verfahren zur Herstellung von Formkörpern ist in DE 101 11 422.2 offenbart. Mit diesem ist ein direktes Polymerisieren eines photosensitiven Harzes ortsselektiv innerhalb des Badvolumens, also auch ggf. weit unterhalb der Badoberfläche, mit hoher Genauigkeit möglich. Gemäß diesem Verfahren wird Strahlung aus einem Spektralbereich verwendet, der normalerweise nicht von der photosensitiven Flüssigkeit unter Hervorrufung des Verfestigungseffektes absorbiert wird. Bei nicht zu hohen Strahlungsintensitäten ist die Flüssigkeit somit für die verwendete Strahlung im Wesentlichen transparent. Das zu verfestigende Bad wird mit Strahlung bestrahlt, deren Wellenlänge λ in etwa n x λₒ beträgt, wobei λₒ die Wellenlänge bezeichnet, bei der das flüssige Material Strahlung absorbieren würde und der Absorptionsvorgang mit einer optischen Anregung unter Auslösung des Material verfestigungsprozesses einherginge. n ist eine ganze Zahl größer als 1. In der Praxis ist n üblicherweise 2. Erzeugt man nun durch Intensitätskonzentration der Strahlung innerhalb des Flüssigkeitsbades eine Intensitätserhöhung, bei der Mehrphotonenabsorption, nämlich n-Photonenabsorption (vorwiegend mit n gleich 2 , aber auch z.B. 3) stattfindet, so kann in dem Bereich dieser lokal erhöhten Intensität die energetische Anregung des Materials zur Induzierung des Verfestigungseffektes durch Bestrahlung mit Licht der Wellenlänge λ erfolgen, wobei diese Strahlung außerhalb der genannten Zone erhöhter Strahlungsintensität die Flüssigkeit in der bereits genannten Weise durchdringt, ohne den Verfestigungseffekt auszulösen. Die Zone erhöhter Intensität kann dabei mit relativ einfachen Mitteln dadurch realisiert werden, dass man den Strahl, ggf. nach vorausgegangener Strahlaufweitung, fokussiert und Ultrakurzpulslaser (Femtosenkundenpuls-Laser) mit hohen Pulsspitzenintensitäten einsetzt, so dass nur in unmittelbarer Umgebung des Fokus die Intensität hoch genug ist, um Zweiphotonenabsoprtion (n = 2) oder Mehrphotonenabsorption (n = 3, ...) in dem flüssigen Material zu erreichen. Zur Herstellung eines Formkörpers in dem Bad kann dann der Fokus nach Maßgabe von Geometriebeschreibungsdaten des Formkörpers innerhalb des Badvolumens zu den zu verfestigenden Stellen geführt werden. Ein ähnliches Verfahren wurde von Kawata et al. in einer Kurzmitteilung in Nature, Vol 412, S. 697 vorgestellt. Verwiesen sei auch auf die Raid-Protoyping-Verfahren, die von M. Lang, R. P. Wong, C.-C. Chu in J. Polym. Sci. A: Polym. Chem. 2002, 40,1127-1141 und von H. Gruber, H. Lichtenegger, R. Liszka, R. Inführ, in EP 1 907 192 B1, 2008 beschrieben sind.

Im Gegensatz zu den stereolithographischen Verfahren wird bei der Erzeugung mittels Femtosekundenlaser-Bestrahlung ein zu erzeugender dreidimensionaler Körper also in nur einem Arbeitsschritt auf einer Oberfläche oder - insbesondere - im Volumen produziert. Dies führt dazu, dass keine unerwünschten Gradienteneffekte im Bauteil auftreten, die bei stereolithographischen Verfahren durch die Art und Weise der Herstellung von Formkörpern durchaus auftreten können. Es können jedoch durch die Variation von z.B. der eingestrahlten Leistungsdichte gezielt Gradienten generiert werden, was zur Ausbildung dichter oder weniger dichter Netzwerke führt.

Darüber hinaus existieren verschiedene Strukturgebungsverfahren, wie die Synthese über Leeching-Verfahren (J. S. Tjia, P. V. Moghe, J. Biomed. Mater. Res. 1998, 43, 291-299; R. Zhang, P. X. Ma, J. Biomed. Mater. Res. 2000, 52, 430-438). Diese haben jedoch unterschiedliche Schwächen: die erreichbaren Strukturgrößen bzw. Scaffolds sind entweder zu klein oder erheblich zu groß/grob, ihre Porosität lässt sich nicht gezielt einstellen oder sie führen zu mechanisch instabilen Scaffolds, die mitunter auch zu schnell degradieren, oder der Materialaufwand zur Herstellung von Scaffolds bzw. die Herstellungsdauer ist sehr hoch (J. Stampfl, M. Schuster, S. Baudis, H. Lichtenegger, R. Liska, Virtual Rapid Manufac. Proc. VRAP 2007, 283-288).

Die für diese Verfahren eingesetzten Materialien sind häufig organische Thermoplaste (z.B. Polymethylmethacrylat), die teilweise schon bei Temperaturen ab 80°C erweichen oder schmelzen. Verbesserte, anorganisch-organische Hybrid-Materialien sind in WO 03/037606 A1 offenbart.

Gerüste für das Aufwachsen von Zellen, sogenannte Scaffolds, werden teilweise mit Hilfe des Rapid Prototyping erzeugt. R. Landers et al. beschreiben in Biomaterials 23 (2002) 4436-4447 die Herstellung solcher Scaffolds ausgehend von thermoreversiblen Hydroygelen aus Agarose, Gelatine und Agar. Dreidimensionale Strukturen, hergestellt aus dem bioabbaubaren Dreiblock-Copolymeren Poly(ε-caprolacton-cotrimethylencarbonat)-b-poly(ethylenglycol)-b-poly (ε-caprolacton-cotrimethylencarbonat) mit Hilfe der Zwei-Photonen-Polymerisation, stellten F. Claeyssens et al. in Langmuir (2009), 25, 3219-3223 vor. In WO 96/40002 wird eine Matrix für die Regenerierung von Gewebe vorgeschlagen, die mit Hilfe von Stereolithographie, Lasersintern, Dreidimensionalem Drucken oder dgl. hergestellt werden kann. Verschiedenste Materialien werden für die Herstellung der Matrix vorgeschlagen, darunter synthetische thermoplastische Polymere, Polyorthoester, Polymere von Milchsäure und Glycolsäure und anderen α-Hydroxysäuren sowie Proteine wie Albumin oder Collagen oder Polysaccharide.

Als biodegradierbare Trägermaterialien sind verschiedene Stoffklassen geeignet. Vorraussetzung ist, dass das Material unter physiologischen Bedingungen eine Zersetzungsreaktion eingeht und keine gravierende Autoimmunreaktionen hervorruft. Neben bisher bekannten natürlich Polymeren, wie Collagen oder Alginat, sind die Polyester, wie u. a. Polyglycolsäure (PGA), Polylactidsäure (PLA) und Polylactid-coglycolidsäure (PLGA), sowie Polycaprolacton (PCL) kommerziell erhältlich und von der FDA zugelassen. Einige davon werden bereits kommerziell als Implantatmaterial (z.B. als Knochennägel) genutzt. Der Nachteil dieser Polyester ist allerdings, dass ihre Degradationsprodukte zu einer starken lokalen Reduktion des pH-Wertes führen und damit Entzündungsreaktionen hervorrufen, siehe E. Wintermantel et al., Medizintechnik Life Science Engineering, 4. Auflage, Springer, Berlin (2008), 143-268.
Ein dextranbasiertes, durch Photovernetzung erzeugtes Hydrogel beschreiben S. H. Kim et al. in Carbohydrate Polymers 40 (1999) 183-190. Dafür wurde Dextran bromacetyliert und mit Natriumacrylat umgesetzt, und das acrylierte Dextran wurde mit langwelligem UV bestrahlt. Ein bioabbaubares, hydrophob-hydrophiles Hydrogel-Polymer auf Dextranbasis mit kontrollierten Quelleigenschaften stellten Y. Zhang et al. in J. Polym. Chem. 38 (2000) 2392-2404 vor. Sie erhielten es durch die Bestrahlung einer Mischung von acryloylierter Milchsäure und einem allylisocyanat-modifiziertem Dextranderivat.

Weitere, von natürlichen Stoffklassen abgeleitete Materialien können sich ebenfalls für die Herstellung von Trägermaterialien eignen, wenn sie mit vernetzbaren Gruppen funktionalisiert sind. Hierfür wurden in der Vergangenheit bereits häufiger (Meth)Acrylatgruppen vorgeschlagen. K. Ohno et al. haben in Macromol. Chem. Phys. 200 (1999), 1619-1625 das zuckerhaltige Acrylat, 2-O-Acryloyl-1,2:5,6-di-O-isopropyliden-α-D-glucofuranosid vorgestellt, das mittels radikalischer Polymerisation allein oder in Form eines Block-Copolymerisats polymerisiert werden konnte. Die Acidolyse dieser Produkte lieferte wasserlösliche bzw. amphiphile Produkte. Für einen völlig anderen Zweck, nämlich die kontrollierte Freisetzung von 5-Fluoruracil *in vivo,* synthetisierten T. Ouchi et al. gemäß Journal of Polymer Science: PartA: Polymer Chemistry 23 (1986), 2059-2074 ein acryloylartiges Polymer, das 1-β-Carbonylethyl-5-fluoruracil-Reste durch Esterbindungen an D-Glucofuranose binden kann. In beiden Fällen wurde die Anbindung der Acrylate mittels Acrylsäurechlorid bewirkt, einer sehr toxischen Verbindung. T. Bird et al. synthetisierten dagegen die 6-(Meth)Acryloylester von 1,2:3,4-di-O-isopropyliden-a-D-Galactopyranose über eine Versterung mit Methacrylsäureanhydrid. Die Produkte ließen sich entschützen und thermisch (in Gegenwart eines Initiators) zu wasserlöslichen, zu Zuckersäuren oxidierbaren Produkten polymerisieren. Die Na-Salze der Zuckersäuren wiederum erwiesen sich als viskose Polyelektrolyte, siehe J. Chem. Soc. (C) 1966, 1913-1918. Die enzymatische Acylierung (mit einer Lipase) von D-Glucose mit Vinylacrylat und anschließende Polymerisation untersuchten I. Ikeda et al. in Biotechnology and Bioengineering 42 (1993) 788-791.

Neben Zuckern waren vor allem Polymilchsäurederivate im Fokus von nach biokompatiblen Trägermaterialien suchenden Forschem. Extrazelluläre Matrices für Tissue Engineerung sollen nach den Vorstellungen von R. Zhang et al. in J. Biomed. Mater. Res 52 (2000) 430-438 unter Verwendung von Poly-L-Milchsäure nach einem komplizierten Verfahren derart hergestellt werden, dass sie die anatomische Form der natürlichen Matrix nachbilden, also makroporöse Elemente, Fasern und Faserzwischenräume im Mikrometerbereich aufweisen. Solche ebenfalls poröse Matrices können nach den Vorschlägen von J. Tjia et al. in J. Biomed. Mater. Res. 43 (1998) 291-299 aus einem Poly(milchsäure-Glycolsäure)-Copolymer hergestellt werden. M. Lang et al., J. Polym. Sci. A: Polym. Chem. 40 (2002) 1127-1141, beschreiben die Herstellung von biologisch abbaubarem Poly-ε-caprolacton, das mit Maleinsäureanhydrid funktionalisiert worden war. Das dadurch angekoppelte Michaelsystem könnte nach den Vorstellungen der Autoren später für eine radikalische Polymerisation des Caprolactons genutzt werden.

D. Tian et al. berichten in J. Polym. Sci. A: Polym. Chem. 35 (1997), 2295-2309 über Materialien, die sie durch Umsetzung von hydroxyliertem Poly-ε-caprolacton mit 3-Isocyanatopropyltriethoxysilan oder durch Umsetzung von mit Vinylgruppen modifiziertem Poly-ε-caprolacton mit Triethoxysilan erhielten. Außerdem wurde ein Hybridmaterial aus Tetraethoxysilan und einem silylierten Polycaprolacton hergestellt.

Die Druckschrift "Three-Dimensional Biodegradable Structures Fabricated by Two-Photon Polymerization" von Frederik Claeyssens et al., Langmuir 2009, 25, 3219-3223 offenbart ein Verfahren zum Erzeugen von dreidimensionalen Formkörpern mit Hilfe von Zwei-Photonen Polymerisation.

Der vorliegenden Erfindung liegt die Aufgabe zugrunde, ein Verfahren bereitzustellen, mit dessen Hiilfe beliebige, sowohl großdimensionierte als auch extrem feinstrukturierte, gegebenenfalls poröse dreidimensionale Strukturen erzeugt werden können, die die Form von (ggf. selbsttragenden) Körpern oder die Form von oberflächenstrukturierten oder sonstigen Schichten besitzen. Diese Körper sollen biokompatibel, biodegradierbar und/oder bioresorbierbar sein und keine Toxizität gegenüber dem Organismus bzw. den Organismen aufweisen, mit denen sie in Kontakt kommen. Die Erfindung soll weiterhin spezifische Produkte mit den genannten Eigenschaften bereitstellen sowie Verwendungsmöglichkeiten dafür angeben. Schließlich soll die Erfindung spezifische Ausgangsmaterialien bereitstellen, die sich für das erfindungsgemäße Verfahren eignen.

Die Erfindung betrifft außerdem die Herstellung von toxisch unbedenklichen Acrylsäure- und Methacrylsäurederivaten von monomeren und oligomeren Zuckern sowie Kohlehydraten. Mit "toxisch" werden im Rahmen der vorliegenden Erfindung Stoffe bezeichnet, die in physiologischer Umgebung unerwünschte Nebenwirkungen, insbesondere eine detektierbare, meist starke immunologische Abwehrreaktion hervorrufen.

Die genannte Aufgabe wird gelöst durch ein Verfahren gemäß Anspruch 1 zum Erzeugen von dreidimensionalen selbsttragenden und/oder substratgestützten Formkörpern oder von dreidimensionale Oberflächenstrukturen durch ortsselektives Verfestigen eines flüssigen bis pastösen, organischen oder organisch modifizierten Materials innerhalb eines Bades aus diesem Material mit Hilfe von Zwei- oder Mehr-Photonen-Polymerisation, wobei das Material einen oder mehrere Bestandteile aufweist, ausgewählt unter Verbindungen mit einem organischen, über 2-Photonen- oder Mehrphotonen-Polymerisation polymerisierbaren Rest und/oder Verbindungen, die mindestens einen biokompatiblen, biodegradierbaren oder bioresorbierbaren Rest besitzen, mit der Maßgabe, dass in dem Material sowohl ein organischer, über 2-Photonen- oder Mehrphotonen-Polymerisation polymerisierbarer Rest als auch eine biokompatible, biodegradierbare oder bioresorbierbare Gruppe enthalten sein müssen. Als "dreidimensionale Oberflächenstruktur" ist eine erfindunggsgemäß aufgebrachte Schicht auf einem beliebig geformten, bereits bestehenden Körper zu verstehen. Das bedeutet, dass diese Schicht entweder bei im Wesentlichen gleich bleibender oder bei schwankender Dicke eine Oberflächenstrukturierung und/oder eine der Gestalt des bereits bestehenden Körpers folgende Kontur aufweist.

Die Aufgabe wird weiterhin gelöst durch die Bereitstellung der entsprechenden Formkörper mit vorteilhaften Eigenschaften.

Im Gegensatz zu gängigen Verfahren zur Herstellung dreidimensionaler Trägerstrukturen zeichnet sich die Technologie der vorliegenden Erfindung hinsichtlich des Materials und des Verfahrens dadurch aus, dass
(a) beliebige dreidimensionale Trägerstrukturen direkt und schnell in einem Material hergestellt werden können,
(b) eine variable Porenstruktur vom 100 nm bis in den mm-Bereich eingestellt werden kann,
(c) eine wahlweise interkonnektierende Porenstruktur erhalten werden kann,
(d) eine großflächige Strukturierung bis in den cm-Maßstab mit hoher Präzision und sehr hoher Wiederholgenauigkeit durchgeführt werden kann,
(e) auf Sinterprozesse, wie sie in anderen Verfahren (s. a. Stand der Technik) angewendet werden müssen, verzichtet werden kann,
(f) beliebig viele extrem genaue Kopien der Strukturen erzeugt werden können,
(g) die Strukturen beliebig skalierbar sind,
(h) Patienten-individuelle Implantate hergestellt werden können, wobei die geometrischen Daten aus 3D-Scans (z. B. CT, Laser-Triangulation) direkt im Fertigungsprozess umgesetzt werden können,
(i) insbesondere auch sichtbares Licht sowie infra-rotes Licht durch Ultrakurzpulse, d. h. durch Anregung von Zwei- oder Mehr-Photonen-Prozesse, für die Herstellung der Strukturen verwendet werden kann,
(j) dioden-gepumpte Festkörperlaser oder Halbleiterlaser, Faserlaser, etc. verwendet werden können,
(k) in der Materialvorstufe auch biologische Komponenten integriert werden können.

Die Erfindung beschreibt unter anderem die Herstellung zumindest teilweise physiologisch degradierbarer, biokompatibler oder bioresorbierbarer Trägermaterialien und Funktionselemente, die z.B. bei der Regeneration von Geweben und Organen und der Wiederherstellung ihrer Funktion eine zentrale Rolle einnehmen können. Die Trägermaterialien oder Funktionselemente können nicht-porös sein, z. B. in Form von Nanostrukturen, oder sie können eine Porenstruktur im Bereich zwischen vorzugsweise 100 nm und 2 mm besitzen, die dem besseren Einwachsen von Zellen und ihrer verbesserten Vernetzung dient. Die Strukturen können zusätzlich oder eigenständig als Drug-Delivery-Depots dienen. Insbesondere lassen sich großflächige, u. a. für die Zellreifung, Zellvermehrung und Zelldifferenzierung notwendige dreidimensionale Trägerstrukturen mit geeigneter Porenstruktur direkt in drei Dimensionen durch Mehr-Photonen-Prozesse herstellen, wobei bevorzugt Zwei- oder Drei-Photonen-Prozesse angewendet werden. Auch Prozesse weiterer höherer Ordnung können angeregt werden

Als biödegradierbare Trägermaterialien sind unter anderem die in der Einleitung genannten Materialien einsetzbar. Neben den dort genannten Polyestern kommen aber noch einige weitere Stoffklassen als biodegradierbare Strukturelemente in Frage. Diese sind insbesondere:
1. Kohlenhydrate (Mono-, Di- und Polysaccharide)
2. Aminosäuren, Peptide und Proteine, z.B. Kollagene oder Enzyme, sowie deren Lactam-Strukturen
3. Polyanhydride
4. Polyorthoester
5. Polyether
6. Polycarbonate
7. Polyamide
8. Monohydroxysäuren (insbesondere α- oder ω-Hydroxysäuren sowie deren Lactone und Lactide) sowie deren Oligomere oder Polymere
9. Der biokompatible, biodegradierbare oder bioresorbierbare Rest wird demnach je nach Bedarf vom Fachmann ausgewählt. Günstig sind Reste, die mindestens eine (und vorzugsweise 2 oder, stärker bevorzugt, mindestens 3) Gruppen aufweisen, die jeweils unabhängig voneinander ausgewählt sind unter -O-, -OC(O)O-, -C(O)NH-, -NHC(O)NH-, -NHC(O)O-, -C(O)OC(O)- oder -C(O)O-, wobei diese Gruppe(n) besonders bevorzugt Bestandteil einer Verbindung aus der jeweils hierfür in Frage kommenden Stoffklasse wie oben erwähnt ist/sind, also Bestandteil eines Kohlenhydrats (Mono-, Di- oder Polysaccharide), einer Aminosäure, eines Peptids oder Proteins, z.B. eines Kollagens oder Enzyms, oder einer Lactam-Struktur der vorgenannten Amino-Verbindungen, eines Polyanhydrids, eines Polyorthoesters, eines Polyethers, eines Polycarbonats, eines Polyamids oder einer Monohydroxysäure (insbesondere einer α- oder ω-Hydroxysäure oder einem Lacton oder Lactid davon) oder eines Oligomeren oder Polymeren der in diesem Absatz genannten Verbindungen ist/sind, wobei diese Stoffe natürlichen oder synthetischen Ursprungs sein können. Der biokompatible, biodegradierbare oder bioresorbierbare Rest wird zusätzlich oder alternativ ausgewählt unter Resten, die unter physiologischen Bedingungen im menschlichen oder tierischen Körper oder durch den Befall von Mikroorganismen gespalten werden können. Günstig sind ferner Aldose- oder Ketosegruppen mit einer Kette von mindestens 4 C-Atomen und mindestens 2 freien oder geschützten Hydroxygruppen. Bevorzugt enthalten die Reste mindestens einen Di- oder Oligosaccharid-, einen Di- oder Oligopeptid-, einen Di- oder Oligoester-, einen Di- oder Oligocarbonsäureamid-, einen Di- oder Oligoguanidino-, einen Di- oder Oligoanhydrid- oder einen Di- oder Oligourethan-Rest. Besonders bevorzugt handelt es sich um Reste mit mindestens einer Kohlehydrat-, Peptid-, Protein-, Polyanhydrid-, Polycarbonat-, Polyorthoester-, Polyether, Polyester-, oder Polyamidgruppe. Jeder der vorgenannten Reste kann eine einzelne, mehrere identische oder eine Kombination von mehreren unterschiedlichen der vorgenannten Gruppen aufweisen. Das Material kann identische oder unterschiedliche Reste enthalten.

Im Rahmen der Erfindung wird der Ausdruck "oligomer" für 2 bis 5 aufeinander folgende, verwandte Struktureinheiten verwendet; eine Molekülstruktur mit 6 oder mehr solchen Struktureinheiten wird mit "polymer" bezeichnet.

Um strukturierbare biodegradierbare Trägermaterialien zu erhalten, müssen die o. g. physiologisch degradierbaren Strukturelemente funktionalisiert werden, so dass eine gemeinsame Vernetzungsreaktion, z. B. eine Polymerisation oder eine Polykondensation und damit eine durch äußere Einflüsse initiierte Vernetzung durch photolithographische Prozesse ermöglicht wird.

Es ist für alle Ausführungsformen der Erfindung bevorzugt, dass die Zwei- oder Mehrphotonenpolymerisation des organischen, über 2-Photonen- oder Mehrphotonen-Polymerisation polymerisierbaren Rests über eine bzw. mehrere Gruppen erfolgt, die sich radikalisch polymerisieren lassen. Zwar sind auch solche Systeme erfindungsgemäß geeignet, die sich mit Hilfe eines kationischen UV-Starters polymerisieren lassen, beispielsweise Epoxysysteme (siehe z.B. C.G. Roffey, Photogeneration of Reactive Species for UV Curing, John Wiley & Sons Ltd, (1997)). Doch neigen diese Systeme zu parasitärer Polymerisation, d.h. es findet auch eine Polymerisation in die nicht belichteten Bereiche hinein statt, weswegen sie für Anwendungsbereiche mit extrernen Anforderungen an die Feinheit und Glätte der Oberflächen, z.B. hochaufgelöste Lithographie, weniger gut geeignet sein können (je nach Modifikation). Als radikalisch polymerisierbare Gruppen eignen sich nichtaromatische C=C-Doppelbindungen wie Allyl- oder Vinylgruppen, besonders bevorzugt sind allerdings einer Michael-Addition zugängliche Doppelbindungen. Acrylat- und Methacrylatreste sind ganz besonders bevorzugt. Nachstehend wird häufig der Ausdruck (Meth)Acryl... verwendet, um Acrylsäure(derivate) und/oder Methacrylsäure(derivate) alleine oder in Kombination zu bezeichnen. Es sei darauf hingewiesen, dass (Meth)Acrylatreste im Sinne der vorliegenden Erfindung nicht als biokompatibel, biodegradierbar oder bioresorbierbar anzusehen sind, auch wenn sie über eine Estergruppe an den Rest des jeweiligen Moleküls angebunden sind.

In einer ersten Ausführungsform der Erfindung wird für die Polymerisationsreaktion ein rein organisches Material eingesetzt, das die erfindungsgemäßen Bestandteile aufweist: In dieser Ausführungsform enthält das genannte Material bevorzugt mindestens eine Verbindung, die sowohl einen organischen, über 2-Photonen- oder Mehrphotonen-Polymerisation polymerisierbaren Rest als auch eine biokompatible, biodegradierbare oder bioresorbierbare Gruppe.

Bei dem organischen, über 2-Photonen- oder Mehrphotonen-Polymerisation polymerisierbaren Rest handelt es sich demnach vorzugsweise um einen radikalisch polymerisierbaren Rest, der stärker bevorzugt mindestens eine C=C-Doppelbindung und ganz besonders bevorzugt einen einer Michael-Addition zugänglichen Rest, insbesondere einen (Meth)Acrylatrest aufweist. Dieser ist in geeigneter Weise mit dem Rest des Moleküls verbunden. Handelt es sich bei dem über 2-Photonen- oder Mehrphotonen-Polymerisation polymerisierbaren Rest beispielsweise um einen (Meth)Acrylatrest oder einen Rest, der eine freie oder aktivierbare Säurefunktion aufweist, kann die Verknüpfung beispielsweise über eine OH-Gruppe unter Ausbildung einer Esterfunktion erfolgen. Dies ist insbesondere günstig, wenn es sich bei der biokompatiblen, biodegradierbaren oder bioresorbierbaren Gruppe um einen Zucker, ein Kohlenhydrat oder eine Hydroxysäure bzw. ein Oligomer/Polymer einer solchen Säure handelt, weil eine solche Gruppe in der Regel über freie Hydroxygruppen verfügt. Eine weitere Alternative ist die Verknüpfung über eine Isocyanatguppe, z.B. dann, wenn der über 2-Photonen- oder Mehrphotonen-Polymerisation polymerisierbare Rest keine Acylfunktion besitzt. Die Isocyanatgruppe kann mit einer freien Hydroxygruppe zu einer Urethangruppe als Verknüpfung zwischen den beiden Bestandteilen reagieren.

Weitere Beispiele für Materialien, die sowohl einen organischen, über 2-Photonen- oder Mehrphotonen-Polymerisation polymerisierbaren Rest als auch eine biokompatible, biodegradierbare oder bioresorbierbare Gruppe aufweisen, sind Polypeptide/Polyamide, in denen eine endständige Aminogruppe oder eine freie Aminogruppe innerhalb des Moleküls mit einer Säurefunktion umgesetzt ist, an die wieiterhin ein solcher organischer, über 2-Photonen- oder Mehrphotonen-Polymerisation polymerisierbarer Rest gebunden ist. Darunter fallen z.B. mit Acrylsäure oder Methacrylsäure umgesetzte Polypeptide/Polyamide. Diese können in der Kette durch andere Gruppen wie Polyethylenglycol oder dgl. unterbrochen und/oder zusätzlich substituiert sein. Als Beispiele seien H₂C=CH-C(O)NH(As(As)ₙ)-PEG-(As(As)ₙ)-NH-C(O)-CH=CH₂ genannt, worin (As(As)ₙ) für eine Aminosäure oder ein Peptid mit mehreren, beispielsweise 2-5 Aminosäuren (für n = 1-4) steht. In J. West et al., Macromolecules (1999) 32, 241-244 sind solche Moleküle mit (As(As)ₙ) = Ala-Pro-Gly-Leu oder Val-Arg-Asn genannt. Weitere Beispiele sind Polyanhydride, abgeleitet aus gesättigten oder ungesättigten Di- oder Polycarbonsäuren und einer Säure mit einem entsprechenden organischen, über 2-Photonen- oder Mehrphotonen-Polymerisation polymerisierbaren Rest wie (Meth)Acrylsäure. Als Beispiel sei H₂C=CR-C(O)-(CH₂)ₙ-C(O)-CR=CH₂ genannt. Diese Verbindung ist mit R = H und n = 8 in K.S. Anseth et al., Nature Biotechnology (1999) 17, 1156-159 beschrieben. Auch Polycarbonate mit Acrylat- oder Methacrylatgruppen sind aus dem Stand der Technik bekannt, z.B. Verbindungen, die die Gruppe -(C₆H₄)-X-(C₆H₄)-O-C(O)-O- sowie Acrylat- oder Methacrylatgruppen gebunden enthält, wobei X = O, S, SO₂ oder CO sein kann, siehe EP 499 435 A2. Auch Polyorthoester, die über 2-Photonen- oder Mehrphotonen-Polymerisation vernetzt werden können, sind bekannt, z.B. 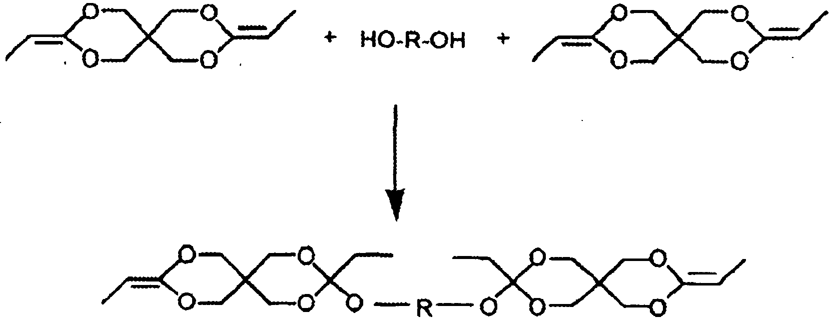 (siehe J. Heller et al., Advanced Drug Delivery Reviews (202), 54, 1015-1039). Als Beispiele für Polyether, die einer solchen Vernetzungsreaktion zugänglich sind, sei schließlich auf die große Anzahl von Polyethylenglycoldi(meth)acrylaten verwiesen, die im Handel erhältlich sind und beispielsweise von Sigma Aldrich vertrieben werden, darunter diacryliertes und dimethacryliertes PEG mit Molekulargewichten im Bereich von >200 bis ca. 6000, sowie dimethacryliertes Polypropylenglycol.

Die Erfindung offenbart in diesem Zusammenhang bevorzugte Funktionalisierungsreaktionen (potentiell) physiologisch degradierbarer Stoffe und Materialklassen, deren Produkte in Hinblick auf die mögliche Toxizität unbedenklicher sind als die im Stand der Technik beschriebenen. Es handelt sich dabei um die Verknüpfung von hydroxygruppenhaltigen Ausgangsmaterialien, insbesondere von Zuckern, Kohlehydraten, Zuckeralkoholen und gesättigten Hydroxysäuren sowie deren Oligo- und Polymeren, mit Acrylsäure- und Methacrylsäureguppen. Bei den bisher in der Regel genutzten Umsetzungen entstehen toxische Nebenprodukte, die für die Zwecke der vorliegenden Erfindung nachteilig sind, da sie kaum abtrennbar sind und beim erfindungsgemäßen Verfahren deshalb in den dabei erzeugten Gegenständen verbleiben. Erfindungsgemäß werden daher zur Erzeugung der obigen Ausgangsmaterialien Umsetzungen mit (Meth)Acrylsäureestem oder mit (Meth)acrylsäureanhydrid, wobei letzteres teilweise aus dem Stand der Technik bekannt ist, eingesetzt. Die Anmeldung beschreibt ebenfalls ein Verfahren zum Herstellen einer Verbindung mit der Formel (A)

R'-CHR"-O-C(O)-CR"'=CH₂ (A)

bereit, worin der Rest R"' H oder CH₃ bedeutet und worin R'CHR" abgeleitet ist von einer Verbindung R'-CHR"OH, ausgewählt unter
(a) Monosacchariden, nämlich Aldo- und Keto-Pentosen, -Hexosen und -Heptosen mit mindestens einer (und vorzugsweise nur einer einzigen) freien Hydroxygruppe, worin die nicht frei vorliegenden Hydroxygruppen in geschützter Form, insbesondere als Ether oder Ester, z.B. Borsäureester, vorliegen,
   dimeren, oligomeren oder polymeren Kohlehydraten, die mindestens eines der genannten Monosaccharide enthalten,
(b) monomeren oder oligomeren oder polymeren Zuckeralkoholen, erhältlich durch Reduktion der Aldehyd- oder Ketogruppe in den unter (a) genannten Molekülen,
(c) gesättigten monomeren, oligomeren oder polymeren Mono-Hydroxysäuren sowie deren Lactone und Lactide, ausgewählt unter α-Hydroxysäuren mit 3 oder 4 Kohlenstoffatomen und ω-Hydroxysäuren mit 4-8 Kohlenstoffatomen,
dadurch gekennzeichnet, dass eine Verbindung

R'-CHR"OH

mit einer Verbindung

R^{IV}O-C(O)-CR'"=CH₂,

worin R^{IV} ein Alkylrest mit vorzugsweise 1 bis 20, stärker bevorzugt mit 1 bis 10 Kohlenstoffatomen, der gegebenenfalls mit einer oder mehreren Gruppen O-C(O)-CR"'=CH₂ substituiert sein kann und vorzugsweise CH₃, C₂H₅ oder CH₂-CH₂-O-C(O)-CR"'=CH₂ ist, unter Bedingungen umgesetzt wird, die das Reaktionsgleichgewicht zum Produkt hin verschieben. Als Bedingung, die das Reaktionsgleichgewicht zum Produkt hin verschiebt, ist vorzugsweise ein großer Überschuss an (Meth)Acrylsäureester zu nennen, die gleichzeitig als Lösungsmittel fungieren kann. Statt dessen oder zusätzlich kann ein Katalysator verwendet werden, beispielsweise ein Alkoxytitanat. Beispiele für geeignete Ausgangsmaterialien sind C-6-Pyranosen oder Furanosen oder davon abgeleitete reine oder gemischte Di- oder Polysaccharide, in denen pro Zuckermolekül zweimal jeweils zwei Hydroxygruppen durch eine Isopropylidengruppe geschützt sind, Milchsäure, ggf. derivatisierte Polylactate und Polymere von y.-Butyro-, Valerio- oder Caprolacton. Überraschenderweise hat sich dabei herausgestellt, dass bei Verwendung von Polylactonen je nach Reaktionsführung die Polymeren unter Umesterung mit dem (Meth)Acrylester gespalten werden, so dass man in manchen Fällen monomere Produkte der Formel (A) aus polymeren Ausgangsmaterialien erhält. Anstelle der Verbindung R^{IV}O-C(O)-CR"'=CH₂ kann auch das Säurebromid Br-C(O)-CH₂-Br als Reaktionspartner dienen, worauf das dabei entstandene Produkt mit Natriurn(meth)acrylat umgesetzt wird.

Bei den oben beschriebenen, erfindungsgemäßen Funktionalisierungsreaktionen über die Anhydride, eine Umesterung oder durch eine nucleophile Substitution werden insbesondere toxische Funktionalisierungsstoffe vermieden. Bei den Produkten handelt es sich um Zucker, Kohlehydrate, Polyether oder Polyester, die eine definierte Länge aufweisen können und durch Mehr-Photönen-Prozesse vernetzbar und somit strukturierbar sind.

Es ist jedoch bevorzugt, die Trägermaterialien der Erfindung aus reaktiven Monomeren (oder deren Prepolymeren) aufzubauen, die neben einer organisch vernetzbaren Funktionalität und einer biologisch verträglichen Einheit zusätzlich eine anorganische Vernetzbarkeit aufweisen.

In einer bevorzugten Ausgestaltung der Erfindung enthält das Badmaterial aus diesem Grunde weiterhin Gruppen oder Reste, die einer anorganischen Vernetzung zugänglich sind oder bereits anorganisch vernetzt sind, darunter insbesondere oligomerisierte oder stärker polymerisierte Einheiten mit -O-Si-O- oder -O-M-O-Bindungen mit M gleich Bor, Aluminium oder einem Übergangsmetall. Insbesondere enthält das Bandmaterial vorzugsweise ein Organopolysiloxan-Material, das beispielsweise durch Hydrolyse und mindestens teilweise Kondensation mindestens eines Silans der Formel (I)

R¹ₐR²_{b}SiX_{4-a-b} (I)

sowie gegebenenfalls zusätzlich mindestens einer Verbindung des Typs

M^{III}(OR³)₃ (II)

und/oder des Typs

M^{IV}(OR³)₄ (III)

erhalten wurde bzw. erhältlich ist, und/oder mindestens eine noch nicht hydrolytisch kondensierte Verbindung (I) und gegebenenfalls mindestens eine solche der Formeln (II) und/oder (III), worin R¹ gleich oder verschieden ist und einen organischen, über Zwei- oder Mehrphotonenpolymerisation polymerisierbaren Rest wie oben definiert darstellt, R² gleich oder verschieden ist und einen organischen, nicht auf diese Art und Weise polymerisierbaren Rest bedeutet, R³ eine Alkoxygruppe (mit vorzugsweise 1 bis 4 Kohlenstoffatomen) darstellt und X OH oder ein unter Hydrolysebedingungen hydrolytisch kondensierbarer Rest ist, der Index a 0, 1, 2 oder 3 bedeutet, der Index b 0, 1 oder 2 bedeutet und a+b zusammen 0, 1, 2 oder 3 und vorzugsweise 1, 2 oder 3 sind. Derartige anorganisch-organische Hybrid-Materialien sind unter der für die Anmelderin eingetragenen Marke ORMOCER® bekannt und existieren in einer sehr großen Vielfalt. Durch 2-Photonen-Polymerisation verfestigbare Badmaterialien aus Silanen mit a=1 sind beispielsweise in WO 03/037606 beschrieben; solche Materialien werden auch in R. Houbertz et al., Thin Solid Films 442 (2003), 194-200 vorgestellt.

In dieser besonders bevorzugten Ausgestaltung der Erfindung können in einer ersten Variante die drei für das Badmaterial notwendigen Bestandteile, nämlich der organische, über 2-Photonen- oder Mehrphotonen-Polymerisation polymerisierbare Rest, der biokompatible, biodegradierbare oder bioresorbierbare Rest und die Gruppe oder der Rest, die/der einer anorganischen Vernetzung zugänglich ist oder bereits anorganisch vernetzt ist, in beliebiger Weise an ein und derselben strukturellen Einheit gebunden sein.

Wenn die drei vorgenannten Bestandteile an einer einzigen strukturellen Einheit gebunden sind, so gibt es zwei Möglichkeiten: Entweder muss R² im Silan der Formel (I) ein biokompatibler, biodegradierbarer oder bioresorbierbarer Rest sein bzw. einen solchen aufweisen, und a und b müssen jeweils mindestens 1 bedeuten, oder der Rest R¹ in diesem Silan muss zusätzlich zu seiner Eigenschaft, ein über Zwei- oder Mehrphotonenpolymerisation polymerisierbaren Rest zu sein, biokompatibel, biedegradierbar oder bioresorbierbar sein bzw. einen Bestandteil mit einer dieser Eigenschaften aufweisen. Dann kann R² beliebig gewählt sein oder - im Falle von b=0 - fehlen, siehe auch Anspruch 9.

Derartige Silane sind prinzipiell aus dem Stand der Technik bekannt. So zeigt DE 10 2008 057 684.0 Silane, die an einem über Kohlenstoff an das Siliciumatom gebundenen Rest eine Aminosäure oder ein Peptid sowie mindestens eine (Meth)Acrylatgruppe aufweisen. Solche Silane lassen sich beispielsweise durch Umsetzung eines Trialkoxysilans, welches über z.B. eine Propylengruppe eine Methacrylatgruppe und außerdem eine freie (oder aktivierte) Carbonsäuregruppe gebunden enthält (oder durch Umsetzung des entsprechenden, durch hydrolytische Kondensation erhaltenen Polysiloxans) mit einer freien Aminosäure oder einem Peptid mit einer freien Aminogruppe, z.B. 3,4-Dihydroxy-L-phenylalanin, unter Bedingungen erhalten, die das Gleichgewicht der Reaktion zum Säureamid hin verschieben.

In einer zweiten Variante können die drei vorgenannten Bestandteile an zwei strukturelle Einheiten gebunden sein. Hier können in einer ersten Ausführungsform der organische, über 2-Photonen- oder Mehrphotonen-Polymerisation polymerisierbare Rest und der biokompatible, biodegradierbare oder bioresorbierbare Rest Bestandteil einer rein organischen Verbindung sein, während das Badmaterial zusätzlich beliebige Organopolysiloxane, die durch zumindest teilweise hydrolytischen Kondensation aus Verbindungen des Typs

R¹ₐR²_{b}SiX_{4-a-b} (I)

sowie gegebenenfalls zusätzlich mindestens einer Verbindung des Typs

M^{III}(OR³)₃ (II)

und/oder des Typs

M^{IV}(OR³)₄ (III)

erhalten wurden oder erhältlich sind, und/oder noch nicht hydrolytisch kondensierte Verbindungen (I) und gegebenenfalls (II) und/oder (III) enthält, worin die Reste und Indices R¹, R², X, a, b und a+b wie oben definiert sind. Es ist dabei aber besonders bevorzugt, dass a mindestens 1 ist. Denn dann kann das Polykondensat über die 2-oder Mehrphotonenpolymerisation in das organische Netzwerk einpolymerisiert werden, das bei der Polymerisation der rein organischen Verbindung entsteht.

Diese Ausführungsform ist besonders bevorzugt. Zum Beispiel kann es sich hierbei um Badmaterialien mit acrylsäure- oder methacrylsäurefunktionalisierten Mono-, Di-, Oligo- und/oder Polysacchariden und/oder einem derart funktionalisierten Lactat oder Capronsäurederivat in Kombination mit einer monomeren oder hydrolytisch kondensierten Verbindung (I) mit a gleich 1 handeln, worin R¹ vorzugsweise eine Acryl- oder Methycrylgruppe aufweist, z.B. ω-(Meth)Acryloxyalkyl-trialkoxysilan. In solchen Varianten lassen sich die organische Verbindung und das Silan/Polysiloxan gemeinsam organisch polymerisieren. Solche Bandmaterialien können selbstverständlich weitere Zusätze enthalten, die unten näher erläutert sind, beispielsweise (bereits einkondensierte oder einkondensierbare) Silane der Formel (VI).

In einer zweiten Ausführungsform enthält das Badmaterial eine rein organische Verbindung mit einem über 2-Photonen- oder Mehrphotonen-Polymerisation polymerisierbaren Rest sowie ein organisch modifiziertes Polysiloxan, das durch Hydrolyse und mindestens teilweise Kondensation eines Ausgangsmaterials erhältlich ist oder erhalten wurde, welches mindestens ein Silan der Formel (la")

R¹ₐR²_{b}SiX_{4-a-b} (Ia")

sowie gegebenenfalls zusätzlich mindestens einer Verbindung des Typs

M^{III}(OR³)₃ (II)

und/oder des Typs

M^{IV}(OR³)₄ (III)

aufweist, und zusätzlich zu dem Polysiloxan oder an dessen Stelle mindestens eine noch nicht hydrolytisch kondensierte Verbindung (la") und gegebenenfalls Verbindungen des Typs (II) und/oder (III), worin R¹ gleich oder verschieden ist und einen organischen, über Zwei- oder Mehrphotonenpolymerisation polymerisierbaren Rest darstellt, R² ein biokompatibler, biodegradierbarer oder bioresorbierbarer Rest ist bzw. einen solchen aufweist, R³ eine Alkoxygruppe darstellt und X OH oder ein unter Hydrolysebedingungen hydrolytisch kondensierbarer Rest ist, der Index a 0, 1, 2 oder 3 bedeut0et, der Index b 1 oder 2 bedeutet und a+b zusammen 1, 2 oder 3 sind. Wenn a=0 ist, entstehen bei der Bestrahlung dieses Bades Produkte, die unabhängig voneinander ein organisches und ein anorganisches Netzwerk aufweisen, die ggf. interpenetrieren. Für a=1 wird die Siloxankomponente über die Polymerisation der Reste R¹ zusätzlich in das organische Netzwerk eingebunden.

In einer dritten Ausführungsform enthält das Badmaterial (mindestens) eine rein organische Verbindung mit einem biokompatiblen, biodegradierbaren oder bioresorbierbaren Rest sowie ein Organopolysiloxan, das durch zumindest teilweise hydrolytische Kondensation aus Verbindungen des Typs

R¹ₐR²_{b}SiX_{4-a-b} (I)

sowie gegebenenfalls zusätzlich mindestens einer Verbindung des Typs

M^{III}(OR³)₃ **(II)**

und/oder des Typs

M^{IV}(OR³)₄ (III)

erhältlich ist oder erhalten wurde, und/oder mindestens eine noch nicht hydrolytisch kondensierte Verbindung (I) und gegebenenfalls solche des Typs (II) und/oder (III), worin die Reste und Indices R¹, R², X und b wie oben für Formel (I) definiert sind, a 1, 2 oder 3 bedeutet und a+b zusammen 1, 2 oder 3 sind.

Es besteht alternativ auch die Möglichkeit, die drei genannten Bestandteile in Form eines Badmaterials einzusetzen, welches Silane der Formel (I) mit a=1, 2 oder 3 und Silane der Formel (Ia) bzw. ein Polykondensat aus mindestens diesen beiden Silanen enthält. Solche Materialien sind in DE 10 2008 057 684.0 offenbart, z.B. ein Polysiloxan, das an einem Teil der Siliciumatome Reste R¹ wie oben definiert trägt, z.B. Methacrylatreste (diese lassen sich, wie aus älterem Stand der Teichnik bekannt, u.a. durch Anbindung eines hydroxygruppenhaltigne Methacrylats wie Glycerin-1,3-dimethacrylat an ein Silylisocyanat, beispielsweise 3-Isocyanatopropyltriethoxysilan, erhalten), und an einem weiteren Teil der Siliciumatome Reste R² wie oben definiert trägt, beispielsweise eine über eine Säureamid-Kopplungsgruppe angebundene Aminosäure oder ein solches Peptid, erhältlich durch Reaktion einer Aminogruppe der Aminosäure oder des Peptids mit einem Silan, das eine aktivierte Bernsteinsäuregruppe enthält, beispielsweise (Triethoxysilyl)propylbernsteinsäureanhydrid.

Die physikalischen und chemischen Eigenschaften der auf die voranstehenden Methoden generierten Körper oder Schichten sind ausgezeichnet. Die Körper, Schichten oder deren Strukturen können dabei vom cm-Bereich bis in den sub-µm-Bereich generiert werden.

Die Herstellung von organisch modifizierten Polysiloxanen oder Kieselsäurekondensaten (häufig auch als "Silanharze" bezeichnet) und deren Eigenschaften ist in einer Fülle von Publikationen beschrieben worden. Stellvertretend sei hier z.B. auf Hybrid Organic-Inorganic Materials, MRS Bulletin 26(5), 364ff (2001) verwiesen. Ganz allgemein werden solche Substanzen in der Regel mit Hilfe des sogenannten Sol-Gel-Verfahrens hergestellt, indem hydrolyseempfindliche, monomere oder präkondensierte Silane, ggf. in Gegenwart weiterer cokondensierbarer Substanzen wie Alkoxiden des Bor, Germanium oder Titan, sowie ggf. von zusätzlichen Verbindungen, die als Modifikatoren oder Netzwerkwandler dienen können, oder von sonstigen Additiven wie Füllstoffen, einer Hydrolyse und Kondensation unterworfen werden.

Ganz besonders bevorzugt wird für das Badmaterial ein Material aus einem oder mehreren (ggf. zumindest teilweise hydrolytisch kondensierten) Silanen der Formel (I) eingesetzt, worin mindestens in einem Silan der Formel (I) R¹ gleich oder verschieden ist und einen organischen, über Zwei- oder Mehrphotonenpolymerisation polymerisierbaren Rest darstellt, der eine bzw. mehrere Gruppen enthält, die sich radikalisch polymerisieren lassen. Die Gründe sowie bevorzugte Ausgestaltungen dafür sind bereits weiter oben bei der Beschreibung dieser Reste erläutert.

Bevorzugt enthält der Rest R¹ in Formel (I) eine nichtaromatische C=C-Doppelbindung, besonders bevorzugt eine einer Michael-Addition zugängliche Doppelbindung. R² kann in allen Formeln der Erfindung eine unsubstituierte oder substituierte Alkyl-, Aryl-, Alkylaryl- oder Arylalkylgruppe sein, wobei die Kohlenstoffkette dieser Reste ggf. durch O, S, NH, CONH, COO, NHCOO oder dgl. unterbrochen sein kann. R² kann dabei auch Gruppen enthalten, die mit C=C Doppelbindungen eine Additionsreaktion eingehen können, beispielsweise SH-Gruppen. Die Gruppe X ist in der Regel Wasserstoff, Halogen, Alkoxy, Acyloxy oder NR³₂ mit R³ gleich Wasserstoff oder Niederalkyl. Alkoxygruppen sind als hydrolytisch kondensierbare Gruppen bevorzugt, insbesondere Niederalkoxygruppen wie C₁-C₆-Alkoxy.

Das verfestigbare Organopolysiloxan kann unter Verwendung mindestens eines weiteren Silans der Formel (IV)

SiX₄ (IV)

erzeugt worden sein, worin X gleich oder verschieden ist und die gleiche Bedeutung wie in Formel (I) besitzt. Eine hierfür gut einsetzbare Verbindung ist Tetraethoxysilan. Durch Zugabe solcher Silane zu der zu hydrolysierenden und kondensierenden Mischung, aus der schließlich das polymerisierbare Badmaterial entsteht, wird der SiO-Anteil des Harzes, also der anorganische Anteil, erhöht. Die Biodegradierbarkeit dieser Materialien steht nicht im Vordergrund; sie sind jedoch z.B. als Scaffolds einsetzbar.

Umgekehrt kann das erfindungsgemäß organisch zu polymerisierende Siloxanpolykondensat unter Verwendung mindestens eines Silans mit der Formel (V)

R¹ₐSiR²₄₋ₐ (V)

hergestellt worden sein, worin R¹ und R² die oben für Formel (I) angegebene Bedeutung haben. Dadurch wird der Vernetzungsgrad des Polykondensates heruntergesetzt, die Abbaubarkeit nimmt zu.

Die Mischung, aus der das Silankondensat erzeugt wird, kann weiterhin mindestens ein Silanol der Formel (VI)

R⁴ₐSi(OH)₄₋ₐ (VI)

enthalten, worin R⁴ gleich oder verschieden sein kann und jeweils die Bedeutung von R¹ wie in Formel (I) definiert oder von R² wie in Formel (I) definiert besitzt oder ein geradkettiges, verzweigtes oder cyclisches Alkyl mit vorzugsweise 1 bis 10 Kohlenstoffatomen oder ein Aryl mit vorzugsweise 6 bis 20 Kohlenstoffatomen ist und worin der Index a 1, 2 oder 3, vorzugsweise 2 bedeutet. Ganz besonders bevorzugt sind Verbindungen (VI) mit R⁴ gleich Phenyl und/oder a = 2. Silanole werden ohne Wasserbildung in das anorganische Netzwerk einkondensiert. Die Hydrolyse kann in Gegenwart dieser Verbindungen daher mit Hilfe von katalytisch wirksamen Mengen von Wasser erfolgen; im Übrigen kann das System wasserfrei bleiben. In einer bevorzugten Ausgestaltung der Erfindung werden Disilanole der Formel (VI) im Mischungsverhältnis von 1:1 (Mol/Mol) mit Silanen der Formel (I), die vorzugsweise mindestens eine Gruppe R¹ enthalten, als zu hydrolysierendes und kondensierendes Ausgangsmaterial eingesetzt.

Wenn R¹ in Formel (I) eine C=C-Doppelbindung trägt (oder wenn eine rein organische Verbindung mit einem eine solche Doppelbindung tragenden Rest im Badmaterial vorhanden ist) und R² in dieser Formel nicht vorhanden (d.h. b = 0) ist oder keine funktionellen Gruppen besitzt, kann in einer spezifischen Ausgestaltung dem zu hydrolysierenden und kondensierenden Material mindestens ein Silan der Formel (VII)

R⁵ₐSiX₄₋ₐ (VII)

zugegeben werden, worin R⁵ eine Gruppe trägt, die radikalisch an eine C=C-Doppelbindung addiert werden kann. Entsprechende Kondensate sind dann einer Polymerisation durch Additionsreaktionen der Gruppen R⁵ der Silane der Formel (VII) an Doppelbindungen der Reste R¹ der Silane mit der Formel (I) zugänglich.

Die für die Zwecke der vorliegenden Erfindung zu hydrolysierende und kondensierende Mischung kann weitere Substanzen enthalten, z.B. vorzugsweise niedere Alkoxide, insbesondere C₁-C₆-Alkoxide, von Metallen der III. Hauptgruppe, von Germanium und von Metallen der II., III., IV., V., VI., VII. und VIII. Nebengruppe. Ein Teil dieser Verbindungen ist bereits oben als Metallverbindungen (II) und (III) erwähnt.

Insgesamt sollte das organisch modifizierte Kieselsäurepolykondensat, aus dem erfindungsgemäß durch Zwei- oder Mehrphotonenpolymerisation Körper verfestigt werden können, vorzugsweise mindestens 10 Mol-% an einer Zweiphotonenpolymerisation zugänglichen Gruppen (als R¹ der Formel (I) oder in der rein organischen Verbindung) aufweisen, bezogen auf die Molmenge an Siliciumatomen plus ggf., soweit vorhanden, der Metallatome der III. Hauptgruppe, von Germanium und der II., III., IV., V., VI., VII. und VIII. Nebengruppe (die die Referenz von 100 Mol-% bilden).

In einer bevorzugten Ausgestaltung der Erfindung wird die Flüssigkeit des Bads mittels Bestrahlung mit Femtosekunden-Laserpulsen verfestigt. Als hierfür geeignete Laser lassen sich vorzugsweise Ti-Saphir-Laser einsetzen (entweder mit der fundamentalen Wellenlänge von 780 nm oder, je nach Absorptionsverhalten der zu erhärtenden Flüssigkeit, mit der zweiten Harmonischen bei 390 nm); auch andere NIR-Laser (z.B. mit emittierten Wellenlängen von 800 nm bis etwa 1500 nm) sind geeignet. Aber auch andere Laserbestrahlung ist möglich, sofern die eingesetzte Lichtquelle mit einer Intensität in das Bad einstrahlen kann, die für eine Mehrphotonenanregung ausreichend bzw. geeignet ist. Diese Eigenschaft bieten bei moderaten mittleren Leistungen insbesondere Kurzpulslaser. Das auszuhärtende Material muss transparent für die verwendete Laserwellenlänge sein. Wenn das zu verfestigende Material beispielsweise bei 390 nm einer Einphotonen-Polymerisation zugänglich wäre, kann jede Wellenlänge von 400 nm oder darüber für die Zwei- oder Mehrphotonenpolymerisation eingesetzt werden; je nach Harz sind wegen der Transparenzbedingungen vor allem 500-1000 nm geeignet. Bei Verwendung größerer Wellenlängen kann die Polymerisation auch durch eine n-Photonenabsorption initialisiert werden, wobei n größer 2 gilt. Die Schwellen-Fluenz, bei welcher der Potymerisationsprozess startet, kann durch die Wahl geeigneter Komponenten, wie z.B. Co-Initiatoren und/oder Aminkomponenten mit einem erhöhten Mehrphotonen-Absorptionsquerschnitt im Harz gesenkt werden. Dadurch wird das Prozessfenster, in dem die Polymerisation stattfindet, vergrößert.

Insbesondere bei Einsatz von Femtosekundenlasern als Strahlungsquelle erhält man Körper/Schichten mit einer drastisch erhöhten lithographischen Auflösung ohne unerwünschte Nebeneffekte der Strukturierung, wie beispielsweise Kantenverrundung, Inhibierung oder geringer lithographischer Auflösung. Dabei kann auf die herkömmliche, teilweise aufwendige Präparation von Schichten, welche üblicherweise zur Strukturierung verwendet wird, verzichtet werden. Die Erzeugung echter, formgenauer dreidimensionaler Strukturen und Komponenten für optische, (di)elektrische, magnetische, mechanische, biochemische und medizinische Anwendungen wird dadurch erheblich präziser, schneller und zuverlässiger. Art und Dauer der Bestrahlung liefern darüber hinaus die Möglichkeit, den Vernetzungsgrad gezielt zu variieren, so dass mit ein und demselben Material bei Bedarf unterschiedliche physikalische Eigenschaften (z.B. unterschiedlich schnelle Abbaubarkeit) erzielt werden können. Im Unterschied zur dreidimensionalen Strukturierung durch Prägen können mit diesem Verfahren nicht nur dreidimensionale Strukturen auf Substraten (Trägermaterialien) gefertigt werden, sondern es können dreidimensionale freitragende Körper vollständig aus dem Volumen heraus gefertigt werden.

Die 2-Photonenpolymerisation bewirkt dabei eine Polymerisation der organischen, dieser Reaktion zugänglichen Reste; darüber hinaus kann damit in selteneren Fällen auch noch eine weitere hydrolytische Kondensationsreaktion noch nicht hydrolysierter Reste X an den Siliciumatomen bewirkt oder diese sogar vervollständigt werden, sofern das Bad noch solche Reste enthält.

Zur Initiierung der Vernetzungsreaktion kann den erfindungsgemäßen physiologisch degradierbaren Materialien ein geeigneter Initiator zugesetzt werden. Insbesondere können die Vernetzungsreaktionen aber auch ohne Initiator durchgeführt werden, so dass auch auf Komponenten mit bedingt toxischem Potential gänzlich verzichtet werden kann.

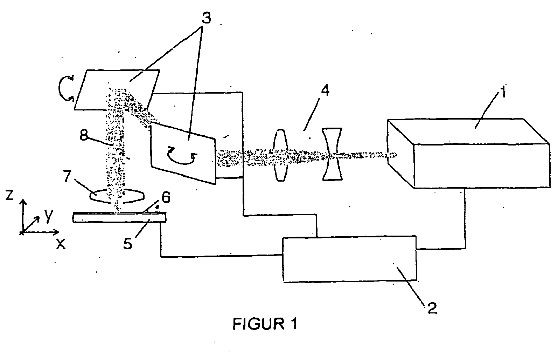

Figur 1 zeigt beispielhaft eine Anordnung zur Erzeugung dreidimensionaler Strukturen: Als Strahlquelle (1) dient ein Laser, z.B. ein gepulster Laser für die Erzeugung ultrakurzer Laserpulse (Pulsdauer 10 ps bis 10 fs). Um die Qualität des Laserfokus zu kontrollieren, können strahlformende Optiken (4) zum Einsatz kommen (z.B. eine Strahlaufweitung für einen kleinstmöglichen Fokusdurchmesser). Zwei computergesteuerte, rotierende Spiegel (3) werden eingesetzt, um den Laserstrahl in definierter Weise in x- und y-Richtung abzulenken. Der Laserstrahl (8) wird mit Hilfe einer fokussierenden Optik (7) in das zu polymerisierende Harz (5) fokussiert. Bei dieser Optik handelt es sich um eine einzelne Linse oder um Linsensysteme, z.B. Mikroskop-Optiken. Das zu polymerisierende Harz befindet sich unter oder auf einem transparenten Substrat (6). Das Harz kann entweder durch das Substrat (6) bestrahlt werden (siehe Skizze), das in diesem Falle eine hohe optische Oberflächengüte aufweisen sollte, oder es kann sich auf der Oberseite des Substrates befinden und direkt bestrahlt werden (ohne Abbildung). Das Substrat mit dem Harz kann mit Hilfe von computergesteuerten Positioniersystemen in x-, y- und z-Richtung in definierter Weise bewegt werden. Dies, zusammen mit den rastemden Spiegeln, ermöglicht es, den Laserfokus in drei Dimensionen durch das Harz bzw. das Harz durch den Fokus zu bewegen. Alternativ kann die fokussierende Optik (7) in z-Richtung bewegt werden, um den Fokus in z-Richtung durch das Harz zu bewegen. Die Komponenten Laser (1), Scanner (3), sowie alle Positioniereinheiten werden von einem PC (2) gesteuert und kontrolliert.

Ein besonderer Vorteil des im Rahmen der vorliegenden Erfindung aufgefundenen Verfahrens ist es, dass dreidimensionale Körper mit einer enormen Flankensteilheit, einem hohen Aspektverhältnis und einer sehr guten Auflösung erzeugt werden können, so dass beliebige dreidimensionale Strukturen, sei es in Form von (ggf. selbsttragenden) Körpern, sei es in Form von oberflächenstrukturierten oder sonstigen Schichten, die ggf. von einem Substrat gehalten werden, generiert werden können. Das bedeutet, dass auch Hinterschnitte oder Hohlräume realisiert werden können.

Die Begrenzung der lithographischen Auflösung bei Verwendung optischer Lithographie wird dabei nicht durch das Material selbst bestimmt (mit Ausnahme solcher Systeme, die möglicherweise einer parasitären Polymerisation unterliegen können, siehe oben), sondern durch die üblicherweise für die Strukturierung angewendeten Verfahren, wie z.B. Belichtung durch eine Maske unter *Proximity-*Bedingungen; eine Kontaktbelichtung wäre lediglich unter Inkaufnahme einer Verschmutzung der verwendeten Masken aufgrund der sehr guten Haftung der Organopolysiloxane zu fast allen Materialien oder durch Verwendung teurer, speziell zu diesem Zweck beschichteter Masken möglich. Darüber hinaus gibt es bei der UV-Strukturierung von speziellen Organopolysiloxanen den Nachteil aller radikalisch polymerisierbaren Systeme, dass sich bei Anwesenheit von Sauerstoff und je nach verwendetem Photoinitiator eine Inhibierungsschicht in Oberflächennähe ausbildet, welche in diesen Bereichen die UV-Vernetzung des Materials behindert. Dies ist je nach verwendetem Heteropolysiloxan mehr oder weniger stark ausgeprägt, lässt sich jedoch bisher ohne besondere Vorkehrungen schwer vermeiden. Alternativ kann unter Ausschluss von Sauerstoff unter Stickstoffexposition belichtet werden, was einerseits hinsichtlich der Inhibierungsschicht zum Erfolg führt, andererseits jedoch an der Auflösungsbegrenzung (*Proximity*) nichts ändert sowie auch apparativ erheblich aufwendiger ist. Daher ist ein weiterer Vorteil der erfindungsgemäßen Strukturierung mit Hilfe von Organopolysiloxanen, dass die Körper und/oder Oberflächen Komponenten aus dem Volumen heraus, d.h. vollkommen unter Ausschluss von Luftsauerstoff bzw. nur noch bei Anwesenheit des im Harz/Lack gelösten Sauerstoffs und unter Verzicht einer Stickstoffspülung, strukturiert werden können.

Wie erwähnt, sind Harze auf Organopolysiloxanbasis Materialien, die in einer großen Vielzahl und Vielfalt in Hinblick auf verschiedene physikalische, chemische und biologische Eigenschaften ausgewählt werden können, da sie eine Vielzahl verschiedener funktioneller Gruppen tragen können, welche die physikalischen und chemischen Eigenschaften des Harzes beeinflussen (z.B. Netzwerkbildner, Netzwerkwandler). Daher sind diese Harze für eine Anwendung in den bezeichneten Gebieten von besonderem Vorteil. Vor allem die erfindungsgemäß bevorzugte Nutzung von Femtosekundenlaser-Bestrahlung von Silanharzen zur Strukturierung und Herstellung dreidimensionaler Strukturen, sei es in Form von (ggf. selbsttragenden) Körpern, sei es in Form von oberflächenstrukturierten oder sonstigen Schichten, die ggf. von einem Substrat gehalten werden, für biochemische und medizinische Anwendungen ermöglicht es, die strukturellen Eigenschaften der Materialien mit hoher Auflösung zu nutzen. Grundlage des erfindungsgemäßen Polymerisationsprozesses ist, wie erwähnt, die Zwei-Photonen-Polymerisation, wobei die Erfinder die Feststellung machen konnten, dass der Wechselwirkungsquerschnitt (die Wahrscheinlichkeit der 2P-Absorption) der erfindungsgemäß einsetzbaren Organopolysiloxane hinreichend groß ist, um dieses Verfahren zur Erzeugung dreidimensionaler Strukturen, sei es in Form von (ggf. selbsttragenden) Körpern, sei es in Form von oberflächenstrukturierten oder sonstigen Schichten, die ggf. von einem Substrat gehalten werden, einzusetzen. Die erfindungsgemäß bevorzugte organische Polymerisation von Organopolysiloxanen mit der Zwei-Photonen-Polymerisation sollte darüber hinaus die Möglichkeit bieten, eine Initialisierung des Polymerisationsprozesses ohne UV-Initiator durchführen zu können, welches im wesentlichen von der eingestrahlten Leistungsdichte (→ notwendige Temperaturen dafür im Bereich bis 200 °C, weit unter der typischen Temperaturstabilität bis 270 °C) abhängen sollte.

Bei den in der vorliegenden Erfindungsmeldung beschriebenen Materialien ist es möglich, dreidimensionale Formkörper mit einem durchgängigen porösen Netzwerk innerhalb des Formkörpers großflächig durch Licht-induzierte Vernetzungsprozesse, insbesondere durch die Mehr-Photonen-Absorptions-Technologie über einen weiten Wellenlängenbereich unter Verwendung verschiedenster Laser- und optischer Systeme *in-situ* herzustellen. Insbesondere lassen sich mit dem beschriebenen Verfahren große Strukturen und Formkörper mit einer Größe bis in den cm-Bereich herzustellen.

Die vorliegende Erfindung eignet sich auch für ein Verfahren zur Herstellung von Körpern zur *in vitro*-Vermehrung von Zellen, wobei die Körper die Form einer dreidimensionalen Trägermatrix aufweisen, die als Zellstützsubstanz und/oder Leitstruktur für die von den Zellen gebildete extrazelluläre Matrix dient bzw. den Zellen die Möglichkeit gibt, eine räumliche Anordnung zu finden, die es den Zellen erlaubt, sich zu vermehren und/oder ihre genetisch determinierte Differenzierung zu erreichen. Die Zellen können nachträglich auf die Trägermatrix zur Vermehrung aufgebracht und kultiviert werden, alternativ aber auch wie andere biologische Moleküle (z.B. Proteine wie Kollagen, Enzyme, Peptide, Aminosäuren, Antikörper, Wachstumsfaktoren) bereits in der Phase des Herstellungsprozesses der Trägermatrix Bestandteil der flüssigen Materialvorstufe (des Badmaterials) sein. Die Erfindung betrifft auch derartige Körper mit aufgebrachten Zellen.

Als Zellen können beispielsweise gentechnisch veränderter oder nativer differenzierter Zellen verschiedener Differenzierungsarten und -grade verwendet werden.

Die auf die Trägermatrix aufzubringenden Zellen heften sich an die Matrix an oder vermehren sich vornehmlich zweidimensional auf dieser Matrix, wobei sie zusammen eine extrazelluläre Matrix bzw. Botenstoffe (Hormone) bilden können. Die Trägermatrix ist vorzugsweise porös, so dass die ein-/aufgebrachten Zellen sie penetrieren, eine dreidimensionale Verteilung annehmen und entsprechend ihrer genetisch determinierten oder durch hinzu gegebene Differenzierungsfaktoren induzierten Differenzierung ein räumliches Gewebe- und Organwachstum auslösen können bzw. Botenstoffe freisetzen. Die Porenstrukturen werden in Abhängigkeit von den Zelltypen gewählt, die auf die Trägermatrix aufzubringen sind; sie haben in der Regel durchschnittliche Durchmesser zwischen 2 µm und 1 mm, bevorzugt zwischen 20 und 500 µm, und häufig zwischen 40 und 250 µm. In einer alternativen Ausführungsform der Erfindung ist die Matrix als dichtes, von den ein-/aufgebrachten Zellen nicht penetrierbares Volumenmaterial mit der Möglichkeit der zweidimensionalen Zellverteilung und der gleichzeitigen Möglichkeit eines dreidimensionalen Gewebe- und Organwachstums im Sinne eines "Composite grafts" ausgebildet.

Ein bevorzugter Erfindungsgegenstand betrifft einen Zellenverbund, Gewebe und/oder Organe, herstellbar nach dem oben beschriebenen Verfahren. Ein solcher/s Zellverbund, Gewebe und/oder Organe eignet sich beispielsweise als *in vitro*-Modell für Medikament-Gewebe-Organinteraktionen. Zur Herstellung von Geweben außerhalb des menschlichen Körpers kommen vielfältige Verfahren zur Anwendung, die unter dem weitläufigen Begriff "tissue engineering" zusammengefasst werden. Hierzu werden je nach Gewebeart Zellen aus ihrem bestehenden Gewebeverbund isoliert und zur Vermehrung gebracht. Hiernach werden die Zellen entweder auf flächige Materialien unterschiedlicher Konsistenz aufgebracht oder in poröse bzw. Gel-artige Materialien eingebracht, hierdurch die Gewebereifung induziert und gegebenenfalls durch Differenzierungsfaktoren stimuliert. Die Gewebereifung kann außerhalb oder innerhalb des-Körpers erfolgen. Die erfindungsgemäße Trägermatrix hat dabei den Vorteil, dass sie biologisch degradierbar und/oder biologisch resorbierbar ist. Beispielsweise degradiert und/oder resorbiert die erfindungsgemäße Trägermatrix bei einer solchen Ausführungsform vorzugsweise erst nach Auf-/Einbringen des Zellverbundes, Gewebes und/oder Organs auf/in einen tierischen oder menschlichen Körper.

Je nach Zellart müssen die Zellen entweder zuvor durch enzymatischen Verdau oder durch mechanische Trennung aus ihrem Matrixverbund gelöst werden oder durch Aufoder Einbringen auf/in ein Nährmedium unter physiologischen Bedingungen zum Wachstum angeregt werden. Die oben genannte Trägermatrix fungiert hierbei als Leitstruktur für das Zellwachstum oder als Leitstruktur für die Akkumulation extrazellulärer Matrix- und Gewebebestandteile. Erfindungsgemäß kann das Trägermaterial in verschiedenen Anordnungen zur Anwendung kommen. Welche Anordnung zu wählen ist, weiß der Fachmann an Hand des herzustellenden (Zell-) Gewebes zu bestimmen. Die in Betracht kommenden Anordnungen sind die Folgenden:
1. Als Flächenelement, d.h. als dichter Formkörper, das zwar eine über die Dimension der aufgebrachten Zellen hinausgehende, aber doch nur eine begrenzte Penetration ermöglicht (d.h. die durchschnittliche Größe der Löcher ist keinesfalls größer, bevorzugt sogar kleiner als die durchschnittliche Größe der zu kultivierenden Zellen; somit können die Zellen zwar einwachsen, dies aber nur derart, dass sie auf der Unterlage gut haften), mit der im Wesentlichen alleinigen, zumindest aber vornehmlichen Möglichkeit der zweidimensionalen Zellverteilung und einem flächigen Zell-, Gewebe- und Organwachstum. Dies lässt sich z. B. auch durch klassische UV-Lithographie generieren.
2. Als dreidimensionales Raumelement, d.h. als poröses, von den Zellen penetrierbare Trägermatrix (d.h. die durchschnittliche Größe der Löcher ist keinesfalls kleiner, bevorzugt sogar größer als die durchschnittliche Größe der zu kultivierenden Zellen) mit der Möglichkeit der dreidimensionalen Zellverteilung und einem räumlichen Zell-, Gewebe- und Organwachstum. Dies lässt sich bevorzugt mit der Mehr-Photonen-Absorptionstechnologie *in situ* herstellen.
3. Als Kombination von 1) und 2) im Sinne eines "composite graft" oder Organes durch Kombination von Zellen, Geweben bzw. Organen und Oberflächen-Hüllgewebe (z.B. Organkapsel); diese Variante kommt in Betracht für Gewebestrukturen, die aus mehreren Zellarten zusammengesetzt sind. Beispielsweise bestehen Gefäße aus Endothel und Bindegewebe, wobei das Endothel mit flächiger Struktur zur Auskleidung eines Blutgefäßes dient, während das Bindegewebe als Stützsubstanz des Gefäßes fungiert und die dreidimensionale Hohlstruktur bildet. Durch die Kombination von 1) als Flächenelement für das Wachstum von Endothel und 2) als dreidimensionales Raumelement für das Wachstum von Bindegewebe kann letztendlich ein Gefäß rekonstruiert werden.

Nachfolgend werden einige Gewebe- bzw. Zelltypen aufgelistet, die für die Vermehrung/Herstellung mittels einer der drei Varianten besonders geeignet sind und demnach erfindungsgemäß bevorzugt sind.

Für die Anwendung 1) vorzugsweise die folgenden Gewebe: Epithel, Endothel, Urothel, Mukosa, Dura, Bindegewebe; und vorzugsweise Chondrozyten (Knorpel; zur Chondrozyten-Vermehrung braucht man ein zweidimensionales Medium, zur Chondrozyten-Differenzierung und Knorpelmatrix-Bildung hingegen braucht man ein dreidimensionales Medium. Hier sind bezüglich Knorpel nur die Zellen gemeint, wenn sie dedifferenzieren und sich vermehren. Die Differenzierung folgt in Anwendung 2), Osteozyten (Knochen; entweder zwei oder dreidimensional, hier gilt dasselbe wie für die Chondrozyten), Nervenzellen (Nerven), Haarzellen (Innenohr-Hörorgan) oder deren Vorläuferzellen.

Für die Anwendung 2) die folgenden Zellen: die für Anwendung 1) beschriebenen Zellen nach ihrer flächigen Vermehrung, organspezifische Zellen (z.B. Hepatozyten, Nephrozyten, Kardiomyozyten, Pankreozyten), Zellen des ZNS mit/ohne endokrine/r Funktion, z. B. Netzhaut, Neurozyten, Zirbeldrüse, dopaminerge Zellen, Gefäßbildende Zellen (z. B. Angiozyten), Zellen mit endo- oder exokriner Funktion (z. B. Inselzellen, Nebennierenzellen, Speicheldrüsenzellen, Epithelkörperchen, Thyrozyten), Zellen des Immunsystems (z. B. Makrophagen, B-Zellen, T-Zellen oder deren Vorläuferzellen). Die Zellen des Immunsystems werden dreidimensional gezüchtet, weil sie im Gewebe, nach Penetration der Blut-Gewebeschranke auf ein dreidimensionales Gerüst je nach Gewebeart) treffen und dort im Dreidimensionalen ihre Wirkung entfalten.

Für die Anwendung 3) die folgenden Zeilen/Gewebe/Organe: u. a. Trachea, Bronchien, Gefäße, Lymphgewebe, Harnröhre, Harnleiter, Niere, Blase, Nebenniere, Leber, Milz, Herz, Gefäße, Schilddrüse, Tonsillen, Speicheldrüsen, Gehirn, Muskel (glatt, quergestreift), Bandscheiben, Meniskus, Herz, Lunge, Galle, Speiseröhre, Darm, Auge, Ohr.

Eine weitere Anwendungsmöglichkeit des in der Erfindung zur Anwendung kommenden Materials ist die Besiedlung des Materials mit Zellen, die eine endo- bzw. exokrine Funktion besitzen und Wirkstoffe (z.B. Hormone, Interleukine, Entzündungsmediatoren, Enzyme) freisetzen, die eine Wirkung im Organismus oder außerhalb davon entfalten. Das heißt, das erfindungsgemäß verwendete Material kann, bei seiner Besiedelung mit Zellen mit endo-oder exokriner Funktion, innerhalb, aber auch außerhalb des Körpers zur Herstellung der o.g. Wirkstoffe dienen, die dann über bekannte Verfahren als Arzneimittel dem Körper zur Verfügung gestellt werden. Eine außerhalb des Körpers entfaltete Wirkung kann dazu dienen, Gewebe oder Zellen mit der freigesetzten Substanz zu beeinflussen.

Eine weitere Verwendung der Trägermatrix ist eine solche als physiologisch degradierbar und/oder bioresorbierbares Bio-Implantat als Leitschiene für die körpereigene Wundheilung unter oder auf Niveau der Haut, Schleimhaut bzw. im Körperinneren im Rahmen von Operationen an Organen und Geweben. Hierzu wird das Material falls möglich durch einen Arzt als Flächenelement oder dreidimensionales Raumelement direkt oder zusammen mit weiteren Substanzen in die Wunde oder Organe/Gewebe, beispielsweise während einer Operation, eingebracht. Darüber hinaus kann die Trägermatrix zusätzlich mit weiteren Wirkstoffen unterschiedlicher Substanzgruppen versetzt werden mit der Möglichkeit einer positiven Beeinflussung der Gewebe- und Organdifferenzierung durch Entfaltung einer aktiven und passiven Wirkung am Ort der Anwendung, aber auch durch Wirkungsentfaltung an einem entfernt liegenden Wirkort. Hierzu zählen die oben genannten therapeutisch aktiven Wirkstoffe insbesondere einerseits antiinfektiöse Wirkstoffe, andererseits aber auch die Wundheilung, die Entzündungsreaktion sowie die Gewebedifferenzierung unterstützende und modulierende Wirkstoffe wie beispielsweise einerseits Wachstumsfaktoren (IGF, TGF, FGF etc.), andererseits Glukocortikoide und Interleukine, aber auch Chemotherapeutika und Immunsuppressiva.

Ein weiterer Aspekt der vorliegenden Erfindung betrifft die Verwendung der erfindungsgemäßen Zellen, Organe und Gewebe, nachdem sie mit Medikamenten und/oder Wirkstoffen versetzt worden sind, als in vitro-Modell für Medikament-Gewebe-Organinteraktionen. Hierdurch können tierexperimentelle Untersuchungen minimiert bzw. auch ganz vermieden werden.

Für alle vorgenannten Einsatzgebiete, d.h. die Verwendung als individualisierte, in der Regel (aber nicht zwingend) bioresorbierbare Trägerstrukturen in vivo (als Implantat) oder *in vitro* (siehe oben: Schlagwort "tissue engineering"), ist eine möglichst physiologische Zellbesiedelung (durch menschliche oder tierische, körpereigene Zellen oder durch entsprechende, *in vitro* zugegebene Zellen) wünschenswert. Die Zelladsorption an der Trägerstruktur kann dabei gezielt gefördert werden, da Zellen in der Regel über Membranproteine, sogenannte Integrine, an Oberflächen binden und dabei wiederum gewisse funktionelle Gruppen wie Amino oder Carboxygruppen als Bindungspartner bevorzugen. Entsprechende oder andere geeignete Funktionalitäten auf den Oberflächen der erfindungsgemäßen Körper können auf unterschiedliche Weise zur Verfügung gestellt werden: In situ durch eine geeignete Auswahl der organischen Komponenten, beispielsweise an den organisch modifizierten Alkoxysilanen, oder durch biologische Komponenten wie Aminosäuresequenzen oder Proteine, die in den Herstellungsprozess dieser Körper, d.h. in das Badmaterial integriert werden. Eine weitere Möglichkeit ist die nachträgliche biologische oder biochemische Funktionalisierung oder Oberflächenmodifizierung der strukturierten, ausgehärteten Matrix. Die Wechselwirkungen zwischen dem erfindungsgemäßen Matrixmaterial und den biologischen Komponenten der physiologischen Umgebung können durch
- Chemisorption (chemische Bindungen, wie kovalente, ionische, Dipol-Dipol-Wechselwirkungen oder auch Mischformen dieser Bindungsarten),
- Physisorption (durch Van-der-Waals-Wechselwirkungen) oder
- Mischformen beider Anbindungsarten
zustandekommen.

Ebenso können in die Matrix Wirkstoffe wie Wachstumsfaktoren integriert werden, die die Ausbildung von gewebeähnlichen Strukturen aus adhärierten Zellen fördern.

Darüber hinaus ist ein zusätzlicher Aspekt der vorliegenden Erfindung die Verwendung als Monolith. Massive Implantate in beliebiger dreidimensionaler Form können als Drug Delivery-Systeme, z. B. von Flüssigarzneimitteln eingesetzt werden und beispielsweise subkutan Verwendung finden. Dabei ist es wichtig, dass die den Wirkstoff umgebende Matrix ebenfalls nicht toxisch ist und keine Reaktionsprodukte mit dem Wirkstoff bildet.

Die Erfindung soll nachstehend anhand von Ausführungsbeispielen näher erläutert werden.

### Allgemeines Beispiel Herstellung eines Badmaterials aus hydrolytisch kondensierten Silanen und acrylierten oder methacrylierten organischen Verbindungen

Aus Methacryloxypropyl-trimethoxisilan und Diphenylsilandiol im Molverhältnis 1:1 wurde ein anorganisch-organisches Hybridpolymer hergestellt wie in WO 01/04186 A1 beschrieben. Als Katalysator für die hydrolytische Kondensation diente Ba(OH)₂ oder auch Ba(OH)₂·8H₂O.
Die wie unten in den Beispielen 1 bis 5 hergestellten acrylierten und methacrylierten Zielverbindungen wurden in unterschiedlichen Molverhältnissen (3:1, 1:1, 1:2 und 1:3) mit diesem Hybridpolymer versetzt und bis zu 30 Minuten im Ultraschallbad bei 45 kHz behandelt. Anschließend wurden 2 Gew.-% Photoinitiator (Irgacure^{®} 369, Irgacure^{®} Oxe01, Irgacure^{®} Oxe02 oder dgl.) zugegeben und dieser für weitere bis zu 30 Minuten im Ultraschallbad bei 45 kHz gelöst.

### Allgemeines Beispiel Strukturierung mit Hilfe von Mehrphotonenpolymerisation

Das voranstehend beschriebene Hybridpolymer ist bereits anorganisch vemetzt. Durch die nachstehend näher erläuterte Strukturierung mittels TPA (Mehrphotonen-Polymerisation) werden die genannten Zielverbindungen zusammen mit dem Hybridpolymer weiter organisch vernetzt, sodass neue Hybridpolymere mit anorganischen und organischen Vernetzungskomponenten entstehen.

Die auf dem Prinzip der Mehrphotonenpolymerisation beruhende Maschine erlaubt es, dreidimensionale CAD-Files ohne zusätzliche Arbeitsschritte (wie z. B. Abformung, Ätzschritte, Sintern) in das gewünschte reale Funktionselement umzuwandeln. Hierzu wird ein fokussierter Ultrakurzpulslaser verwendet, der das auf eine zähflüssige Konsistenz gebrachte Badmaterial (das nachstehend auch als "Harz" bezeichnet wird) zeilenweise und schichtweise gemäß dem gegebenen CAD-File abrastert und so eine durch den Laserfokus räumlich definierte Polymerisation (Verfestigung) einleitet. Je nach Parametereinstellungen und Größe der Probe dauert deren Herstellung wenige Minuten bis zu mehreren Stunden. Für sehr kleine Strukturen werden Schreibgeschw indigkeiten kleiner 0.5 mm/s und Belichtungsleistungen kleiner 1 mW in Kombination mit einem 100x Immersionsöl-Objektiv mit einer NA (NA = Numerische Apertur) von 1.4 verwendet, wodurch Auflösungen um 100 nm realisiert werden können. Größere Strukturen im Millimeter- und Zentimeterbereich können mit Schreibgeschwindigkeiten von bis zu 40 mm/s und Belichtungsleistungen von bis zu 50 mW hergestellt werden. Dabei werden Objektive mit mittleren NAs (z. B. 0.45) und großen Arbeitsabständen verwendet, welche Auflösungen von wenigen Mikrometern gewährleisten. Der nach der Belichtung folgende Entwicklungsschritt in einer 50/50-Lösung von Methylisobutylketon (MIBK) und Isopropanol dient der Entfernung des nicht verfestigten Harzes und benötigt je nach Größe und Komplexität der Struktur zwischen 5 und 30 Minuten. Zuletzt wird die Probe mit Isopropanol abgespült und für etwa 10 bis 30 Minuten getrocknet.

### Allgemeines Beispiel Degradationstudie

Einige Materialformulierungen wurden unter Bestrahlung durch UV-Licht der Wellenlängen 254 nm und 366 nm für 1 h in einer Teflonform ausgehärtet. Die so entstandenen Stäbchen (25 x 2 x 2 mm³) wurden gewogen, in 1,5 ml PBS-Lösung (phosphatgepufferte physiologische Salzlösung mit einem pH-Wert von annähernd 7,4) eingelegt und jeweils nach 1 d, 3 d, 7 d, 14 d, 21 d und 35 d und anschließend alle 28 Tage entnommen. Die Proben wurden mit Reinstwasser abgespült, für 1 h bei 110°C getrocknet und 30 min bei Zimmertemperatur zum Auskühlen stehen gelassen. Anschließend wurden die Stäbchen nochmals gewogen und der prozentuale Gewichtsverlust in Abhängigkeit der Zeit bestimmt. Die PBS-Lösung wurde im Schnitt alle 3 Tage ausgewechselt, um eine Sättigung der Lösung zu verhindern. Bei jeder Entnahme und jedem Lösungswechsel wurde der pH-Wert gemessen, um so Hinweise auf freigesetzte Säuren zu erhalten.

### Beispiel 1 Funktionalisierung von 1,2:5,6-Di-O-isopropyliden-α-D-glucofuranose mit Acrylsäureethylester und 2-Photonenpolymerisation unter Verwendung des Produkts

### 1.1 Herstellung von (0,2 mmol) 6-O-Acroyl-1,2:5,6-Di-O-isopropyliden- α-D-glucofuranose

In einem 50 ml Schlenckkolben mit Mikrodestille wurden unter Stickstoff-Atmosphäre 0,60 g (4,83 mmol) *para*-Methoxyphenol, 2,93 g (11,3 mmol) 1,2:5,6-Di-*O-*isopropyliden-α-D-glucofuranose, 18 ml (25,3 g, 282,74 mmol) Acrylsäureethylester und 0,60 g (2,03 mmol) Tetra(isopropyloxy)titan gegeben und die rote Reaktionslösung auf 112°C erhitzt. Nach 40 Minuten wurde bei 74-75 °C ein farbloses Acrylsäureethylester-Ethanol-Azeotrop abdestilliert. Nach zwei Stunden wurde die Lösung kurz an Luft abgekühlt und 11 ml Petrolether (Sdp. 30-50°C) und 2,2 ml Wasser zugegeben, wobei über Nacht ein gelber Niederschlag ausfiel. Der Niederschlag wurde 10 Minuten bei 4000 U·min⁻¹ abzentrifugiert, zwei Mal mit je 15-20 ml Pentan gewaschen und erneut abzentrifugiert. Die organischen Lösungen wurden vereinigt und destillativ entfernt bis ein blass gelber Feststoff zurück blieb. Dieser Feststoff wurde erneut in etwa 30 ml Petrolether gelöst und drei Mal mit je 27 ml Wasser, drei Mal mit je 0,5 M Natronlauge gewaschen. Das Lösungsmittel wurde erneut destillativ entfernt und der blass gelbe bis weiße Feststoff im Ölpumpenvakuum getrocknet.
Ausbeute: 1,01 g (3,21 mmol, 28,4%)
¹H-NMR (CDCl₃, 23°C, 400,1 MHz, [ppm]): δ =1,30 (s, 3 H, C*H₃*), 1,41 (s, 3 H, *CH₃*), 1,52 (s, 3 H, *CH₃),* 4,05 (m, 2 H, *CH₂),* 4,24 (m, 2 H, *CH-*C4 + C5), 4,53 (d, ³J_{H,H} = 3,79 Hz, 1 H, *CH* - C2), 5,33 (d, ³J_{H,H} = 1,77 Hz, 1 H, *CH* - C3), 5,90 (m, 2 H, *CH* - C1, C=OCH=*CH₂ cis*), 6,13 (dd, ³J_{H,H} = 10,48 Hz, ³J_{H,H} = 17,31 Hz, 1 H, C=*OCH=*CH₂), 6,45 (d, ³J_{H,H} = 17,43 Hz, 1 H, C=OCH=*CH₂ trans*). *-* ¹³C-NMR (CDCl₃, 23 °C 100,6 MHz, [ppm]): δ = 25,20, 26,17, 26,69, 26,80 (CH₃), 67,09 (CH₂), 72,40 (CH - C5), 76,18 (CH - C3), 79,72 (CH - C4), 83,26 (CH - C2), 105,03 (CH - C1), 109,31 (*C_{q}* - C5 + C6), 112,29 (*C_{q}* - C1 + C2), 127,68 (C=OCH=CH₂), 131,98 (C=OCH=CH₂), 164,69 (C=OCH=CH₂).
FT-IR (ATR, 23 °C, [cm⁻¹]): *υ̃* =1729 (s, v(C=O)), 1635 (m, v(C=C)), 1157 (s, v(C-O)), 1013 (ss, v C-O-C)), 802 (s, δ(CH2)).
µ-Raman (23 °C, [cm¹]): υ̃ =2947 (s, v(C-H_{2/3})), 1735 (m, v(C=O)), 1639 (m, v (C=C)), 1441 (m, v C-H_{2/3})). 802 (s, δ(CH₂)).

### 1.2 Herstellung eines Badmaterials unter Verwendung der gemäß 1.1 hergestellten (0,2 mmol) 6-O-Acroyl-1,2:5,6-Di-O-isopropyliden-α-D-glucofuranose und eines anorganisch-organischen Hybridpolymers

In einem 5 ml Rollrandglas wurden 63,6 mg (0,2 mmol) 6-*O*-Acroyl-1,2:5,6-Di-*O-*isopropyliden- α-D-glucofuranose und 540 mg (1,4 mmol) eines anorganisch-organischen Hybridpolymers aus 3-Methacryloxypropyl-trimethoxisilan und Diphenylsilandiol in einer 1:1-Stöchiometrie, das unter Katalyse von Ba(OH)₂ hydrolytisch kondensiert worden war, gegeben, worauf die Mischung 5 h bei 50°C im Ultraschallbad bei 45 kHz behandelt wurde. Es entstand eine feine weißlich trübe Dispersion. Diese Dispersion wurde nach Zugabe von 12 mg (0,03 mmol, 2 Gew.-%) Irgacure 369 10 Minuten bei 45 kHz im Ultraschallbad behandelt.

### 1.3 Herstellung eines Formkörpers

Die erhaltene Suspension wurde mittels Zwei-Photonen-Absorption (TPA) organisch polymerisiert und strukturiert wie oben im allgemeinen Beispiel "Mehrphotonen-polymerisation" angegeben.

### Beispiel 2 Funktionalisierung von 1,2:5,6-Di-O-isopropyliden-α-D-glucofuranose mit Methacrylsäureanhydrid und Herstellung von Formkörpern unter Verwendung des Produkts

### 2.1 Herstellung von (3-Methacroyl)-1,2:5,6-di-(O)-isopropyliden-α-D-glucofuranose

In einem 100 ml Schlenckkolben mit Rückflusskühler wurden unter Stickstoff-Atmosphäre 2,95 g (11,3 mmol) 1,2:5,6-Di-*O*-isopropyliden-α-D-glucofuranose in 15 ml trockenem Pyridin gelöst und 3 ml (3,13 g, 20,28 mmol) Methacrylsäureanhydrid zugegeben. Die Reaktionslösung wurde 3,5 h auf 65 °C erhitzt und anschließend wurden 16 ml Wasser zugegeben. Die Reaktionslösung wurde eine weitere Stunde auf 65°C und anschließend auf 30 °C für 17 h erhitzt, wobei die klare farblose Lösung milchig trüb wurde. Die Reaktionslösung wurde mit 3 ml, 6 ml und 10 ml Petrolether extrahiert. Die vereinigten organischen Lösungen wurden drei Mal mit je 15 ml Wasser und drei Mal mit 14 ml 5%iger Natriumhydrogencarbonat-Lösung gewaschen. Das Lösungsmittel wurde destillativ entfernt und ein weißer Feststoff erhalten. Dieser Feststoff wurde mit wenig Ethanol-Wasser-Lösung (14:10) gewaschen und im Ölpumpenvakuum getrocknet.
Ausbeute: 1,70 g (5,18 mmol, 45,7%)
¹H-NMR (CDCl₃, 23°C, 400,1 MHz, [ppm]): δ =1,29 (s, 6 H, *CH₃*), 1,40 (s, 3 H, C*H₃*), 1,52 (s, 3 H, C*H₃*), 1,94 (t, ³J_{H,H} = 1,52 Hz, 3 H, C=OCCH₃=CH₂), 4,04 (m, 2 H, *CH₂*), 4,25 (m, 2 H, *CH* - C4 + C5), 4,52 (d, ³J_{H,H} = 3,52 Hz, 1 H, *CH* - C2), 5,28 (d, ³J_{H,H} = 2,28 Hz, 1 H, *CH* - C3), 5,61 (t, ³J_{H,H} = 1,52 Hz, 1 H, C=OCCH₃*=CH₂ cis*), 5,88 (d, ³J_{H,H} = 3,8 Hz, 1 H, *CH* - C1), 6,11 (s, 1 H, C=OCH=*CH₂ trans*). *-* ^{*1*3}C-NMR (CDCl₃, 23°C 100,6 MHz, [ppm]): δ = 18,23 (C=OCCH₃=CH₂), 25,18, 26,15, 26,69, 26,77 (CH₃), 67,20 (CH₂), 72,49 (CH - C5), 76,40 (CH - C3), 79,87 (CH - C4), 83,24 (CH - C2), 105,03 (*C*H - C1), 109,28 (*C_{q} -* C5 + C6), 112,23 (*C_{q}* - C1 + C2), 126,51 (C=OCCH₃=CH₂), 135,78 (C=OCCH₃=CH₂), 165,92 (C=OCH=CH₂).
FT-IR (ATR, 23°C, [cm⁻¹]): *υ̃* =1716 (s, v(C=O)), 1631 (w, v(C=C)), 1159 (s, v(C-O)), 1021 (ss,v(C-O-C)).
µ-Raman (23 °C, [cm⁻¹]): *υ̃* = 2947 (s, v(C-H_{2/3})), 1735 (m, v(C=O)), 1639 (m, v(C=C)), 1441 (m, v(C-H_{2/3}), 802 (s, δ(CH₂)).

### 2.2 Herstellung eines härtbaren Harzes unter Verwendung von (3-Methacroyl)-1,2:5,6-di-(O)-isopropyliden-α-D-glucofuranose

Die entstandene (3-Methacroyl)-1,2:5,6-di-(O)-isopropyliden-α-D-glucofuranose wurde analog zu Beispiel 1.2 mit einem Hybridpolymer aus 3-Methacryloxypropyltrimethoxysilan und Diphenylsilandiol zusammengegeben und behandelt, wobei die Bestandteile im molaren Verhältnis von 33% modifiziertem Zucker und 67% Hybridpolymer eingesetzt wurden.

### 2.3 Polymerisation des Harzes unter UV-Bestrahlung

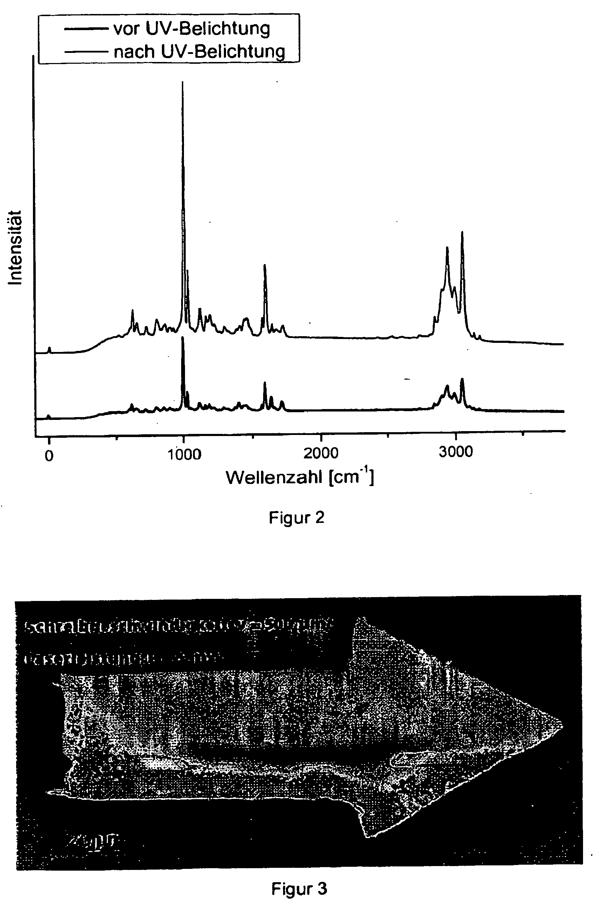

Das gemäß 2.2 hergestellte Harz wurde in eine Form gefüllt und mit Hilfe von UV-Belichtung organisch polymerisiert (ausgehärtet). Das µ-Raman-Spektrum vor und nach der Polymerisation ist in **Figur 2** dargestellt. Man erkennt eine deutliche Abnahme der C=C-Bande bei 1639 cm⁻¹ im Verhältnis zur C=O-Bande bei 1718 cm⁻¹ als Folge der organischen Vernetzung.

### 2.4 Herstellung eines Formkörpers mittels 2-Photonen-Absoprtion

Das gemäß 2.2 hergestellte Harz wurde mittels Zwei-Photonen-Absorption (TPA) organisch polymerisiert und strukturiert wie oben im allgemeinen Beispiel "Mehrphotonenpolymerisation" angegeben. Ein auf diese Weise hergestellter Formkörper ist in **Figur 3** dargestellt.

### Beispiel 3 Funktionalisierung von Polycaprolacton-diol mit Acrylsäureethylester, Herstellung von Formkörpern unter Verwendung des Produkts und Degradationsstudie dazu

### 3.1 Herstellung von (6-Acroyl)hexansäureethylester

In einem 100 ml Schlenckkolben mit Mikrodestille wurden unter Stickstoff-Atmosphäre 9,00 g (16,98 mmol) Polycaprolacton-diol, 26,5 ml (37,2 g, 372 mmol) Acrylsäureethylester, 0,85 g (6,85 mmol) *para*-Methoxyphenol und 0,88 ml (0,84 g 2,94 mmol) Tetra(isopropyloxy)-titan gegeben und die rote Reaktionslösung auf 113°C erhitzt. Nach 40 Minuten wurde bei 74-75 °C ein farbloses Acrylsäureethylester-Ethanol-Azeotrop abdestilliert. Nach 2 Stunden wurde die Lösung für 30 min an Luft abgekühlt und 16,1 ml Petrolether (Sdp. 30-50 °C) und 3,2 ml Wasser zugegeben, wobei nach 4,5 h ein gelber Niederschlag ausfiel. Der Niederschlag wurde nach weiteren 15 h 10 Minuten bei 4000 U·min⁻¹ abzentrifugiert, zwei Mal mit Pentan gewaschen und erneut abzentrifugiert. Die organischen Lösungen wurden vereinigt und drei Mal mit je 40 ml Wasser gewaschen. Das Lösungsmittel wurde destillativ entfernt, sodass eine gelbe Lösung zurückblieb. Diese Lösung wurde bei 7·10⁻³mbar und 50-62°C fraktioniert destilliert, wobei eine farblose Flüssigkeit erhalten wurde. Diese farblose Flüssigkeit wurde anschließend noch säulenchromatographisch gereinigt (Eluens: n-Pentan:Essigsäureethylester = 10:1).
Ausbeute: 3,41 g (5,34 mmol, 31,4%)
¹H-NMR (CDCl₃, 23 °C, 400,1 MHz, [ppm]): δ = 1,19 (t, ³*J*_{HH} = 7,32 Hz, 3 H, OCH₂*CH₃*), 1,36 (m, 2 H, OCH₂CH₂*CH₂*CH₂CH₂C=O), 1,62 (m, 4 H, OCH₂CH₂*CH₂*CH₂CA₂C=O), 2,24 (t, ³*J*_{HH} = 7,56 Hz, 2 H, OCH₂CH₂CH₂*CH₂*CH₂C=O), 4,07 (m, 4 H, OCH₂CHzCH₂CH₂CH₂C=OO*CH*₂CH₃), 5,75 (dd, ²*J*_{HH} = 1,52 Hz, ³*J*_{HH} = 10,36 Hz, 1 H, OC=OCH=*CH₂*cis), 6,05 (dd, ³*J*_{HH} = 10,36 Hz, ³*J*_{HH} = 17,43 Hz, 1 H, OC=OC*H*=CH₂), 6,33 (dd, ²*J*_{HH} = 1,52 Hz, ³*J*_{HH} = 17,43 Hz, OC=OCH=*CH₂*trans). - ¹³C-NMR (CDCl₃, 23°C 100,6 MHz, [ppm]): δ = 13,27 (OCH₂CH₃), 23,57, 24,51, 27,30, 33,15, 59,24, 63,32 (CH₂). 127,52 (OC=OCH=CH₂), 129,54 (OC=OCH=CH₂), 165,25, 172,51 (*C*=O). FT-IR (ATR, 23°C, [cm⁻¹]): υ̃ = 3100-3000 (w, v(=C-H)), 2945, 2866 (m, v(-C-H)), 1722 (s, v(-C=O)), 1636, 1620 (w, v(-C=C)), 1511, 1457, 1408 (m, δ(-CH₂)), 1374, 1270, 1186, 1098, 1061, 1035 (s, v(-C-O-C)).
µ-Raman (23 °C, [cm⁻¹]): υ̃ =1072, 1199, 1304 (v(-C-O-C)), 1412, 1453 (δ(CH₂)), 1639 (v(C=C)), 1726 (v(-C=O)), 2939 (v(-C-H)), 3042, 3072, 3109 (v(=C-H)).

### 3.2. Polymerisation von (6-Acroyl)hexansäureethylester

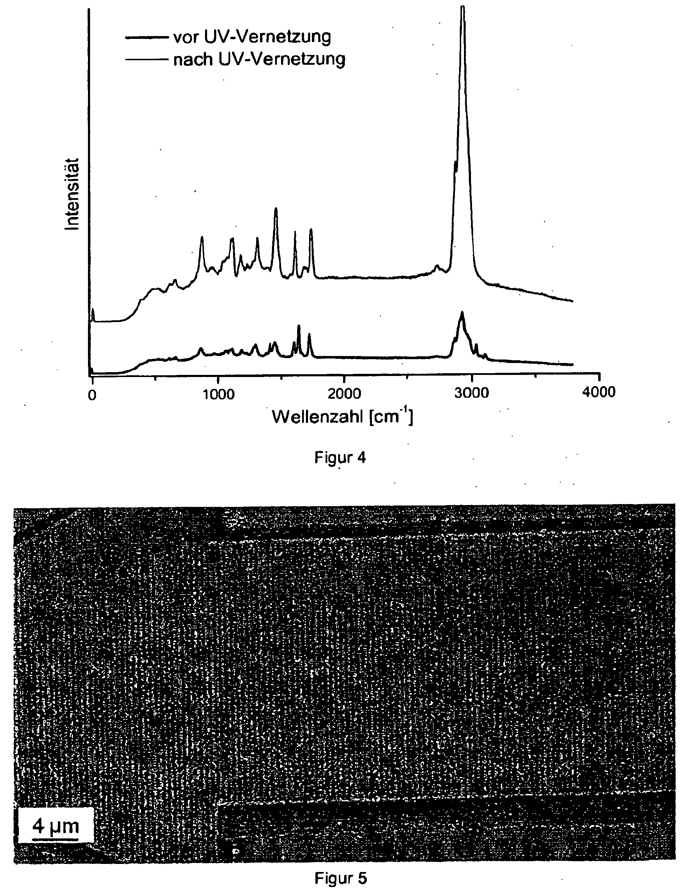

Der gemäß 3.1. hergestellte (6-Acroyl)hexansäureethylester wurde mit 2 Gew.-% Irgacure^{®} 369 als Photostarter versetzt und durch die Belichtung mit UV-Licht vernetzt. Das µ-Raman-Spektrum vor und nach der Polymerisation ist in **Figur 4** dargestellt. Man erkennt eine deutliche Abnahme der C=C-Bande bei 1634 cm⁻¹ im Verhältnis zur C=O-Bande bei 1738 cm⁻¹ als Folge der organischen Vernetzung.

### 3.3 Herstellung eines Formkörpers aus (6-Acroyl)hexansäureethylester mittels 2-Photonen-Absorption

Der gemäß 3.1. hergestellte (6-Acroyl)hexansäureethylester wurde mit 2 Gew.-% Irgacure® 369 als Photostarter versetzt und mittels Zwei-Photonen-Absorption (TPA) organisch polymerisiert und strukturiert wie oben im allgemeinen Beispiel "Mehrphotonenpolymerisation" angegeben. Ein auf diese Weise hergestellter Formkörper ist in **Figur 5** dargestellt.

### 3.4 Herstellung eines härtbaren Harzes unter Verwendung des gemäß 3.1 hergestellten (6-Acroyl)hexansäureethylesters und eines anorganisch-organischen Hybridpolymers

Der (6-Acroyl)hexansäureethylester wurde analog zu Beispiel 1.2 mit einem Hybridpolymer aus 3-Methacryloxypropyl-trimethoxysilan und Diphenylsilandiol zusammengegeben und behandelt, wobei die Bestandteile im molaren Verhältnis von 50% (6-Acroyl)hexansäureethylester und 50% Hybridpolymer eingesetzt wurden.

### 3.5 Polymerisation des gemäß 3.4. erhaltenen Harzes unter UV-Bestrahlung

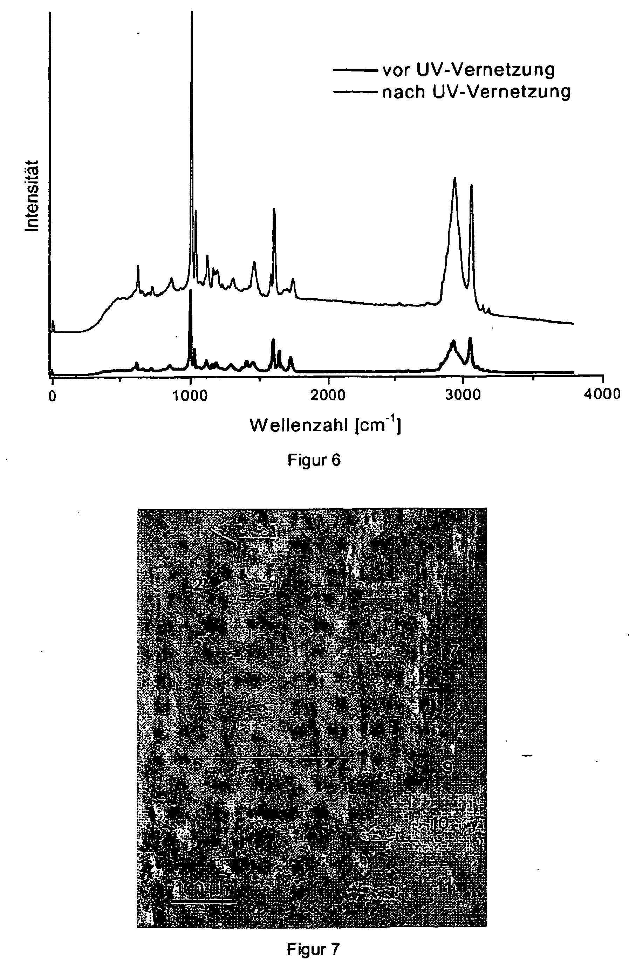

Das gemäß 3.4 hergestellte Harz wurde in eine Form gefüllt und mit Hilfe von UV-Belichtung organisch polymerisiert (ausgehärtet). Das µ-Raman-Spektrum vor und nach der Polymerisation ist in **Figur 6** dargestellt. Man erkennt eine deutliche Abnahme der C=C-Bande bei 1639 cm⁻¹ im Verhältnis zur C=O-Bande bei 1718 cm⁻¹ als Folge der organischen Vernetzung.

### 3.6 Herstellung eines Formkörpers mittels 2-Photonen-Absoprtion

Das gemäß 3.4 hergestellte Harz wurde mittels Zwei-Photonen-Absorption (TPA) organisch polymerisiert und strukturiert wie oben im allgemeinen Beispiel "Mehrphotonenpolymerisation" angegeben. Auf diese Weise hergestellte Formkörper (strukturiert aus einem Tropfen auf einer Glasplatte) sind in **Figur 7** dargestellt.

### 3.7 Degradationsstudie

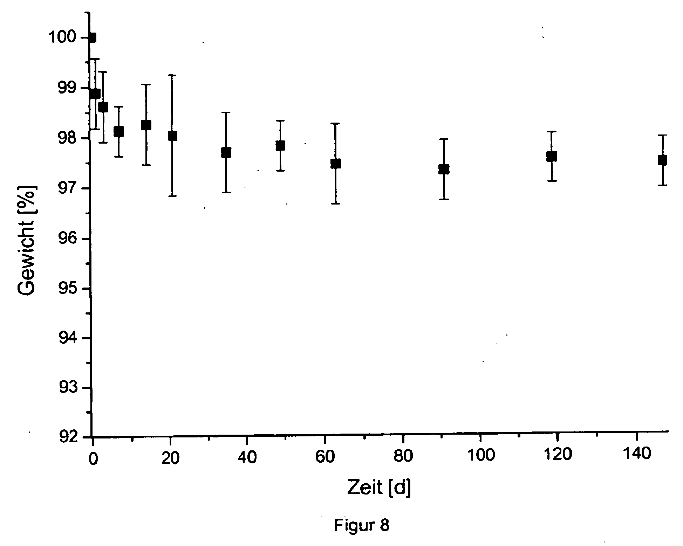

Das gemäß 3.4 hergestellte Harz wurde wie im allgemeinen Beispiel erläutert ausgehärtet und danach in PBS-Lösung eingelegt. Das Ergebnis ist graphisch in **Figur 8** dargestellt. In einem Vergleichsversuch mit dem anorganisch-organischen Hybridpolymer ohne (6-Acroyl)hexansäureethylester konnte keinerlei Degradation beobachtet werden.

### Beispiel 4 Funktionalisierung von Polycaprolacton-diol mit Methylmethacrylat, Herstellung von Formkörpern unter Verwendung des Produkts sowie Degradations- und Biokompatibilitätsstudien dazu

### 4.1 Herstellung von (6-Methacroyl)hexansäuremethylester

10.0 g (18.9 mmol) Polycaprolactondiol wurden unter Schutzgasatmosphäre in 29.7 mL (276 mmol) Methylmethacrylat gelöst und mit 991 mg (7.98 mmol) *p*-Methoxyphenol sowie 941 mg (3.31 mmol) Tetra(isopropyloxy)-titan(IV) versetzt, woraufhin sich die Lösung dunkelrot verfärbte. Unter Rühren wurde auf 110 °C erhitzt und innerhalb von 45 min etwa 4 mL eines Azeotrops aus Methanol und Methylmethacrylat abdestilliert (Übergangstemperatur ca. 45 °C). Man ließ die Mischung 30 min lang abkühlen und gab 15.2 mL *n*-Pentan sowie 3.00 mL Wasser hinzu, wodurch ein orangefarbener Niederschlag ausfiel. Dieser wurde durch Zentrifugieren und Abdekantieren entfernt und zweimal mit je etwa 15 mL *n*-Pentan nachgewaschen. Filtrat und Waschlösungen wurden vereinigt und unter vermindertem Druck vom Lösemittel befreit. Der hellgelbe Rückstand wurde durch Destillation bei 6.0 x 10⁻³ mbar aufgereinigt. Siedepunkt: 65°C, Ausbeute: 3.52 g (5.28 mmol, 28 %) einer hellvioletten Flüssigkeit. NMR- und UV-Vis-Spektrum zeigten, dass noch Verunreinigungen an *p*-Methoxyphenol enthalten waren, welche durch Säulenchromatographie an einer Kieselgelphase (Eluens: 10:1-Mischung von *n*-Pentan und Essigsäureethylester) entfernt werden konnten.
Ausbeute: 713 mg (3.33 mmol, 8.8%)
¹H-NMR (CDCl₃, 23°C, 400,1 MHz): δ = 1,35 (m, 2 H, OCH₂CH₂*CH₂*CH₂CH₂C=O), 1,62 (m, 4 H, OCH₂*CH₂*CH₂*CH₂*CH₂C=O), 1,87 (t, ⁴J_{H.H} = 1,26 Hz, 3 H, OC=OC*CH₃*=CH₂), 2,26 (t, 2 H, ³J_{H,H} = 7,45 Hz, OCH₂CH₂CH₂CH₂*CH₂*C=O), 3,60 (s, 3 H, O*CH₃*), 4,08 (t, ³J_{H,H} = 6,57, 2 H, OC*H₂*CH₂CH₂CH₂CH₂C=O), 5,48 (m, 1 H, OC=OCCH₃*=CH₂ cis),* 6,02 (m, 1 H, OC=OCCH₃=*CH₂ trans*)*.*). -¹³C-NMR (CDCl₃, 23°C 100 MHz): δ = 18,46 (OC=OCCH₃=CH₂), 24.81, 25.72, 28.45, 34.06 (CH₂), 51,67 (OCH₃), 64.58 (CH₂), 125.41 (OC=OCCH3=CH₂), 136.59 (OC=OCCH₃=CH₂), 167.62, 174.12 (C=O).
FT-IR (ATR, 23 °C, [cm⁻¹]) *υ̃* = 3100-3000 (w, v(=C-H)), 2951, 2863 (m, v(-C-H)), 1715 (s, v(-C=O)), 1636 (w, v(-C=C)), 1510, 1438 (m, δ(-CH₂)), 1321, 1296, 1234, 1162, 1101, 1036 (s, v(-C-O-C)).
µ-Raman (23 °C, [cm⁻¹]) υ̃= 1011, 1050, 1101, 1161, 1178, 1258, 1296 (v(-C-O-C)), 1403, 1448 (v(CH₂)), 1635 (v(C=C)), 1714 (v(-C=O)), 2925 (δ(-C-H)), 3062, 3105 (v(=C-H)).

### 4.2 Herstellung härtbarer Harze unter Verwendung des gemäß 4.1 hergestellten (6-Methacroyl)hexansäuremethylesters

Der (6-Methacroyl)hexansäuremethylester wurde analog zu Beispiel 1.2 mit einem Hybridpolymer aus 3-Methacryloxypropyl-trimethoxysilan und Diphenylsilandiol zusammengegeben und behandelt, wobei die Bestandteile (a) im molaren Verhältnis von 50% (6-Methacroyl)hexansäuremethylesters und 50% Hybridpolymer und (b) von 25% (6-Methacroyl)hexansäuremethylesters und 75% Hybridpolymer eingesetzt wurden.

### 4.3 Polymerisation des gemäß 4.2 erhaltenen Harzes unter UV-Bestrahlung

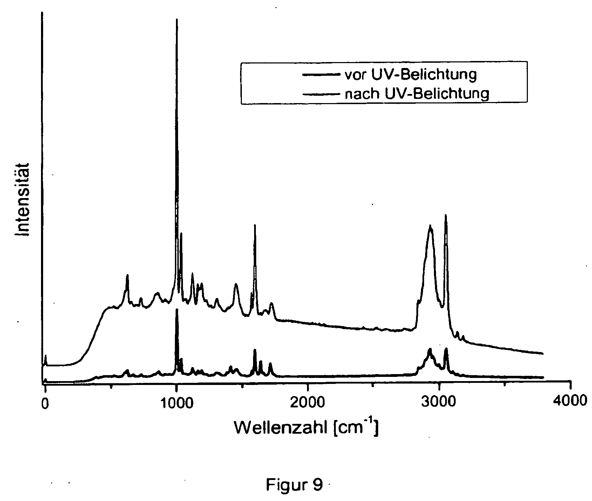

Das gemäß 4.2, Variante (a) hergestellte Harz wurde in eine Form gefüllt und mit Hilfe von UV-Belichtung organisch polymerisiert (ausgehärtet). Das µ-Raman-Spektrum vor und nach der Polymerisation ist in **Figur 9** dargestellt. Man erkennt eine deutliche Abnahme der C=C-Bande bei 1646 cm⁻¹ im Verhältnis zur C=O-Bande bei 1722 cm⁻¹ als Folge der organischen Vernetzung.

### 4.4 Herstellung eines Formkörpers mittels 2-Photonen-Absoprtion

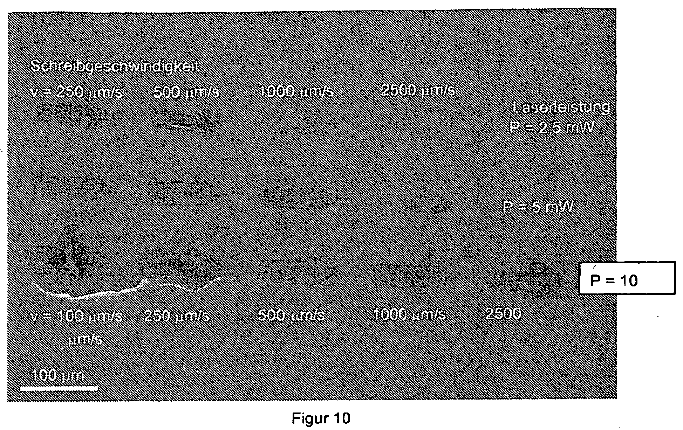

Das gemäß 4.2, Variante (a) hergestellte Harz wurde mittels Zwei-Photonen-Absorption (TPA) organisch polymerisiert und strukturiert wie oben im allgemeinen Beispiel "Mehrphotonenpolymerisation" angegeben. Auf diese Weise hergestellte Formkörper sind in **Figur 10** dargestellt.

### 4.5 Degradationsstudie

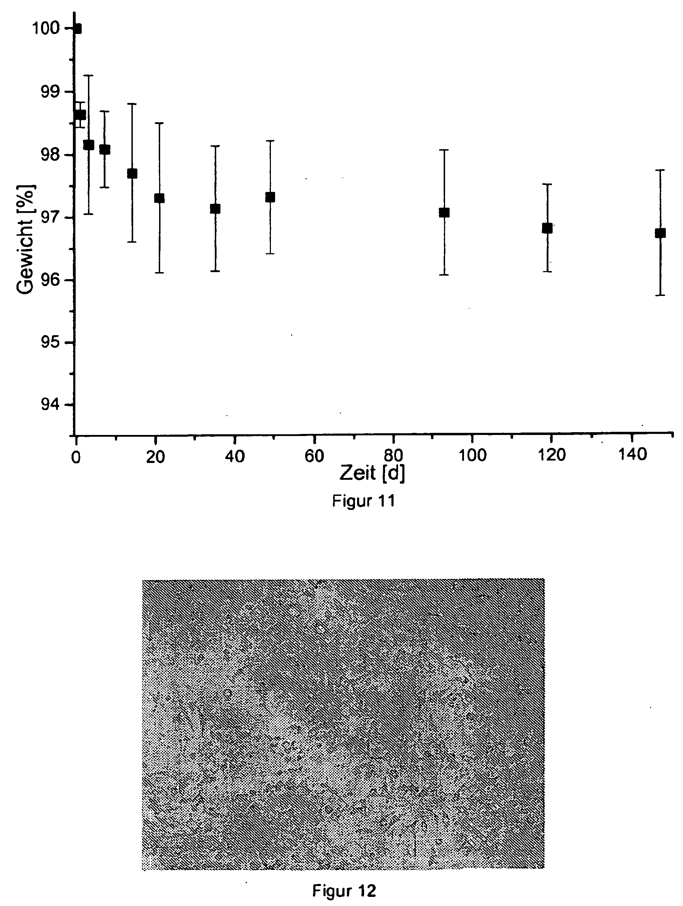

Das gemäß 4.2, Variante (a) hergestellte Harz wurde wie im allgemeinen Beispiel erläutert ausgehärtet und danach in PBS-Lösung eingelegt. Das Ergebnis ist graphisch in **Figur 11** dargestellt.

### 4.6 Biokiompatibilitatsstudie

Eine Form mit gemäß 4.2, Variante (b) hergestelltem Harz wurde mit UV-Licht bestrahlt. Nach dem Aushärten wurde ein Biokompatibilitätsversuch mit Mausfibroblasten L929 durchgeführt, die auf die glatte Oberfläche des ausgehärteten Materials aufgebracht wurden. Nach drei Tagen war das Material mit Zellen besiedelt. Diese hatten sich in alle Richtungen ausgestreckt ("Spreading"), wie in **Figur 12** zu erkennen, was ein Zeichen dafür ist, dass es ihnen gut geht.

### Beispiel 5 Funktionalisierung von Polycaprolacton-diol mit Di(ethylenglykol)-dimethacrylat und organische Polymerisation des Produkts

### 5.1 Herstellung von (6-Methacroyl)hexansäure(di(ethylenglykol)methacrylat)ester

In einem 100 ml Schlenckkolben mit Rückflusskühler wurden unter StickstoffAtmosphäre 4,72 g (8,90 mmol) Polycaprolacton-diol, 20,0 ml (21,64 g, 89,3 mmol) Di(ethylenglykol)-dimethacrylat und 0,50 ml (0,48 g 1,70 mmol) Tetraisopropyltitan(IV) gegeben und die gelbe Reaktionslösung auf 135 °C erhitzt. Nach vier Stunden wurde die Lösung für 30 min an Luft abgekühlt und 10,0 ml Wasser zugegeben, wobei sofort ein gelblicher Niederschlag ausfiel. Der Niederschlag wurde nach weiteren 15 h 10 Minuten bei 4000 U·min⁻¹ abzentrifugiert, mit wenig Pentan gewaschen und erneut abzentrifugiert. Die organischen Lösungen wurden drei Mal mit je 10 ml Wasser gewaschen. Das Lösungsmittel wurde destillativ entfernt, so dass eine farblose Lösung zurückblieb. Diese farblose Flüssigkeit wurde anschließend säulenchromatographisch mit n-Pentan: Essigsäureethylester = 5:1 als Eluens gereinigt.
Ausbeute: 5,754 g (16,15 mmol, 45,4%)
¹H-NMR (CDCl₃, 23 °C, 400,1 MHz, [ppm]): δ = 1,35 (m, 2 H, C=OOCH₂CH₂*CH*₂CH₂-CH₂C=OO), 1,61 (m, 4 H, C=OOCH₂*CH*₂CH₂*CH*₂CH₂=OO), 1,86 (s, 3 H, H₂C=*CCH*₃C=OOCH₂CH₂CH₂CH₂CH₂C=O), 1,87 (s, 3 H, OCH₂CH₂OC=OCC*H*₃=CH₂), 2,28 (t, ³J_{H,H} = 7,58 Hz, 2 H, , C=OOC*H*₂CH₂CH₂CH₂CH₂C=OO), 3,64 (t, ³*J*_{H,H} = 4,80 Hz, 2 H), 3,67 (t, ³*J*_{H,H} = 4,92 Hz, 2 H, C=OOC*H*₂CH₂OCH₂C*H*₂OC=O), 4,06 (t, ³J_{H,H} = 6,57 Hz, 2 H, C=OOCH₂CH₂CH₂CH₂C*H*₂C=OO), 4,16 (t, ³J_{H,H} = 4,67 Hz, 2 H, C=OOCH₂C*H*₂OCH₂CH₂OC=O), 4,23 (t, ³J_{H,H} = 4,80 Hz, 2 H, C=OOCH2CH2OC*H*2-CH2OC=O), 5,47 (t, ⁴J = 1,52 Hz, 1 H, *H*₂C=CCH₃C=OOCH₂CH₂CH₂CH₂CH₂C=O *cis*), 5,51 (t, ⁴J = 1,52 Hz, 1 H, OCH₂CH₂OC=OCCH₃=*CH*₂ *cis*), 6,01 (s, 1 H, *H₂*C=CCH₃C=OOCH₂CH₂CH₂CH₂CH₂C=O *trans*), 6,05 (s, 1 H, OCH₂CH₂OC=OCCH₃=C*H*₂ *trans*). - ¹³C-NMR (CDCl₃, 23°C 100,6 MHz, [ppm]): δ = 17,31, 17,32 (CH₃), 23,51 (C=OOCH₂CH₂CH₂CH₂CH₂C=OO), 24,56 (C=OOCH₂CH₂CH₂CH₂CH₂C=OO), 27,31 (C=OOCH₂CH₂CH₂CH₂CH₂C=OO), 32,98 (C=OO*C*H₂CH₂CH₂CH₂CH₂C=OO), 62,34 (C=OOCH₂CH₂OCH₂CH₂OC=O), 62,75 (C=OOCH₂CH₂O*C*H₂CH₂OC=O), 63,44 (C=OOCH₂CH₂CH₂CH₂CH₂C=OO), 68,05, 68,12 ((C=OO*C*H₂CH₂OCH₂CH₂OC=O), 124,28, 124,80 (CCH₃=CH₂), 13.5,11, 135,41 (*C*CH₃=CH₂), 166,27, 166,43 (*C*=OCCH₃=CH₂), 172,43 (C=OOCH₂CH₂CH₂CH₂CH₂*C*=OO).
FT-IR (ATR, 23 °C, [cm⁻¹]): υ̃ =2951 (m, vₐₛ(C-H)). 2867 (w, vₛ(C-H)), 1715 (s, v(C=O)), 1637 (m, v(C=C)), 1296 (s, v(C-O-C)). 1160 (ss, v(C-0-C)), 940 (s, δ(C-H)), 814 (m, skel. bend).
µ-Raman (23 °C, [cm⁻¹]): υ̃ =868 (m, γ(C-H)), 1444 (m, δ(C-H)), 1639 (m, v(C=C)), 1718 (m, v(C=O)), 2928 (s, vₐₛ(C-H)).

### 5.2 Herstellung eines härtbaren Harzes unter Verwendung von (6-Methacroyl)hexansäure(di(ethylenglykol)methacrylat)ester

(6-Methacroyl)hexan-säure(di(ethylenglykol)methacrylat)ester wurde analog zu Beispiel 1.2 mit einem Hybridpolymer aus 3-Methacryloxypropyl-trimethoxysilan und Diphenylsilandiol zusammengegeben und behandelt, wobei die Bestandteile im molaren Verhältnis von 50% Methacrylatester und 50% Hybridpolymer eingesetzt wurden.

### 5.3 Polymerisation des Harzes unter UV-Bestrahlung

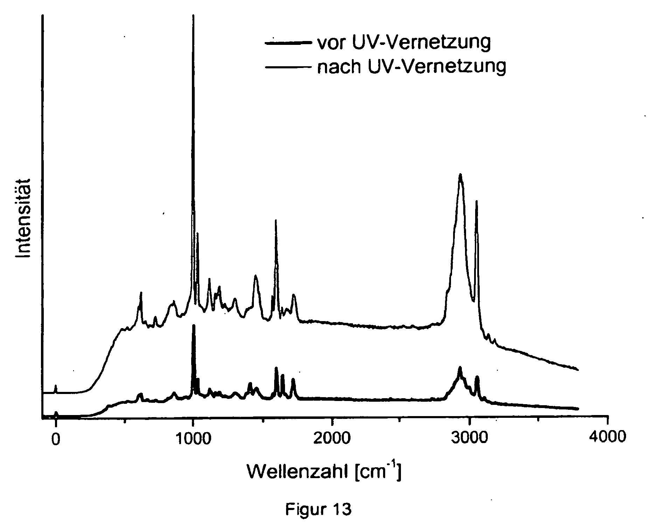

Das gemäß 5.2 hergestellte Harz wurde in eine Form gefüllt und mit Hilfe von UV-Belichtung organisch polymerisiert (ausgehärtet). Das µ-Raman-Spektrum vor und nach der Polymerisation ist in **Figur 13** dargestellt. Man erkennt eine deutliche Abnahme der C=C-Bande bei 1648 cm⁻¹ im Verhältnis zur C=O-Bande bei 1722 cm⁻¹ als Folge der organischen Vernetzung.

### Beispiel 6 Funktionalisierung von 1,2:5,6-Di-O-isopropyliden-D-mannitol mit Methacrylsäureanhydrid

In einen 100 mL Kolben mit aufgesetztem Rückflusskühler wurden 3,01 g (11,5 mmol) 1,2:5,6-di-*O*-isopropyliden-D-mannitol, sowie 15,0 mL Pyridin gegeben und bis zur vollständigen Auflösung des Mannitols gerührt. Anschließend wurden 3,00 mL (3,13 g, 20,3 mmol) Methacrylsäureanhydrid hinzugegeben und die Lösung weitere dreieinhalb Stunden bei 65 °C unter Rückfluss gerührt. Danach wurden 16,2 mL Wasser hinzugegeben und die farblose Lösung für eine weitere Stunde bei 65 °C weiter gerührt. Die Mischung wurde auf 30 °C abgekühlt und 17 h gerührt. Nach diesen 17 h wurde die Lösung drei Mal mit je 10,0 mL Pentan extrahiert. Die organischen Phasen wurden vereinigt, drei Mal mit je 20 mL Wasser und drei Mal mit je 20 mL 5%iger Natriumhydrogencarbonat-Lösung gewaschen und über Natriumsulfat getrocknet. Nach Abfiltrieren des Trockenmittels erhielt man eine klare, farblose Flüssigkeit. Das Lösungsmitten wurde abdestilliert und das Produkt im Ölpumpenvakuum getrocknet, worauf ein farbloser Feststoff erhalten wurde.

### Beispiel 7 Synthese von O-(Methacryloxyethyl)-N-(triethoxysilylpropyl)urethan, Herstellung von Formkörpern unter dessen Verwendung sowie Degradations- und Biokompatibilitätsstudien dazu

### 7.1. Synthese von O-(Methacryloxyethyl)-N-(triethoxysilylpropyl)urethan

247 g (1 mol) (3-isocyanatopropyl)triethoxysilan wurden langsam zu einer Mischung aus 130 g (1 mol) (Hydroxyethyl)methacrylat, 150 mg Hydroquinonmonomethylether und 180 mg Dibutyl-Zinn-Dilaureat gegeben und die Lösung für 24 Stunden bei Raumtemperatur gerührt. Anschließend war die Isocyanat-IR-Bande bei 2245 cm⁻¹ nicht mehr zu beobachten. Das Produkt hat die folgende Strukturformel:

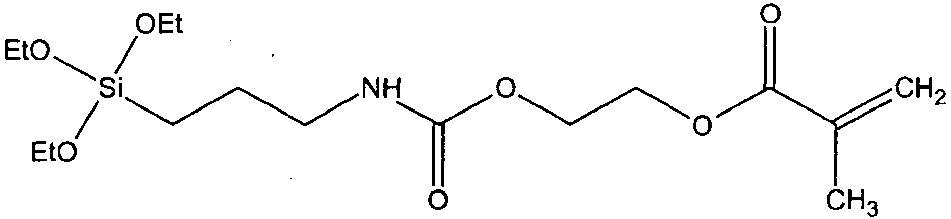

Dieses Molekül bietet die Möglichkeit zur organischen-photochemischen Vernetzung an der Methacrylatgruppe, kann durch die Alkoxysilan-Einheitein anorganisches Netzwerk bilden und an der Carbamat-Gruppe degradieren.

### 7.2 Vernetzung von O-(Methacryloxyethyl)-N-(triethoxysilylpropyl)urethan durch hydrolytische Kondensation der Silyl-Gruppen

In einem 50 ml Rundkolben wurden 15,17 g *O*-(Methacryloxyethyl)-*N-*(triethoxysilylpropyl)urethan in 25 ml Ethylacetat gelöst. Anschließend wurden 1,8 ml 0,5 M Salzsäure (Lösung aus 192 ml Wasser und 8 ml konz. Salzsäure) zugegeben und die weißlich trübe Lösung für 42 Stunden unter Lichtausschluss gerührt. Danach wurde 3 Mal mit je 23 ml destilliertem Wasser gewaschen und nach jedem Waschschritt der pH-Wert kontrolliert. Nach Trocknen der Mischung mit Hilfe von hydrophobem Filterpapier wurde das Lösungsmittel destillativ entfernt und eine viskose, leicht gelbliche Flüssigkeit erhalten.
¹H-NMR ([D₆]Aceton, 23 °C, 400,1 MHz, [ppm]): δ = 0,66 (m, 2 H, NHCH₂C*H*₂CH₂Si), 1,66 (m, 2 H, NHCH₂CH₂C*H*₂Si), 1,91 (s, 3 H, H₂C=CC*H*₃C(O)O), 3,14 (m, 2 H, NHC*H*₂CH₂CH₂Si), 4,30 (m, 4 H, C(O)OC*H*₂C*H*₂OC(O)), 5,62 (m, 1 H, O(O)CCCH₃=C*H*₂ *cis*), 6,08 (m, 1 H, O(O)CCCH₃=C*H*₂ *trans*). -²⁹Si-NMR ([D₆]Aceton, 23 °C, 79,50 MHz, [ppm]): δ = -66,52 - -63,88 (m, T³, NHCH₂CH₂CH₂*Si*(OSi)₃), -60,64 - -59,49 (m, T², NHCH₂CH₂CH₂*Si*(OSi)₂(OEt)), -57,99 - -57,41 (m, T², NHCH₂CH₂CH₂*Si*(OSi)₂(O)), -56,11 --48,77 (m, T¹, NHCH₂CH₂CH₂*Si*(OSi)(OEt)₂, NHCH₂CH₂CH₂*Si*(OSi)(OEt)(OH), NHCH₂CH₂CH₂*Si*(OSi)(OH)₂).
FT-IR (ATR, 23 °C, (cm⁻¹]): υ̃ = 3338 (m, v(N-H)), 1698 (s, v(C=O)), 1636 (m, v(C=C)), 1530 (s, v(CHN)), 1450 (m, δₐₛ(C-H)), 1246 (s, vₐₛ(C-O-C)), 1045 (s, v(Si-O-Si)). µ-Raman (23 °C, [cm⁻¹]): υ̃ = 539 (m, v(C=O)), 1295 (m, v(C-O-C)), 1407 (s, δ(=CH₂)), 1444 (s, δ(CH₂)), 1639 (s, v(C=C)), 1718 (s, v(C=O)), 3363 (w, v(N-H)). Ausbeute: 12,79 g

### 7.3 Polymerisation des Kondensats durch UV-Bestrahlung

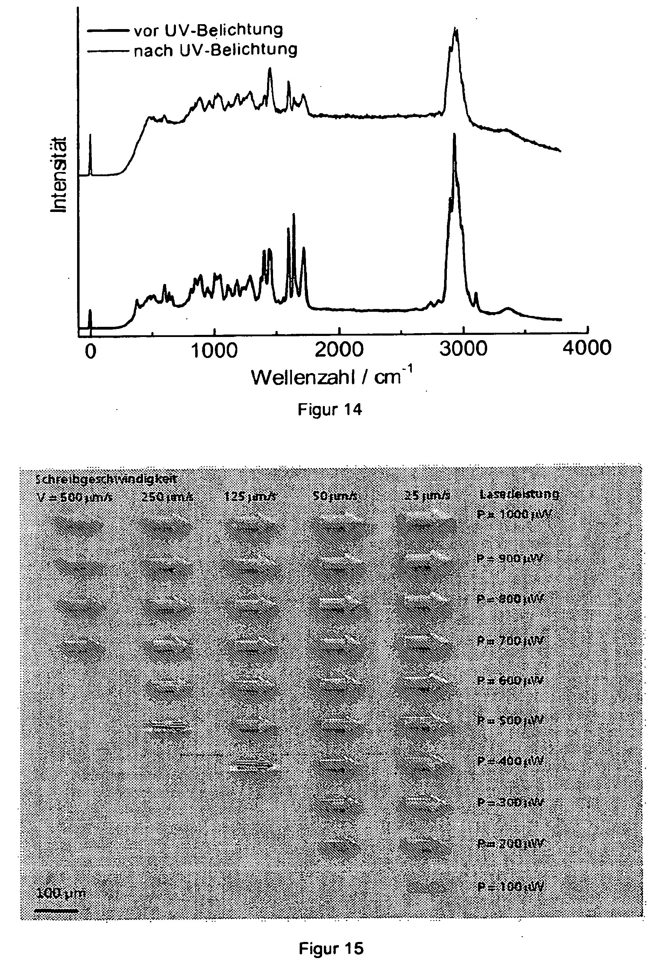

Das gemäß 7.2 hergestellte Harz (Polykondensat) wurde mit 2 Gew.-% Irgacure^{®} 369 versetzt, in eine Form gefüllt und mit Hilfe von UV-Belichtung organisch polymerisiert (ausgehärtet). Das IR-Spektrum vor und nach der Polymerisation ist in Figur 14 dargestellt. Man erkennt eine deutliche Abnahme der C=C-Bande bei 1640 cm⁻¹ im Verhältnis zur C=O-Bande bei 1718 cm⁻¹ als Folge der organischen Vernetzung.

### 7.4 Herstellung von Formkörpern mittels 2-Photonen-Absoprtion

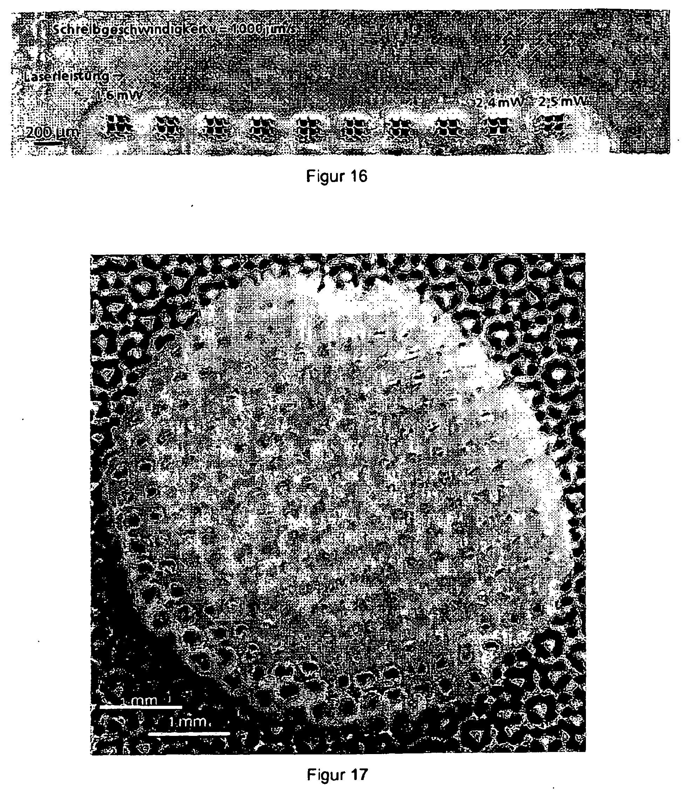

Das gemäß 7.2 hergestellte Harz (Polykondensat) wurde mittels Zwei-Photonen-Absorption (TPA) organisch polymerisiert und wie oben im allgemeinen Beispiel "Mehrphotonenpolymerisation" angegeben strukturiert. Es wurden unterschiedliche Schreibgeschwindigkeiten und Laserleistungen verwendet. **Figur 15** zeigt eine Reihe von Ergebnissen im Vergleich. Besonders feine Strukturierungen (Ausbildung von Kanälen, Vorstudie für die Erzeugung von Scaffolds) sind in **Figur 16** gezeigt. Hier wurde die Laserleistung bei einer gleichbleibenden Schreibgeschwindigkeit von 1000 µm/s schrittweise von 1,6mW auf 2,5mW erhöht.

### 7.5 Scaffold-Herstellung

Das gemäß 7.2 hergestellte Harz (Polykondensat) wurde mittels Zwei-Photonen-Absoprtion (TPA) organisch polymerisiert und wie oben im allgemeinen Beispiel "Mehrphotonenpolymerisation" angegeben unter den nachfolgend angegebenen Bedingungen und mit den angegebenen Abmessungen strukturiert. Das Scaffold ist in Figur 17 gezeigt.
- Design:
   ∘ Durchmesser 5,5 mm, Höhe ca. 1,2 mm
   ∘ kubische Poren (200 Mikrometer Seitenlänge)
- Prozessierung
   1. Zweiphotonenpolymerisation mit Standardbelichtungsaufbau (siehe Figur 1, die auch detailliert in WO 03/037606 A1 erläutert ist)
   2. Entwicklung (3 h in Isopropanol/MBIK (1:1))
   3. UV Nachbelichtung mit dem Maskaligner (20 mW/cm² für 3 min)

### 7.6 Degradationsstudie

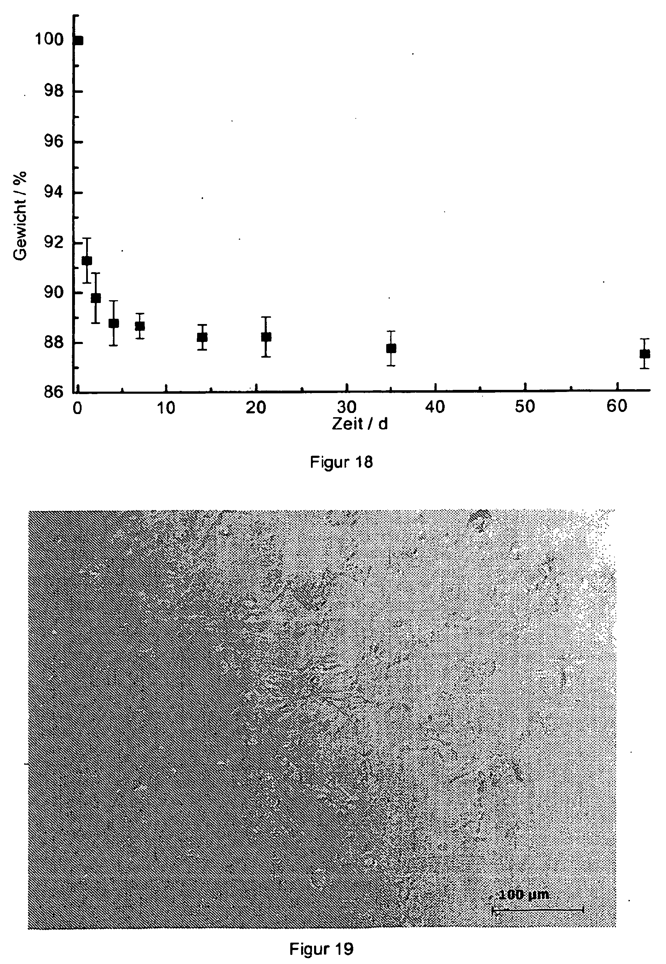

Das gemäß 7.2 hergestellte Harz (Polykondensat) wurde wie im allgemeinen Beispiel erläutert ausgehärtet und danach in PBS-Lösung eingelegt. Das Ergebnis ist graphisch in **Figur 18** dargestellt.

### 7.7 Biokompatibilitätsstudie

Das gemäß 7.2 hergestellte Harz wurde mit UV-Licht bestrahlt. Nach dem Aushärten wurde ein Biokompatibilitätsversuch mit Mausfibroblasten L929 durchgeführt, die auf das ausgehärtete Materials (Bulkmaterial) aufgebracht wurden. Nach sieben Tagen war das Material mit Zellen besiedelt. Diese hatten sich in alle Richtungen ausgestreckt ("Spreading"), wie in **Figur 19** zu erkennen, was ein Zeichen dafür ist, dass es ihnen gut geht.

Die Erfindung stellt demnach unter anderem die folgenden Verfahren, Gegenstände und Verwendungsmöglichkeiten bereit:
1. Ein Verfahren zum Erzeugen von dreidimensionalen selbsttragenden und/oder substratgestützten Formkörpern oder von Strukturen auf Oberflächen durch ortsselektives Verfestigen eines flüssigen bis pastösen, organischen oder organisch modifizierten Materials innerhalb eines Bades aus diesem Material mit Hilfe von Zwei- oder Mehr-Photonen-Polymerisation, wobei das Material mindestens eine Verbindung aufweist, die sowohl einen organischen, über 2-Photonen- oder Mehrphotonen-Polymerisation polymerisierbaren Rest als auch eine biokompatible, biodegradierbare oder bioresorbierbare Gruppe aufweist, und/oder worin das Badmaterial weiterhin Gruppen oder Reste enthält, die einer anorganischen Vernetzung zugänglich oder bereits anorganisch vernetzt sind, mit der Maßgabe, dass in dem Material sowohl ein organischer, über 2-Photonen- oder Mehrphotonen-Polymerisation polymerisierbarer Rest als auch eine biokompatible, biodegradierbare oder bioresorbierbare Gruppe enthalten sein müssen.
2. Ein Verfahren wie unter Punkt 1 definiert, worin der biokompatible, biodegradierbare.oder bioresorbierbare Rest ein solcher ist, der mindestens eine Gruppe aufweist, ausgewählt unter einer Sauerstoffbrücke zwischen zwei Kohlenstoffatomen, -OC(O)O-, -C(O)NH-, -NHC(O)NH-, -NHC(O)O-, -C(O)OC(O)- und -C(O)O-, und/oder der unter physiologischen Bedingungen im menschlichen oder tierischen Körper oder durch Mikroorganismen gespalten wird, und worin der genannte Rest vorzugsweise ein solcher ist, der mindestens eine Gruppe, ausgewählt unter -OC(O)O-, -NHC(O)NH-, und -C(O)OC(O)-, und/oder mindestens zwei Gruppen, ausgewählt unter einer Sauerstoffbrücke zwischen zwei Kohlenstoffatomen, -OC(O)O-, -C(O)NH-, -NHC(O)NH-, -NHC(O)O-, -C(O)OC(O)- und -C(O)O-, aufweist.
3. Ein Verfahren wie unter den Punkten 1 oder 2 definiert, worin das organische oder organisch modifizierte Material mindestens eine Verbindung aufweist, die sowohl einen organischen, über 2-Photonen- oder Mehrphotonen-Polymerisation polymerisierbaren Rest als auch eine biokompatible, biodegradierbare oder bioresorbierbare Gruppe aufweist.
3a. Ein Verfahren wie unter Punkt 3 definiert, worin die genannte Verbindung ausgewählt ist unter Verbindungen der Formel (A)

   R'-CHR"-O-C(O)-CR"'=CH₂ (A)

   worin der Rest R"' H oder CH₃ bedeutet und worin R'CHR" abgeleitet ist von einer Verbindung R'-CHR"OH, ausgewählt unter
   (a) Monosacchariden, nämlich Aldo- und Keto-Pentosen, -Hexosen und -Heptosen mit mindestens einer freien Hydroxygruppe, worin die nicht-freien Hydroxygruppen in geschützter Form, insbesondere als Ether oder Ester vorliegen,
      dimeren, oligomeren oder polymeren Kohlehydraten, die mindestens eines der genannten Monosaccharide enthalten,
   (b) monomeren oder oligomeren oder polymeren Zuckeralkoholen, erhältlich durch Reduktion der Aldehyd- oder Ketogruppe in den unter (a) genannten Molekülen,
   (c) gesättigten monomeren, oligomeren oder polymeren Mono-Hydroxysäuren sowie deren Lactonen und Lactiden, ausgewählt unter α-Hydroxysäuren mit vorzugsweise 3 oder 4 Kohlenstoffatomen und ω-Hydroxysäuren mit vorzugsweise 4-8 Kohlenstoffatomen.
4. Ein Verfahren wie unter den voranstehenden Punkten definiert, worin das Badmaterial weiterhin Gruppen oder Reste enthält, die einer anorganischen Vernetzung zugänglich oder bereits anorganisch vernetzt sind.
5. Ein Verfahren wie unter Punkt 4 definiert, worin die Gruppen oder Reste, die einer anorganischen Vernetzung zugänglich oder bereits anorganisch vernetzt sind, ausgewählt sind unter oligomerisierten oder polymerisierten Einheiten mit -O-Si-O- und -O-M-O-Bindungen mit M gleich Bor, Aluminium oder einem Übergangsmetall.
6. Ein Verfahren wie unter Punkt 5 definiert, worin das Badmaterial ein organisch modifiziertes Polysiloxan, das durch Hydrolyse und mindestens teilweise Kondensation eines Ausgangsmaterials erhältlich ist, welches mindestens ein Silan der Formel (I)

   R¹ₐR²_{b}SiX_{4-a-b} (I)

   sowie gegebenenfalls zusätzlich mindestens eine Verbindung des Typs

   M^{III}(OR³)₃ (II)

   und/oder des Typs

   M^{IIIV}(OR³)₄ (III)

   enthält, und/oder worin das Badmaterial mindestens eine noch nicht hydrolytisch kondensierte Verbindung (I) und gegebenenfalls Verbindungen des Typs (II) und/oder (III) aufweist, worin R¹ gleich oder verschieden ist und einen organischen, über Zwei- oder Mehrphotonenpolymerisation polymerisierbaren Rest darstellt, R² gleich oder verschieden ist und einen organischen, nicht auf diese Art und Weise polymerisierbaren Rest bedeutet, R³ eine Alkoxygruppe darstellt und X -OH oder ein unter Hydrolysebedingungen hydrolytisch kondensierbarer Rest ist, der Index a 0, 1, 2 oder 3 bedeutet, der Index b 0,1 oder 2 bedeutet und a+b zusammen 0, 1, 2 oder 3 sind.
7. Ein Verfahren wie unter Punkt 5 definiert, worin das Badmaterial ein organisch modifiziertes Polysiloxan, das durch Hydrolyse und mindestens teilweise Kondensation mindestens eines Silans der Formel (la)

   R¹ₐR²_{b}SiX_{4-a-b} (la)

   sowie gegebenenfalls zusätzlich mindestens einer Verbindung des Typs

   M^{III}(OR³)₃ (II)

   und/oder des Typs

   M^{IV}(OR³)₄ (III)

   erhältlich ist, und/oder mindestens eine noch nicht hydrolytisch kondensierte Verbindung (Ia) und gegebenenfalls Verbindungen des Typs (II) und/oder (III) aufweist, worin R¹ gleich oder verschieden ist und einen organischen, über Zwei- oder Mehrphotonenpolymerisation polymerisierbaren Rest darstellt, R² ein biokompatibler, biodegradierbarer oder bioresorbierbarer Rest ist bzw. einen solchen aufweist, R³ eine Alkoxygruppe darstellt und X -OH oder ein unter Hydrolysebedingungen hydrolytisch kondensierbarer Rest ist, der Index a 1, 2 oder 3 bedeutet, der Index b 1 oder 2 bedeutet und a+b zusammen 2 oder 3 sind.
8. Ein Verfahren wie unter Punkt 5 definiert, worin das Badmaterial ein organisch modifiziertes Polysiloxan, das durch Hydrolyse und mindestens teilweise Kondensation eines Ausgangsmaterials erhältlich ist, welches mindestens ein Silan der Formel (Ib)

   R¹ₐR²_{b}SiX_{4-a-b} (Ib)

   sowie gegebenenfalls zusätzlich mindestens einer Verbindung des Typs

   M^{III}(OR³)₃ (II)

   und/oder des Typs

   M^{IV}(OR³)₄ (III)

   enthält, und/oder worin das Badmaterial mindestens eine noch nicht hydrolytisch kondensierte Verbindung (Ib) und gegebenenfalls Verbindungen des Typs (II) und/oder (III) enthält, worin R¹ gleich oder verschieden ist und einen organischen, über Zwei- oder Mehrphotonenpolymerisation polymerisierbaren Rest darstellt, der zusätzlich einen biokompatiblen, biodegradierbaren oder bioresorbierbaren Anteil aufweist, R² einen organischen, nicht über Zwei- oder Mehrphotonenpolymerisation polymerisierbaren Rest darstellt, R³ eine Alkoxygruppe darstellt und X -OH oder ein unter Hydrolysebedingungen hydrolytisch kondensierbarer Rest ist, der Index a 1, 2 oder 3 bedeutet, der Index b 0.1 oder 2 bedeutet und a+b zusammen 1, 2 oder 3 sind.
9. Ein Verfahren wie unter Punkt 5 definiert, worin das Badmaterial mindestens eine rein organische Verbindung mit einem biokompatiblen, biodegradierbaren oder bioresorbierbaren Rest sowie entweder ein organisch modifiziertes Polysiloxan, das durch Hydrolyse und mindestens teilweise Kondensation mindestens eines Silans der Formel (la')

   R¹ₐR²_{b}SiX_{4-a-b} (Ia')

   sowie gegebenenfalls zusätzlich mindestens einer Verbindung des Typs

   M^{III}(OR³)₃ (II)

   und/oder des Typs

   M^{IV}(OR³)₄ (III)

   erhältlich ist, und/oder mindestens eine noch nicht hydrolytisch kondensierte Verbindung (Ia') und gegebenenfalls Verbindungen des Typs (II) und/oder (III) aufweist, worin R¹ gleich oder verschieden ist und einen organischen, über Zwei- oder Mehrphotonenpolymerisation polymerisierbaren Rest darstellt, R² ein biokompatibler, biodegradierbarer oder bioresorbierbarer Rest ist bzw. einen solchen aufweist, R³ eine Alkoxygruppe darstellt und X -OH oder ein unter Hydrolysebedingungen hydrolytisch kondensierbarer Rest ist, der Index a 1, 2 oder 3 bedeutet, der Index b 0, 1 oder 2 bedeutet und a+b zusammen 1, 2 oder 3 sind.
10. Ein Verfahren wie unter Punkt 5 definiert, worin das organisch modifizierte Material mindestens eine rein organische Verbindung, die einen organischen, über 2-Photonen- oder Mehrphotonen-Polymerisation polymerisierbaren Rest aufweist, sowie entweder ein organisch modifiziertes Polysiloxan, das durch Hydrolyse und mindestens teilweise Kondensation mindestens eines Silans der Formel (Ia)

   R¹ₐR²_{b}SiX_{4-a-b} (la")

   sowie gegebenenfalls zusätzlich mindestens einer Verbindung des Typs

   M^{III}(OR³)₃ (II)

   und/oder des Typs

   M^{IV}(OR³)₄ (III)

   erhältlich ist, und/oder mindestens eine noch nicht hydrolytisch kondensierte Verbindung (Ia") und gegebenenfalls Verbindungen des Typs (II) und/oder (III) enthält, worin R¹ gleich oder verschieden ist und einen organischen, über Zwei- oder Mehrphotonenpolymerisation polymerisierbaren Rest darstellt, R² ein biokompatibler, biodegradierbarer oder bioresorbierbarer Rest ist bzw. einen solchen aufweist, R³ eine Alkoxygruppe darstellt und X OH oder ein unter Hydrolysebedingungen hydrolytisch kondensierbarer Rest ist, der Index a 0, 1, 2 oder 3 bedeutet, der Index b 1 oder 2 bedeutet und a+b zusammen 1, 2 oder 3 sind.
11. Ein Verfahren wie unter Punkt 5 definiert, worin das organisch modifizierte Material mindestens ein organisch modifiziertes Polysiloxan, das durch Hydrolyse und mindestens teilweise Kondensation mindestens eines Silans der Formel (Ia')

   R¹ₐR²_{b}SiX_{4-a-b} (la')

   wie unter Punkt 9 definiert, und
   mindestens teilweise Kondensation mindestens eines Silans der Formel (Ia")

   R¹ₐR²_{b}SiX_{4-a-b} (Ia")

   wie unter Punkt 10 definiert,
   sowie gegebenenfalls zusätzlich mindestens einer Verbindung des Typs

   M^{III}(OR³)₃ (II)

   und/oder des Typs

   M^{IV}(OR³)₄ (III)

   erhalten wurde oder erhältlich ist, und/oder mindestens eine noch nicht hydrolytisch kondensierte Verbindung (Ia') wie unter Punkt 9 definiert und mindestens eine noch nicht hydrolytisch kondensierte Verbindung (Ia") wie unter Punkt 10 definiert und gegebenenfalls Verbindungen des Typs (II) und/oder (III) enthält.
12. Ein Verfahren wie unter Punkt 5 definiert, worin das organische oder organisch modifizierte Material mindestens eine rein organische Verbindung umfasst, die sowohl einen organischen, über 2-Photonen- oder Mehrphotonen-Polymerisation polymerisierbaren Rest als auch einen biokompatiblen, biodegradierbaren oder bioresorbierbaren Rest aufweist und das Badmaterial weiterhin ein Organopolysiloxan, das ausschließlich oder zusätzlich durch zumindest teilweise hydrolytische Kondensation mindestens eines Silans der Formel (I)

   R¹ₐR²_{b}SiX_{4-a-b} (I)

   sowie gegebenenfalls zusätzlich mindestens einer Verbindung des Typs

   M^{III}(OR³)₃ (II)

   und/oder des Typs

   M^{IV}(OR³)₄ (III)

   erhältlich ist, und/oder noch nicht hydrolytisch kondensierte Verbindungen (I) und gegebenenfalls (II) und/oder (III) enthält, wobei die Reste und Indices R¹, R², X und b wie unter Punkt 6 für Formel (I) oder wie unter Punkt 7 für Formel (la) definiert sind und der Index a 0, 1, 2 oder 3 ist.
12a. Ein Verfahren wie unter Punkt 12 definiert, worin die rein organische Verbindung, die sowohl einen organischen, über 2-Photonen- oder Mehrphotonen-Polymerisation polymerisierbaren Rest als auch einen biokompatiblen, biodegradierbaren oder bioresorbierbaren Rest aufweist, ausgewählt ist unter Verbindungen der Formel (A)

   R'-CHR"-O-C(O)-CR"'=CH₂ (A)

   worin der Rest R"' H oder CH₃ bedeutet und worin R'CHR" abgeleitet ist von einer Verbindung R'-CHR"OH, ausgewählt unter
   (a) Monosacchariden, nämlich Aldo- und Keto-Pentosen, -Hexosen und -Heptosen mit mindestens einer freien Hydroxygruppe, worin die nicht-freien Hydroxygruppen in geschützter Form, insbesondere als Ether oder Ester vorliegen,
      dimeren, oligomeren oder polymeren Kohlehydraten, die mindestens eines der genannten Monosaccharide enthalten,
   (b) monomeren oder oligomeren oder polymeren Zuckeralkoholen, erhältlich durch Reduktion der Aldehyd- oder Ketogruppe in den unter (a) genannten Molekülen,
   (c) gesättigten monomeren, oligomeren oder polymeren Mono-Hydroxysäuren sowie deren Lactonen und Lactiden, ausgewählt unter α-Hydroxysäuren mit vorzugsweise 3 oder 4 Kohlenstoffatomen und ω-Hydroxysäuren mit vorzugsweise 4-8 Kohlenstoffatomen.
12b. Ein Verfahren wie in Punkt 12 a definiert, worin das Silan der Formel (I) wie in Anspruch 6 definiert ist mit der Maßgabe, dass a nicht 0 sein kann, wobei a vorzugsweise 1 ist und stärker bevorzugt b außerdem 0 ist, während R¹ bevorzugt eine (Meth)Acrylatgruppe aufweist.
13. Ein Verfahren wie unter Punkt 12 oder Punkt 12a definiert, worin a 1, 2 oder 3 ist.
14. Ein Verfahren wie unter einem der Punkte 6 bis 13 definiert, worin R¹ ein eine nichtaromatische C=C-Doppelbindung, vorzugsweise eine einer Michaeladdition zugängliche Doppelbindung, enthaltender Rest ist und/oder worin X Wasserstoff, Halogen, Hydroxy, Alkoxy, Acyloxy oder NR³₂ mit R³ gleich Wasserstoff oder Niederalkyl ist.
15. Ein Verfahren wie unter Punkt 6 oder Punkt 8 definiert, worin R² ggf. substituiertes Alkyl, Aryl, Alkylaryl oder Arylalkyl ist, wobei die Kohlenstoffkette dieser Reste ggf. durch O, S, NH, COHN, COO, NHCOO unterbrochen sein kann, und/oder mindestens eine Gruppe enthält, die mit C=C-Doppelbindungen radikalisch eine Additionsreaktion eingehen kann.
16. Ein Verfahren wie unter einem der Punkte 5 bis 15 definiert, worin das Badmaterial ein organisch modifiziertes Polysiloxan, das ausschließlich oder zusätzlich durch Hydrolyse und mindestens teilweise Kondensation oder Co-Kondensation mindestens eines Silans der Formel (IV)

   SiX₄ (IV)

   erhältlich ist oder erhalten wurde, und/oder eine noch nicht hydrolytisch kondensierte Verbindung (IV) aufweist, worin X gleich oder verschieden ist und ein unter Hydrolysebedingungen hydrolytisch kondensierbarer Rest ist, wobei bis zu 2 oder sogar bis zu 3 Gruppen X außerdem OH bedeuten können.
17. Ein Verfahren wie unter einem der Punkte 5 bis 16 definiert, worin das Badmaterial ein organisch modifiziertes Polysiloxan, das ausschließlich oder zusätzlich durch Hydrolyse und mindestens teilweise Kondensation oder Co-Kondensation mindestens eines Silans der Formel (V)

   R¹ₐSiR²₄₋ₐ (V)

   erhältlich ist oder erhalten wurde, und/oder eine noch nicht hydrolytisch kondensierte Verbindung der Formel (V) aufweist, worin R¹ und R² die in Anspruch 6 für Formel (I) oder die in Anspruch 8 für Formel (Ia) angegebene Bedeutung haben.
18. Ein Verfahren wie unter einem der Punkte 5 bis 17 definiert, worin das Badmaterial ein organisch modifiziertes Polysiloxan, das ausschließlich oder zusätzlich durch Hydrolyse und mindestens teilweise Kondensation oder Co-Kondensation mindestens eines Silans der Formel (VI)

   R⁴ₐSi(OH)₄₋ₐ (VI)

   erhältlich ist oder erhalten wurde, und/oder eine noch nicht hydrolytisch kondensierte Verbindung (VI) aufweist, worin R⁴ gleich oder verschieden sein kann und entweder die Bedeutung von R¹ oder von R² wie in in Anspruch 6 für Formel (I) oder wie in Anspruch 9 für Formel (la) definiert besitzt oder ein geradkettiges, verzweigtes und/oder cyclisches Alkyl oder Aryl ist und worin der Index a 1, 2 oder 3 bedeutet, enthält.
19. Ein Verfahren wie unter einem der Punkte 5 bis 18 definiert, worin das Badmaterial ein organisch modifiziertes Polysiloxan, das ausschließlich oder zusätzlich durch Hydrolyse und mindestens teilweise Kondensation oder Co-Kondensation mindestens eines Silans der Formel (VII)

   R⁵ₐSiX₄₋ₐ (VII)

   erhältlich ist oder erhalten wurde, und/oder eine noch nicht hydrolytisch kondensierte Verbindung der Formel (VII) aufweist, worin R⁵ eine Gruppe ist oder aufweist, die radikalische an eine C=C-Doppelbindung addiert werden kann.
20. Ein Verfahren wie unter einem der Punkte 5 bis 19 definiert, dadurch gekennzeichnet, dass innerhalb des Formkörpers unterschiedliche Vernetzungsgrade erhalten werden, indem die Bestrahlung mit unterschiedlichen Intensitätsgradienten erfolgt, vorzugsweise durch Senken der Intensität und Aufweitung des Fokus'.
21. Ein Verfahren zum Herstellen einer Verbindung mit der Formel (A)

   R'-CHR"-O-C(O)-CR"'=CH₂ (A) worin der Rest R"' H oder CH₃ bedeutet und worin R'CHR" abgeleitet ist von einer Verbindung R'-CHR"OH, ausgewählt unter

   (a) Monosacchariden, nämlich Aldo- und Keto-Pentosen, -Hexosen und -Heptosen mit mindestens einer freien Hydroxygruppe, worin die nicht-freien Hydroxygruppen in geschützter Form, insbesondere als Ether oder Ester vorliegen,
      dimeren, oligomeren oder polymeren Kohlehydraten, die mindestens eines der genannten Monosaccharide enthalten,
   (b) monomeren oder oligomeren oder polymeren Zuckeralkoholen, erhältlich durch Reduktion der Aldehyd- oder Ketogruppe in den unter (a) genannten Molekülen,
   (c) gesättigten monomeren, oligomeren oder polymeren Mono-Hydroxysäuren sowie deren Lactonen und Lactiden, ausgewählt unter α-Hydroxysäuren mit vorzugsweise 3 oder 4 Kohlenstoffatomen und ω-Hydroxysäuren mit vorzugsweise 4-8 Kohlenstoffatomen,
   dadurch gekennzeichnet, dass eine Verbindung

   R'-CHR"OH

   mit einer Verbindung

   R^{IV}O-C(O)-CR"'=CH₂,

   worin R^{IV} ein Alkylrest mit vorzugsweise 1 bis 20, stärker bevorzugt mit 1 bis 10 Kohlenstoffatomen ist, der gegebenenfalls mit einer oder mehreren Gruppen O-C(O)-CR"'=CH₂ substituiert sein kann, unter Bedingungen umgesetzt wird, die das Reaktionsgleichgewicht zum Produkt hin verschieben.
21 a. Ein Verfahren wie unter Punkt 21 definiert, worin das Reaktionsgleichgewicht mit Hilfe eines Überschusses an der Verbindung R^{IV}O-C(O)-CR"'=CH₂ in der Reaktionsmischung oder durch einen Katalysator zum Produkt hin verschoben wird.
22. Ein dreidimensionaler selbsttragender und/oder substratgestützter Formkörper oder dreidimensionale Oberflächenstruktur, erzeugt mit Hilfe des Verfahrens gemäß einem der Punkte 4 bis 20, dadurch gekennzeichnet, dass er bzw. sie eine räumliche Oberflächenstruktur aufweist, die die Anbindung und Vermehrung von Zellen erlaubt.
23. Ein Formkörper oder eine Oberflächenstruktur wie unter Punkt 22 definiert mit daran haftenden lebenden Zellen und/oder anderen biologischen Komponenten, wie Proteinen, Enzymen, Peptiden, Aminosäuren, Antikörpern, DNA- oder RNA-Fragmenten.
24. Ein Formkörper oder eine Oberflächenstruktur wie unter Punkt 23 definiert, dadurch gekennzeichnet, dass der Formköper oder die Oberflächenstruktur Poren aufweist, die kleiner als die daran haftenden Zellen und/oder anderen biologischen Komponenten, wie Proteine, Enzyme, Peptide, Aminosäuren, Antikörper, DNA- oder RNA-Fragmente sind, oder dass der Formköper oder die Oberflächenstruktur Poren aufweist, die größer als die daran haftenden Zellen sind, oder dass der Formköper oder die Oberflächenstruktur Poren aufwest, die teilweise kleiner und teilweise größer als die daran haftenden Zellen sind, wobei die Poren vorzugsweise einen Durchmesser im Bereich von 20 und 500 µm aufweisen.
25. Die Verwendung eines Formkörpers oder einer Oberflächenstruktur, wie er/sie in einem der Punkte 22 bis 24 definiert wurden, als Matrix für einen Verbund aus von den Zellen und/oder anderen biologischen Komponenten, wie Proteinen, Enzymen, Peptiden, Aminosäuren, Antikörpern, DNA- oder RNA-Fragmenten, gebildeter extrazellulärer Matrix und diesen Zellen und/oder anderen biologischen Komponenten, wie Proteinen, Enzymen, Peptiden, Aminosäuren, Antikörpern, DNA- oder RNA-Fragmenten selbst, oder als Matrix für einen Verbund aus unterschiedlichen Arten von Zellen undloder anderen biologischen Komponenten, wie Proteinen, Enzymen, Peptiden, Aminosäuren, Antikörpern, DNA- oder RNA-Fragmenten, oder für sich nachbildende Organe oder Organbestandteile.
26. Die Verwendung eines Formkörpers oder einer Oberflächenstruktur, wie er/sie in einem der Punkte 22 bis 24 definiert wurden, als Matrix für Zellen zur Gewinnung von Sekretionsprodukten dieser Zellen.
27. Die Verwendung eines Formkörpers oder einer Oberflächenstruktur, wie er/sie in einem der Punkte 22 bis 24 definiert wurden, als Stützkörper für in einem beschädigten oder unvollständigen Gewebe nachreifenden Zellen und/oder einer Matrix für diese.
28. Die Verwendung eines Formkörpers oder einer Oberflächenstruktur, wie er/sie in einem der Punkte 22 bis 24 definiert wurden, als auf die Epitheloberfläche oder die Oberfläche eines Auges eines Menschen oder eines Tiers aufzubringendes oder zu implantierendes Depot zur Abgabe von Wirkstoffen.
29. Die Verwendung eines Formkörpers oder einer Oberflächenstruktur, wie er/sie in einem der Punkte 22 bis 24 definiert wurden, als Implantat in einen tierischen oder menschlichen Körper.
30. Die Verwendung wie in Punkt 29 definiert, worin das Implantat teilweise oder vollständig abbaubar ist.
31. Die Verwendung wie in Punkt 29 definiert, worin das Implantat als Matrix für einen Verbund aus von den Zellen und/oder anderen biologischen Komponenten, wie Proteinen, Enzymen, Peptiden, Aminosäuren, Antikörpern, DNA- oder RNA-Fragmenten gebildeter extrazellulärer Matrix und diesen Zellen selbst, oder für einen Verbund aus unterschiedlichen Arten von Zellen, oder für sich nachbildende Organe oder Organbestandteile dient.

## Patentansprüche

1. Verfahren zum Erzeugen von dreidimensionalen selbsttragenden und/oder substratgestützten Formkörpern oder von Strukturen auf Oberflächen durch ortsselektives Verfestigen eines flüssigen bis pastösen, organischen oder organisch modifizierten Materials innerhalb eines Bades aus diesem Material mit Hilfe von Zwei- oder Mehr-Photonen-Polymerisation, wobei das Material mindestens eine Verbindung aufweist, die sowohl einen organischen, über 2-Photonen- oder Mehrphotonen-Polymerisation polymerisierbaren Rest als auch eine biokompatible, biodegradierbare oder bioresorbierbare Gruppe aufweist, und/oder worin das Badmaterial weiterhin Gruppen oder Reste enthält, die einer anorganischen Vernetzung zugänglich oder bereits anorganisch vernetzt sind, mit der Maßgabe, dass in dem Material sowohl ein organischer, über 2-Photonen- oder Mehrphotonen-Polymerisation polymerisierbarer Rest als auch eine biokompatible, biodegradierbare oder bioresorbierbare Gruppe enthalten sein müssen.

2. Verfahren nach Anspruch 1, worin der biokompatible, biodegradierbare oder bioresorbierbare Rest ein solcher ist, der mindestens eine Gruppe aufweist, ausgewählt unter -O-, -OC(O)O-, -C(O)NH-, -NHC(O)NH-, -NHC(O)O-, -C(O)OC(O)- oder -C(O)O-, und/oder der unter physiologischen Bedingungen im menschlichen oder tierischen Körper oder durch den Befall von Mikroorganismen gespalten wird wobei der genannte Rest vorzugsweise mindestens eine Gruppe, ausgewählt unter -OC(O)O-, -NHC(O)NH-, und -C(O)OC(O)-, oder mindestens zwei Gruppen, ausgewählt unter einer Sauerstoffbrücke zwischen zwei Kohlenstoffatomen, -OC(O)O-, -C(O)NH-, -NHC(O)NH-, -NHC(O)O-, -C(O)OC(O)- und -C(O)O-, aufweist.

3. Verfahren nach Anspruch 1 oder 2, worin die genannte Verbindung ausgewählt ist unter Verbindungen der Formel (A)
R'-CHR"-O-C(O)-CR"'=CH₂ (A)
worin der Rest R"' H oder CH₃ bedeutet und worin R'CHR" abgeleitet ist von einer Verbindung R'-CHR"OH, ausgewählt unter
(a) Monosacchariden, nämlich Aldo- und Keto-Pentosen, -Hexosen und -Heptosen mit mindestens einer freien Hydroxygruppe, worin die nicht-freien Hydroxygruppen in geschützter Form vorliegen,
dimeren, oligomeren oder polymeren Kohlehydraten, die mindestens eines der genannten Monosaccharide enthalten,
(b) monomeren oder oligomeren oder polymeren Zuckeralkoholen, erhältlich durch Reduktion der Aldehyd- oder Ketogruppe in den unter (a) genannten Molekülen,
(c) gesättigten monomeren, oligomeren oder polymeren Mono-Hydroxysäuren sowie deren Lactonen und Lactiden, ausgewählt unter α-Hydroxysäuren und ω-Hydroxysäuren.

4. Verfahren nach einem der voranstehenden Ansprüche, worin das Badmaterial ein organisch modifiziertes Polysiloxan, das durch Hydrolyse und mindestens teilweise Kondensation eines Ausgangsmaterials erhältlich ist, welches mindestens ein Silan der Formel (I)
R¹ₐR²_{b}SiX_{4-a-b} (I)
sowie gegebenenfalls zusätzlich mindestens eine Verbindung des Typs
M^{III}(OR³)₃ (II)
und/oder des Typs
M^{IV}(OR³)₄ (III)
enthält, und/oder worin das Badmaterial mindestens eine noch nicht hydrolytisch kondensierte Verbindung (I) und gegebenenfalls Verbindungen des Typs (II) und/oder (III) aufweist, worin M ausgewählt ist unter Bor, Aluminium und einem Übergangsmetall, worin R¹ gleich oder verschieden ist und einen organischen, über Zwei- oder Mehrphotonenpolymerisation polymerisierbaren Rest darstellt, R² gleich oder verschieden ist und einen organischen, nicht auf diese Art und Weise polymerisierbaren Rest bedeutet, R³ eine Alkoxygruppe darstellt und X -OH oder ein unter Hydrolysebedingungen hydrolytisch kondensierbarer Rest ist, der Index a 0, 1, 2 oder 3 bedeutet, der Index b 0, 1 oder 2 bedeutet und a+b zusammen 0, 1, 2 oder 3 sind.

5. Verfahren nach einem der Ansprüche 1 bis 3, worin das Badmaterial ein organisch modifiziertes Polysiloxan, das durch Hydrolyse und mindestens teilweise Kondensation mindestens eines Silans der Formel (la)
R¹ₐR²_{b}SiX_{4-a-b} (Ia)
sowie gegebenenfalls zusätzlich mindestens einer Verbindung des Typs
M^{III}(OR³)₃ (II)
und/oder des Typs
M^{IV}(OR³)₄ (III)
erhältlich ist, und/oder mindestens eine noch nicht hydrolytisch kondensierte Verbindung (la) und gegebenenfalls Verbindungen des Typs (II) und/oder (III) aufweist, worin M ausgewählt ist unter Bor, Aluminium und einem Übergangsmetall, worin R¹ gleich oder verschieden ist und einen organischen, über Zwei- oder Mehrphotonenpolymerisation polymerisierbaren Rest darstellt, R² ein biokompatibler, biodegradierbarer oder bioresorbierbarer Rest ist bzw. einen solchen aufweist, R³ eine Alkoxygruppe darstellt und X -OH oder ein unter Hydrolysebedingungen hydrolytisch kondensierbarer Rest ist, der Index a 1, 2 oder 3 bedeutet, der Index b 1 oder 2 bedeutet und a+b zusammen 2 oder 3 sind.

6. Verfahren nach einem der Ansprüche 1 bis 3, worin das Badmaterial ein organisch modifiziertes Polysiloxan, das durch Hydrolyse und mindestens teilweise Kondensation eines Ausgangsmaterials erhältlich ist, welches mindestens ein Silan der Formel (Ib)
R¹ₐR²_{b}SiX_{4-a-b} (Ib)
sowie gegebenenfalls zusätzlich mindestens eine Verbindung des Typs
M^{III}(OR³)₃ (II)
und/oder des Typs
M^{IV}(OR³)₄ (III)
enthält, und/oder worin das Badmaterial mindestens eine noch nicht hydrolytisch kondensierte Verbindung (Ib) und gegebenenfalls Verbindungen des Typs (II) und/oder (III) enthält, worin M ausgewählt ist unter Bor, Aluminium und einem Übergangsmetall, worin R¹ gleich oder verschieden ist und einen organischen, über Zwei- oder Mehrphotonenpolymerisation polymerisierbaren Rest darstellt, der zusätzlich einen biokompatiblen, biodegradierbaren oder bioresorbierbaren Anteil aufweist, R² einen organischen, nicht über Zwei- oder Mehrphotonenpolymerisation polymerisierbaren Rest darstellt, R³ eine Alkoxygruppe darstellt und X -OH oder ein unter Hydrolysebedingungen hydrolytisch kondensierbarer Rest ist, der Index a 1, 2 oder 3 bedeutet, der Index b 0, 1 oder 2 bedeutet und a+b zusammen 1, 2 oder 3 sind.

7. Verfahren nach einem der Ansprüche 1 bis 3, worin das Badmaterial mindestens eine rein organische Verbindung mit einem biokompatiblen, biodegradierbaren oder bioresorbierbaren Rest sowie entweder ein organisch modifiziertes Polysiloxan, das durch Hydrolyse und mindestens teilweise Kondensation mindestens eines Silans der Formel (la')
R¹ₐR²_{b}SiX_{4-a-b} (Ia')
sowie gegebenenfalls zusätzlich mindestens einer Verbindung des Typs
M^{III}(OR³)₃ (II)
und/oder des Typs
M^{IV}(OR³)₄ (III)
erhältlich ist, und/oder mindestens eine noch nicht hydrolytisch kondensierte Verbindung (la') und gegebenenfalls Verbindungen des Typs (II) und/oder (III) aufweist, worin M ausgewählt ist unter Bor, Aluminium und einem Übergangsmetall, worin R¹ gleich oder verschieden ist und einen organischen, über Zwei- oder Mehrphotonenpolymerisation polymerisierbaren Rest darstellt, R² ein biokompatibler, biodegradierbarer oder bioresorbierbarer Rest ist bzw. einen solchen aufweist, R³ eine Alkoxygruppe darstellt und X -OH oder ein unter Hydrolysebedingungen hydrolytisch kondensierbarer Rest ist, der Index a 1, 2 oder 3 bedeutet, der Index b 0, 1 oder 2 bedeutet und a+b zusammen 1, 2 oder 3 sind.

8. Verfahren nach einem der Ansprüche 1 bis 3, worin das organisch modifizierte Material mindestens eine rein organische Verbindung, die einen organischen, über 2-Photonen- oder Mehrphotonen-Polymerisation polymerisierbaren Rest aufweist, sowie entweder ein organisch modifiziertes Polysiloxan, das durch Hydrolyse und mindestens teilweise Kondensation mindestens eines Silans der Formel (la")
R¹ₐR²_{b}SiX_{4-a-b} (Ia")
sowie gegebenenfalls zusätzlich mindestens einer Verbindung des Typs
M^{III}(OR³)₃ (II)
und/oder des Typs
M^{IV}(OR³)₄ (III)
erhältlich ist, und/oder mindestens eine noch nicht hydrolytisch kondensierte Verbindung (Ia") und gegebenenfalls Verbindungen des Typs (II) und/oder (III) enthält, worin M ausgewählt ist unter Bor, Aluminium und einem Übergangsmetall, worin R¹ gleich oder verschieden ist und einen organischen, über Zwei- oder Mehrphotonenpolymerisation polymerisierbaren Rest darstellt, R² ein biokompatibler, biodegradierbarer oder bioresorbierbarer Rest ist bzw. einen solchen aufweist, R³ eine Alkoxygruppe darstellt und X -OH oder ein unter Hydrolysebedingungen hydrolytisch kondensierbarer Rest ist, der Index a 0, 1, 2 oder 3 bedeutet, der Index b 1 oder 2 bedeutet und a+b zusammen 1, 2 oder 3 sind.

9. Verfahren nach einem der Ansprüche 1 bis 3, worin das organisch modifizierte Material mindestens ein organisch modifiziertes Polysiloxan, das durch Hydrolyse und mindestens teilweise Kondensation mindestens eines Silans der Formel (la')
R¹ₐR²_{b}SiX_{4-a-b} (Ia') wie in Anspruch 7 definiert, und
mindestens teilweise Kondensation mindestens eines Silans der Formel (la")
R¹ₐR²_{b}SiX_{4-a-b} (Ia") wie in Anspruch 8 definiert,
sowie gegebenenfalls zusätzlich mindestens eine Verbindung des Typs
M^{III}(OR³)₃ (II)
und/oder des Typs
M^{IV}(OR³)₄ (III) wie in Anspruch 4 definiert,
erhalten wurde oder erhältlich ist, und/oder mindestens eine noch nicht hydrolytisch kondensierte Verbindung (la') wie in Anspruch 7 definiert und mindestens eine noch nicht hydrolytisch kondensierte Verbindung (la") wie in Anspruch 8 definiert und gegebenenfalls Verbindungen des Typs (II) und/oder (III) enthält.

10. Verfahren nach einem der Ansprüche 1 bis 3, worin das organische oder organisch modifizierte Material mindestens eine rein organische Verbindung umfasst, die sowohl einen organischen, über 2-Photonen- oder Mehrphotonen-Polymerisation polymerisierbaren Rest als auch einen biokompatiblen, biodegradierbaren oder bioresorbierbaren Rest aufweist und das Badmaterial weiterhin ein Organopolysiloxan, das ausschließlich oder zusätzlich durch zumindest teilweise hydrolytische Kondensation mindestens eines Silans der Formel (I)
R¹ₐR²_{b}SiX_{4-a-b} (I)
sowie gegebenenfalls zusätzlich mindestens einer Verbindung des Typs
M^{III}(OR³)₃ (II)
und/oder des Typs
M^{IV}(OR³)₄ (III)
erhältlich ist, und/oder noch nicht hydrolytisch kondensierte Verbindungen (I) und gegebenenfalls (II) und/oder (III) enthält, wobei M ausgewählt ist unter Bor, Aluminium und einem Übergangsmetall, R³ eine Alkoxygruppe darstellt, die Reste und Indices R¹, R², X und b wie in Anspruch 4 für Formel (I) oder wie in Anspruch 5 für Formel (la) definiert sind und der Index a 0, 1, 2 oder 3 ist.

11. Verfahren nach einem der Ansprüche 4 bis 10, worin R¹ in den Formeln (I), (la), (la'), (la") oder (Ib) ein eine nichtaromatische C=C-Doppelbindung, vorzugsweise eine einer Michaeladdition zugängliche Doppelbindung, enthaltender Rest ist und/oder worin X Wasserstoff, Halogen, Hydroxy, Alkoxy, Acyloxy oder NR³₂ mit R³ gleich Wasserstoff oder Alkyl mit 1 bis 6, vorzugsweise 1 oder 2 Kohlenstoffatomen ist.

12. Verfahren nach einem der voranstehenden Ansprüche, **dadurch gekennzeichnet, dass** innerhalb des Formkörpers unterschiedliche Vernetzungsgrade erhalten werden, indem die Bestrahlung mit unterschiedlichen Intensitätsgradienten erfolgt, vorzugsweise durch Senken der Intensität und Aufweitung des Fokus'.

13. Dreidimensionaler selbsttragender und/oder substratqestützter Formkörper oder dreidimensionale Oberflächenstruktur, erzeugt mit Hilfe des Verfahrens gemäß einem der Ansprüche1 bis 12, der bzw. die eine räumliche Oberflächenstruktur aufweist, an welche mindestens eine biologische Komponente angebunden vorliegt, oder worin die räumliche Oberflächenstruktur so ausgestaltet ist, dass sie sich zur Anbindung einer biologischen Komponente eignet, wobei die biologische Komponente jeweils ausgewählt ist unter pflanzlichen, tierischen und menschlichen Zellen, Proteinen, Enzymen, Peptiden, Aminosäuren, Antikörpern, DNA- und RNA-Fragmenten.

14. Verwendung eines Formkörpers oder einer Oberflächenstruktur nach Anspruch 13
- als Matrix für einen Verbund aus von Zellen gebildeter extrazellulärer Matrix, und diesen Zellen selbst,
- für einen Verbund aus unterschiedlichen Arten von Zellen oder für sich nachbildende Organe oder Organbestandteile,
- als Matrix für Zellen zur Gewinnung von Sekretionsprodukten dieser Zellen,
- als Stützkörper für in einem beschädigten oder unvollständigen Gewebe nachreifenden Zellen und/oder einer Matrix für diese,
- als auf eine Epitheloberfläche oder eine Oberfläche eines Auges eines Menschen oder Tieres aufzubringendes oder zu implantierendes Depot zur Abgabe von Wirkstoffen, oder
- als ggf. teilweise oder vollständig abbaubares Implantat zum Einbringen in einen tierischen oder menschlichen Körper.

## Claims

1. Process for the production of three-dimensional, self-supporting and/or substrate-supported formed pieces or of structures on surfaces by means of a site-selective solidification of a liquid to pasty, organic or organically modified material within a bath of this material by means of two-photon or multiphoton polymerization, wherein the material comprises at least one compound, which has both an organic radical polymerizable via two-photon or multiphoton polymerization and a biocompatible, biodegradable or bioresorbable group, and/or wherein the bath material additionally contains groups or radicals that are accessible for an inorganic crosslinking or are already inorganically crosslinked, provided that both an organic radical polymerizable via two-photon or multiphoton polymerization and a biocompatible, biodegradable or bioresorbable group must be contained in the material.

2. Process in accordance with claim 1, wherein the biocompatible, biodegradable or bioresorbable radical is such a radical that has at least one group, which is selected from among -O-, -OC(O)O-, -C(O)NH-, -NHC(O)NH-, -NHC(O)O-, -C(O)OC(O)- and -C(O)O-, and/or that is split under physiological conditions in the human or animal body or due to the infestation with microorganisms, wherein the said radical preferably has at least one group, selected from among -OC(O)O-, -NHC(O)NH-, and -C(O)OC(O)-, or at least two groups, selected from among an oxygen bridge between two carbon atoms, -OC(O)O-, -C(O)NH-, -NHC(O)NH-, -NHC(O)O-, -C(O)OC(O)- and -C(O)O-.

3. Process in accordance with claim 1 or 2, wherein said compound is selected from among compounds of formula (A)
R'-CHR"-O-C(O)-CR"'-CH₂ (A)
wherein the radical R'" is H or CH₃ and wherein R'CHR" is derived from a compound R'-CHR"OH, selected from among
(a) monosaccharides, namely aldo- and keto-pentoses, -hexoses and -heptoses with at least one free hydroxyl group, wherein the non-free hydroxyl groups are present in a protected form,
dimeric, oligomeric or polymeric carbohydrates, which contain at least one of said monosaccharides,
(b) monomeric or oligomeric or polymeric sugar alcohols, obtainable by means of reduction of the aldehyde or keto group in the molecules mentioned under (a),
(c) saturated monomeric, oligomeric or polymeric monohydroxy acids as well as their lactones and lactides, selected from among α-hydroxy acids and ω-hydroxy acids.

4. Process in accordance with any of the preceding claims, wherein the bath material contains an organically modified polysiloxane, which is obtainable by means of hydrolysis and at least partial condensation of a starting material, which contains at least one silane of formula (I)
R¹ₐR²_{b}SiX_{4-a-b} (I)
as well as optionally additionally at least one compound of the type
M^{III}(OR³)₃ (II)
and/or of the type
M^{IV}(OR³)₄ (III)
and/or wherein the bath material has at least one not yet hydrolytically condensed compound (I) and optionally compounds of type (II) and/or (III), wherein M is selected from boron, aluminum and a transitional metal, wherein R¹ is identical or different and represents an organic radical polymerizable via two- or multiphoton polymerization, R² is identical or different and is an organic radical not polymerizable in this way, R³ represents an alkoxy group, and X is -OH or a radical hydrolytically condensable under conditions of hydrolysis, subscript a is 0, 1, 2 or 3, subscript b is 0, 1 or 2 and a+b together are 0, 1, 2 or 3.

5. Process in accordance with any of claims 1 to 3, wherein the bath material contains an organically modified polysiloxane, which is obtainable by means of hydrolysis and at least partial condensation of at least one silane of formula (Ia)
R¹ₐR²_{b}SiX_{4-a-b} (Ia)
as well as optionally additionally at least one compound of the type
M^{III}(OR³)₃ (II)
and/or of the type
M^{IV}(OR³)₄ (III)
and/or at least one not yet hydrolytically condensed compound (Ia) and optionally compounds of type (II) and/or (III), wherein M is selected from boron, aluminum and a transitional metal, wherein R¹ is identical or different and represents an organic radical polymerizable via two- or multiphoton polymerization, R² is a biocompatible, biodegradable or bioresorbable radical or has such a radical, R³ represents an alkoxy group and X is -OH or a radical hydrolytically condensable under conditions of hydrolysis, subscript a is 1, 2 or 3, subscript b is 1 or 2 and a+b together are 2 or 3.

6. Process in accordance with any of claims 1 to 3, wherein the bath material contains an organically modified polysiloxane, which is obtainable by means of hydrolysis and at least partial condensation of a starting material, which contains at least one silane of formula (Ib)
R¹ₐR²_{b}SiX_{4-a-b} (Ib)
as well as optionally additionally at least one compound of the type
M^{III}(OR³)₃ (II)
and/or of the type
M^{IV}(OR³)₄ (III)
and/or wherein the bath material contains at least one not yet hydrolytically condensed compound (Ib) and optionally compounds of type (II) and/or (III), wherein M is selected from boron, aluminum and a transitional metal, wherein R¹ is identical or different and represents an organic radical polymerizable via two- or multiphoton polymerization, which additionally has a biocompatible, biodegradable or bioresorbable portion, R² represents an organic radical not polymerizable via two- or multiphoton polymerization, R³ represents an alkoxy group and X is -OH or a radical hydrolytically condensable under conditions of hydrolysis, subscript a is 1, 2 or 3, subscript b is 0, 1 or 2 and a+b together are 1, 2 or 3.

7. Process in accordance with any of claims 1 to 3, wherein the bath material has at least one purely organic compound with a biocompatible, biodegradable or bioresorbable radical as well as either an organically modified polysiloxane, which is obtainable by means of hydrolysis and at least partial condensation of at least one silane of formula (Ia')
R¹ₐR²_{b}SiX_{4-a-b} (Ia')
as well as optionally additionally at least one compound of the type
M^{III}(OR³)₃ (II)
and/or of the type
M^{IV}(OR³)₄ (III)
and/or at least one not yet hydrolytically condensed compound (la') and optionally compounds of type (II) and/or (III), wherein M is selected from boron, aluminum and a transitional metal, wherein R¹ is identical or different and represents an organic radical polymerizable via two- or multiphoton polymerization, R² is a biocompatible, biodegradable or bioresorbable radical or has such a radical, R³ represents an alkoxy group and X is -OH or a radical hydrolytically condensable under conditions of hydrolysis, subscript a is 1, 2 or 3, subscript b is 0, 1 or 2, and a+b together are 1, 2 or 3.

8. Process in accordance with any of claims 1 to 3, wherein the organically modified material contains at least one purely organic compound, which has an organic radical polymerizable via two-photon or multiphoton polymerization, as well as either an organically modified polysiloxane, which is obtainable by means of hydrolysis and at least partial condensation of at least one silane of formula (Ia")
R¹ₐR²_{b}SiX_{4-a-b} (Ia") as well as optionally additionally at least one compound of the type
M^{III}(OR³)₃ (II)
and/or of the type
M^{IV}(OR³)₄ (III)
and/or at least one not yet hydrolytically condensed compound (la") and optionally compounds of type (II) and/or (III), wherein M is selected from boron, aluminum and a transitional metal, wherein R¹ is identical or different and represents an organic radical polymerizable via two- or multiphoton polymerization, R² is a biocompatible, biodegradable or bioresorbable radical or has such a radical, R³ represents an alkoxy group and X is -OH or a radical hydrolytically condensable under conditions of hydrolysis, subscript a is 0, 1, 2 or 3, subscript b is 1 or 2, and a+b together are 1, 2 or 3.

9. Process in accordance with any of claims 1 to 3, wherein the organically modified material contains
at least one organically modified polysiloxane, which was obtained or is obtainable by means of hydrolysis and at least partial condensation of at least one silane of formula (Ia')
R¹ₐR²_{b}SiX_{4-a-b} (Ia'),
as defined in claim 7,
and
at least partial condensation of at least one silane of formula (Ia")
R¹ₐR²_{b}SiX_{4-a-b} (Ia"),
as defined in claim 8,
as well as optionally additionally at least one compound of the type
M^{III}(OR³)₃ (II)
and/or of the type
M^{iIV}(OR³)₄ (III)
as defined in claim 4,
and/or at least one not yet hydrolytically condensed compound (la') as defined in claim 7 and at least one not yet hydrolytically condensed compound (la") as defined in claim 8 and optionally compounds of type (II) and/or (III).

10. Process in accordance with any of claims 1 to 3, wherein the organic or organically modified material comprises at least one purely organic compound, which has both an organic radical polymerizable via two-photon or multiphoton polymerization and a biocompatible, biodegradable or bioresorbable radical, and the bath material additionally contains an organopolysiloxane, which is obtainable exclusively or additionally by means of at least partial hydrolytic condensation of at least one silane of formula (I)
R¹ₐR²_{b}SiX_{4-a-b} (I)
as well as optionally additionally at least one compound of the type
M^{III}(OR³)₃ (II)
and/or of the type
M^{IV}(OR³)₄ (III)
and/or not yet hydrolytically condensed compounds (I) and optionally (II) and/or (III), wherein M is selected from boron, aluminum and a transitional metal, whereby the radicals and subscripts R¹, R², X and b are defined as in claim 4 for formula (I) or in claim 5 for formula (Ia) and subscript a is 0, 1, 2 or 3.

11. Process in accordance with any of claims 4 to 10, wherein R¹ in formulae (I), (Ia), (Ia'), (Ia") or (Ib) is a radical containing a nonaromatic C=C double bond, preferably a double bond available for a Michael addition, and/or wherein X is hydrogen, halogen, hydroxy, alkoxy, acyloxy or NR³₂ with R³ equal to hydrogen or alkyl with 1 to 6, preferably 1 or 2 carbon atoms.

12. Process in accordance with any of the preceding claims, **characterized in that** different degrees of crosslinking are obtained within the molding by the radiation being carried out with different intensity gradients, and preferably by lowering the intensity and expanding the focus.

13. Three-dimensional, self-supporting and/or substrate-supported, formed piece or three-dimensional surface structure, produced by means of the process in accordance with any of claims 1 to 12, **characterized in that** it has a three-dimensional surface structure, to which at least one biological component is bound, or wherein the three-dimensional surface structure is designed such that it is suitable for binding a biological component, wherein the biological component in each case is selected from among plant, animal and human cells, proteins, enzymes, peptides, amino acids, antibodies, DNA fragments and RNA fragments.

14. Use of a formed piece or a surface structure in accordance with claim 13:
- as a matrix for a composite of an extracellular matrix formed by cells, and for these cells themselves,
- for a composite consisting of different types of cells or for reproducing organs or organ components,
- as a matrix for cells for obtaining secretion products of these cells,
- as a support body for cells maturing in damaged or incomplete tissue and/or in a matrix for these,
- as a depot to be applied or implanted onto an epithelial surface or a surface of an eye of a human or animal for the release of active ingredients, or
- as a optionally partly or completely degradable implant for insertion into an animal or human body.

## Revendications

1. Procédé de production de corps façonnés tridimensionnels, autoportants et/ou supportés par un substrat, ou de structures sur des surfaces, par solidification à sélectivité locale d'un matériau liquide à pâteux, organique ou modifié organiquement, à l'intérieur d'un bain de ce matériau, par polymérisation biphotonique ou multiphotonique, le matériau présentant au moins un composé qui contient à la fois un reste organique polymérisable par polymérisation biphotonique ou multiphotonique, et un groupe biocompatible, biodégradable ou biorésorbable, et/ou le matériau du bain renferme en outre des groupes ou des restes qui peuvent être réticulés inorganiquement ou qui sont déjà réticulés inorganiquement, avec la condition qu'un reste organique, polymérisable par polymérisation biphotonique ou multiphotonique, ainsi qu'un groupe biocompatible, biodégradable ou biorésorbable doivent être contenus dans le matériau.

2. Procédé selon la revendication 1, dans lequel le reste biocompatible, biodégradable ou biorésorbable est un reste qui présente au moins un groupe, sélectionné parmi -O-, -OC(O)O-, -C(O)NH-, -NHC(O)NH-, -NHC (0) 0-, - C(O)OC(O)- ou -C(O)O-, et/ou qui est craqué dans des conditions physiologiques dans le corps humain ou animal ou par l'attaque de microorganismes, ledit reste présentant de préférence au moins un groupe, choisi parmi -OC(O)O-, -NHC(O)NH-, et -C(O)OC(O)-, ou au moins deux groupes, choisis parmi un pont d'oxygène entre deux atomes de carbone, -OC(O)O-, -C(O)NH-, -NHC(O)NH-, -NHC(O)O-, -C(O)OC(O)- et -C(O)O-.

3. Procédé selon la revendication 1 ou 2, dans lequel ledit composé est sélectionné parmi les composés de la formule (A)
R'-CHR"-O-C(O)-CR"'=CH₂ (A)
dans laquelle le reste R"' représente H ou CH₃ et dans laquelle R'CHR" est dérivé d'un composé R'-CHR"OH, sélectionné parmi
(a) des monosaccharides, notamment des aldo- et des céto-pentoses, hexoses et heptoses, avec au moins un groupe hydroxy libre, dans lesquels les groupes hydroxy non libres sont présents sous forme protégée,
des hydrates de carbone dimères, oligomères ou polymères, qui contiennent au moins un desdits monosaccharides,
(b) des alcools de sucre monomères ou oligomères ou polymères, qui peuvent être obtenus par réduction du groupe aldéhyde ou cétone en molécules mentionnées sous (a),
(c) des acides mono-hydroxyliques monomères, oligomères ou polymères saturés, ainsi que leurs lactones et lactides, sélectionnés parmi les acides α-hydroxyliques et les acides ω-hydroxyliques.

4. Procédé selon l'une quelconque des revendications précédentes, dans lequel le matériau du bain présente un polysiloxane organiquement modifié, qui peut être obtenu par hydrolyse et condensation au moins partielle d'un matériau de départ, qui contient au moins un silane de la formule (I)
R¹ₐR²_{b}SiX_{4-a-b} (I)
ainsi qu'éventuellement en outre au moins un composé du type
M^{III}(OR³)₃ (II)
et/ou du type
M^{IV}(OR³)₄ (III),
et/ou dans lequel le matériau du bain présente au moins un composé (I) pas encore condensé hydrolytiquement et éventuellement des composés du type (II) et/ou (III), dans lequel M est sélectionné parmi borure, aluminium et un métal transitoire, dans lequel R¹ est identique ou différent et représente un reste organique polymérisable par polymérisation biphotonique ou multiphotonique, R² est identique ou différent et représente un reste organique non polymérisable de cette manière, R³ représente un groupe alcoxy et X est-OH ou un reste hydrolytiquement condensable dans des conditions d'hydrolyse, l'indice a représente 0, 1, 2 ou 3, l'indice b représente 0, 1 ou 2 et a+b sont ensemble 0, 1, 2 ou 3.

5. Procédé selon l'une quelconque des revendications 1 à 3, dans lequel le matériau du bain présente un polysiloxane organiquement modifié, qui peut être obtenu par hydrolyse et condensation au moins partielle d'au moins un silane de la formule (Ia)
R¹ₐR²_{b}SiX_{4-a-b} (Ia)
ainsi qu'éventuellement en outre au moins un composé du type
M^{III}(OR³)₃ (II)
et/ou du type
M^{IV}(OR³)₄ (III),
et/ou au moins un composé (Ia) pas encore condensé hydrolytiquement et éventuellement des composés du type (II) et/ou (III), dans lequel M est sélectionné parmi borure, aluminium et un métal transitoire, dans lequel R¹ est identique ou différent et représente un reste organique polymérisable par polymérisation biphotonique ou multiphotonique, R² est un reste biocompatible, biodégradable ou biorésorbable ou en contient un, R³ représente un groupe alcoxy et X est -OH ou un reste hydrolytiquement condensable dans des conditions d'hydrolyse, l'indice a représente 1, 2 ou 3, l'indice b représente 1 ou 2 et a+b sont ensemble 2 ou 3.

6. Procédé selon l'une quelconque des revendications 1 à 3, dans lequel le matériau du bain présente un polysiloxane organiquement modifié, qui peut être obtenu par hydrolyse et condensation au moins partielle d'un matériau de départ, qui contient au moins un silane de la formule (Ib)
R¹ₐR²_{b}SiX_{4-a-b} (Ib)
ainsi qu'éventuellement en outre au moins un composé du type
M^{III}(OR³)₃ (II)
et/ou du type
M^{IV}(OR³)₄ (III),
et/ou dans lequel le matériau du bain présente au moins un composé (Ib) pas encore condensé hydrolytiquement et éventuellement des composés du type (II) et/ou (III), dans lequel M est sélectionné parmi borure, aluminium et un métal transitoire, dans lequel R¹ est identique ou différent et représente un reste organique polymérisable par polymérisation biphotonique ou multiphotonique, qui présente en outre une fraction biocompatible, biodégradable ou biorésorbable, R² représente un reste organique non polymérisable par polymérisation biphotonique ou multiphotonique, R³ représente un groupe alcoxy et X est -OH ou un reste hydrolytiquement condensable dans des conditions d'hydrolyse, l'indice a représente 1, 2 ou 3, l'indice b représente 0, 1 ou 2 et a+b sont ensemble 1, 2 ou 3.

7. Procédé selon l'une quelconque des revendications 1 à 3, dans lequel le matériau du bain présente au moins un composé purement organique avec un reste biocompatible, biodégradable ou biorésorbable ainsi que soit un polysiloxane organiquement modifié, qui peut être obtenu par hydrolyse et condensation au moins partielle d'un silane de la formule (Ia')
R¹ₐR²_{b}SiX_{4-a-b} (Ia')
ainsi qu'éventuellement en outre au moins un composé du type
M^{III}(OR³)₃ (II)
et/ou du type
M^{IV}(OR³)₄ (III),
et/ou au moins un composé (Ia') pas encore condensé hydrolytiquement et éventuellement des composés du type (II) et/ou (III), dans lequel M est sélectionné parmi borure, aluminium et un métal transitoire, dans lequel R¹ est identique ou différent et représente un reste organique polymérisable par polymérisation biphotonique ou multiphotonique, R² représente un reste biocompatible, biodégradable ou biorésorbable ou en contient un, R³ représente un groupe alcoxy et X est -OH ou un reste hydrolytiquement condensable dans des conditions d'hydrolyse, l'indice a représente 1, 2 ou 3, l'indice b représente 0, 1 ou 2 et a+b sont ensemble 1, 2 ou 3.

8. Procédé selon l'une quelconque des revendications 1 à 3, dans lequel le matériau organiquement modifié contient au moins un composé purement organique, qui présente un reste organique polymérisable par polymérisation biphotonique ou multiphotonique, ainsi que soit un polysiloxane organiquement modifié, qui peut être obtenu par hydrolyse et condensation au moins partielle d'au moins un silane de la formule (Ia")
R¹ₐR²_{b}SiX_{4-a-b} (Ia")
ainsi qu'éventuellement en outre au moins un composé du type
M^{III}(OR³)₃ (II)
et/ou du type
M^{IV}(OR³)₄ (III),
et/ou au moins un composé (Ia") pas encore condensé hydrolytiquement et éventuellement des composés du type (II) et/ou (III), dans lequel M est sélectionné parmi borure, aluminium et un métal transitoire, dans lequel R¹ est identique ou différent et représente un reste organique polymérisable par polymérisation biphotonique ou multiphotonique, R² représente un reste biocompatible, biodégradable ou biorésorbable ou en contient un, R³ représente un groupe alcoxy et X est -OH ou un reste hydrolytiquement condensable dans des conditions d'hydrolyse, l'indice a représente 0, 1, 2 ou 3, l'indice b représente 1 ou 2 et a+b sont ensemble 1, 2 ou 3.

9. Procédé selon l'une quelconque des revendications 1 à 3, dans lequel le matériau organiquement modifié contient au moins un polysiloxane organiquement modifié, qui a été ou qui peut être obtenu par hydrolyse et condensation au moins partielle d'au moins un silane de la formule (Ia')
R¹ₐR²_{b}SiX_{4-a-b} (Ia')
tel que défini dans la revendication 7, et
par condensation au moins partielle d'au moins un silane de la formule (Ia")
R¹ₐR²_{b}SiX_{4-a-b} (Ia")
tel que défini dans la revendication 8,
ainsi qu'éventuellement en outre au moins un composé du type
M^{III}(OR³)₃ (II)
et/ou du type
M^{IV}(OR³)₄ (III),
tel que défini dans la revendication 4,
et/ou au moins un composé (Ia') pas encore condensé hydrolytiquement tel que défini dans la revendication 7 et au moins un composé (Ia") pas encore condensé hydrolytiquement tel que défini dans la revendication 8 et éventuellement des composés du type (II) et/ou (III).

10. Procédé selon l'une quelconque des revendications 1 à 3, dans lequel le matériau organique ou organiquement modifié comprend au moins un composé purement organique, qui présente aussi bien un reste organique polymérisable par polymérisation biphotonique ou multiphotonique qu'un reste biocompatible, biodégradable ou biorésorbable et le matériau du bain contient en outre un organopolysiloxane, qui peut être obtenu exclusivement ou complémentairement par condensation hydrolytique au moins partielle d'au moins un silane de la formule (I)
R¹ₐR²_{b}SiX₄₋ₐ₋₁ (I)
ainsi qu'éventuellement en outre au moins un composé du type
M^{III}(OR³)₃ (II)
et/ou du type
M^{IV}(OR³)₄ (III),
et/ou des composés (I) pas encore hydrolytiquement condensés et éventuellement du type (II) et/ou (III), dans lequel M est sélectionné parmi borure, aluminium et un métal transitoire, les restes et les indices R¹, R², X et b étant définis comme dans la revendication 4 pour la formule (I) ou comme dans la revendication 5 pour la formule (Ia) et l'indice a étant 0, 1, 2 ou 3.

11. Procédé selon l'une quelconque des revendications 4 à 10, dans lequel R¹ dans les formules (I), (Ia), (Ia'), (Ia") ou (Ib) est un reste contenant une double liaison C=C non aromatique, de préférence une double liaison accessible à une addition de Michael et/ou dans lequel X est un hydrogène, un halogène, un hydroxy, un alcoxy, un acyloxy ou NR³₂ avec R³ étant un hydrogène ou un alkyle avec 1 à 6 atomes de carbone, de préférence 1 ou 2 atomes de carbone.

12. Procédé selon l'une quelconque des revendications précédentes, **caractérisé en ce que** l'on obtient à l'intérieur du corps façonné différents niveaux de réticulation, du fait que l'exposition au rayonnement est réalisée avec différents niveaux d'intensité, de préférence par réduction de l'intensité et expansion du foyer.

13. Corps façonné tridimensionnel, autoportant et/ou supporté par un substrat ou structure tridimensionnelle sur des surfaces, produit(e) par un procédé selon l'une quelconque des revendications 1 à 12, caractérisé(e) en ce qu'il ou elle présente une structure en surfaces spatiale à laquelle est lié au moins un composant biologique, ou dans lequel/laquelle la structure en surfaces spatiale est configurée de telle manière qu'elle convienne pour la liaison d'un composant biologique, dans lequel/laquelle le composant biologique est à chaque fois choisi parmi des cellules, protéines, enzymes, peptides, acides aminés, anticorps, fragments de ADN et ARN végétaux, animaux et humains.

14. Utilisation d'un corps façonné ou d'une structure en surfaces selon la revendication 13
- comme matrice pour un composite constitué d'une matrice extracellulaire formée de cellules, et de ces cellules elles-mêmes,
- pour un composite constitué de différents types de cellules ou pour des organes ou des parties d'organes qui se reforment,
- comme matrice pour des cellules destinées à obtenir des produits de sécrétion de ces cellules,
- comme corps de support pour des cellules se régénérant dans un tissu endommagé ou incomplet et/ou d'une matrice pour celles-ci,
- comme dépôt à appliquer ou implanter sur une surface épithéliale ou une surface d'un oeil d'un être humain ou d'un animal pour l'administration de substances actives, ou
- comme implant éventuellement dégradable partiellement ou entièrement pour l'introduction dans un corps animal ou humain.
